# EUROPEAN PATENT APPLICATION

(11) **EP 4 831 173 A1**
(43) Date of publication of application: **28.10.2026**
(21) Application number: 24908152.2
(22) Date of filing: 20.12.2024
(51) Int. Cl.: C07D 471/04, A61K 31/501, A61P 37/00, A61P 29/00, A61P 35/00, C07D 487/04, C07D 491/107

(54) **TYK2 INHIBITOR AND USE THEROF**

(30) Priority: 20.12.2023 KR 20230187651
(71) Applicant: HK inno.N Corporation, Cheongju-si, Chungcheongbuk-do 28158 (KR)
(72) Inventor: PARK, Jinyoung, Icheon-si, Gyeonggi-do 17389 (KR); HWANG, Insoo, Icheon-si, Gyeonggi-do 17389 (KR); CHUNG, Heesung, Icheon-si, Gyeonggi-do 17389 (KR); KIM, Jiwon, Icheon-si, Gyeonggi-do 17389 (KR); KIM, Bongtae, Icheon-si, Gyeonggi-do 17389 (KR); KIM, Hyejeong, Icheon-si, Gyeonggi-do 17389 (KR); PARK, Chanwoo, Icheon-si, Gyeonggi-do 17389 (KR); LEE, Keunho, Icheon-si, Gyeonggi-do 17389 (KR); TAE, Yanghun, Icheon-si, Gyeonggi-do 17389 (KR)
(74) Representative: Meissner Bolte Partnerschaft mbB
(86) International application number: PCT/KR2024/020810
(87) International publication number: WO 2025/135870

(57) **Abstract**

The present disclosure relates to a compound of following Chemical Formula 1, an optical isomer thereof, a stereoisomer thereof, a solvate thereof, a tautomer thereof, or a pharmaceutically acceptable salt thereof, and a pharmaceutical composition including the same, and the compound or the pharmaceutically acceptable salt thereof according to the present disclosure may be effectively used for prevention, improvement, or treatment of TYK2-related diseases.

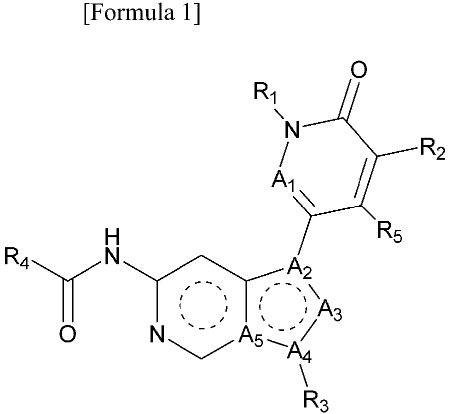

## Description

### BACKGROUND

### 1. Field of the Disclosure

The present disclosure relates to pyrrolopyridine, pyrrolopyrimidine compounds, and a preparing method thereof, which are useful for regulating an activity of tyrosine kinase 2 (TYK2). The present disclosure also relates to its use in treatment of diseases mediated by tyrosine kinase 2, in particular, autoimmune diseases, inflammatory diseases, and cancers.

### 2. Description of the Related Art

Tyrosine kinase 2 (TYK2) is a non-receptor tyrosine kinase that composes the Janus kinase (JAK) family along with Janus kinases 1, 2, and 3. The JAK family forms dimer in various combinations depending on a cytokine binding to a receptor domain of a cell membrane, thereby regulating an activity of an immune cell through Signal Transducer and Activator of Transcription (STAT) (Prior Art Documents 1 and 2). That is, JAK family dimer activates STAT, and the activated STAT enters a nucleus and promotes a production of a new cytokine, thereby inducing immune cell activity.

TYK2 is activated particularly by interleukin-12, interleukin-23, and type 1 and type 3 interferons, and these factors are inflammatory cytokines that are major causes of various autoimmune diseases including psoriasis (Prior Art Document 3). Therefore, TYK2 activity becomes a cause of various diseases. Results of several studies using TYK2-deficient mice support this. For example, in a psoriasis-induced experiment by IL-23, a skin inflammatory response was reduced in TYK2-deficient mice (Prior Art Document 4). In addition, the TYK2-deficient mice showed resistance in colitis and multiple sclerosis induced models (Prior Art Documents 5, 6). Such study results suggest the importance of the TYK2 mechanism in autoimmune diseases.

TYK2 is known to cause or worsen various autoimmune diseases, and the diseases are as follows. That is, TYK2 is associated with several types of psoriasis (plaque psoriasis, scalp psoriasis, nail psoriasis, palmoplantar pustulosis), psoriatic arthritis, several forms of lupus (systemic lupus erythematosus, discoid lupus erythematosus, subacute cutaneous lupus erythematosus), lupus nephritis, ulcerative colitis, crohn's disease, alopecia areata, ankylosing spondylitis, multiple sclerosis, sjogren's syndrome, type 1 diabetes, temporal arteritis, tendonitis, dermatomyositis, polymyositis, leukocyte adhesion deficiency, uveitis, celiac disease, adenomatous polyp, lichen planus, hidradenitis supprativa, acute myeloid leukemia (AML), COVID19, etc.

Accordingly, the inventors completed the present disclosure by studying a new compound that may act as a TYK2 inhibitor and proving that it may be effectively used for various autoimmune diseases, inflammatory diseases or cancers.

### PRIOR ARTS DOCUMENT

O'Shea, J.J., & Plenge, R. (2012). JAK and STAT signaling molecules in immunoregulation and immune-mediated disease. Immunity, 36(4), 542-550.
2. Teng, M. W. L., Bowman, E. P., McElwee, J. J., Smyth, M. J., Casanova, J.-L., Cooper, A. M., & Cua, D. J. (2015). IL-12 and IL-23 cytokines: From discovery to targeted therapies for immune-mediated inflammatory diseases. Nature Medicine, 21(7), 719-729.
3. Garrido-Trigo, A., & Salas, A. Molecular Structure and Function of Janus Kinases: Implications for the Development of Inhibitors. (2020). Journal of Crohn's & Colitis. 14(Supplement_2), S713-S724.
4. Ishizaki, M., Muromoto, R., Akimoto, T., Sekine, Y., Kon, S., Diwan, M., Maeda, H., Togi, S., Shimoda, K., Oritani, K., & Matsuda, T. (2014). Tyk2 is a therapeutic target for psoriasis-like skin inflammation. International Immunology, 26(5):257-267.
5. Hainzl, E., Stockinger, S., Rauch, I., Heider, S., Berry, D., Lassnig, C., Schwab, C., Rosebrock, F., Milinovich, G., Schlederer, M., Wagner, M., Schleper, C., Loy, A., Urich, T., Kenner, L., Han, X., Decker, T., Strobl, B., & Miiller, M., (2015). Intestinal Epithelial Cell Tyrosine Kinase 2 Transduces IL-22 Signals To Protect from Acute Colitis. Journal of Immunology, 195(10), 5011-24.
6. Oyamada, A., Ikebe, H., Itsumi, M., Saiwai, H., Okada, S., Shimoda, K., Iwakura, Y., Nakayama, K.I., Iwamoto, Y., Yoshikai, Y., & Yamada, H., (2009). Tyrosine kinase 2 plays critical roles in the pathogenic CD4 T cell responses for the development of experimental autoimmune encephalomyelitis. Journal of Immunology, 183(11), 7539-7546.
7. Lupardus, P. J., Ultsch, M., Wallweber, H., Bir Kohli, P., Johnson, A. R., & Eigenbrot, C. (2014). Structure of the pseudokinase-kinase domains from protein kinase TYK2 reveals a mechanism for Janus kinase (JAK) autoinhibition. Proceedings of the National Academy of Sciences of the United States of America, 111(22), 8025-8030.

### SUMMARY

An object of the present disclosure is to provide a novel compound of a following Formula 1 which may be effectively used for prevention, improvement, or treatment of TYK2-related diseases, an optical isomer thereof, a stereoisomer thereof, a solvate thereof, a tautomer thereof, or a pharmaceutically acceptable salt thereof.

In addition, an object of the present disclosure is to provide a pharmaceutical composition for prevention, improvement or treatment of the TYK2-related diseases, which includes the compound of Chemical Formula 1, the optical isomer thereof, the stereoisomer thereof, the solvate thereof, the tautomer thereof, or the pharmaceutically acceptable salt thereof, as an effective ingredient.

Another object of the present disclosure is to provide a use of the compound for preparing a medicament for prevention, improvement or treatment of the TYK2-related diseases.

Still another object of the present disclosure is to provide a method for preventing, improving or treating the TYK2-related diseases by administering the compound.

Hereinafter, the present disclosure will be described in detail.

Unless otherwise defined, all technical terms used herein have the same meaning as commonly understood by those having ordinary skill in the art to which the present disclosure belongs. In addition, the numerical values set forth herein are considered to include the meaning of "about" unless explicitly stated otherwise. All publications and other references mentioned herein are incorporated herein by reference in their entirety.

The definition of residues used in this specification are described below. In addition, in a case where there is no separate definition of a residue, it is used in the meaning generally understood by those skilled in the art.

The term "independently" as used herein means that when more than one substituent is selected from a plurality of possible substituents, these substituents may be the same or different from each other.

The terms "optionally substituted" or "substituted" as used herein refers to replacement of one or more hydrogen atoms with a monovalent or divalent radical. Suitable substituents include, for example, deutero, hydroxy, nitro, amino, imino, cyano, halo, thio, sulfonyl, thioamido, amidino, imidino, oxo, oxamidino, methoxamidino, imidino, guanidino, sulfonamido, carboxyl, formyl, lower alkyl, halo lower alkyl, lower alkylamino, halo lower alkylamino, lower alkoxy, halo lower alkoxy, lower alkoxyalkyl, alkylcarbonyl, aminocarbonyl, arylcarbonyl, aralkylcarbonyl, heteroarylcarbonyl, heteroaralkylcarbonyl, alkylthio, aminoalkyl, cyanoalkyl, aryl, heteroaryl, heterocyclyl, cycloalkyl, etc. When the substituted substituent includes a linear chain group, a substitution may occur within a chain (e.g., 2-hydroxypropyl, 2-aminobutyl, etc.) or at a chain terminal (e.g., 2-hydroxyethyl, 3-cyanopropyl, etc.). The substituted substituent may be a linear, branched or cyclic configuration of a covalently bonded carbon or a heteroatom. It is understood that the above definition is not intended to include unacceptable substitution patterns (e.g., a methyl substituted with five fluoro groups, or a halogen atom substituted with another halogen atom). Such unacceptable substitution patterns are known to those skilled in the art.

The terms "halo," "halogen," and "halide(s)" as used herein include fluoro, chloro, bromo, and iodo.

As used herein, "alkyl" refers to an aliphatic hydrocarbon radical, and includes both linear and branched hydrocarbon radicals. For example, C₁-C₆alkyl is an aliphatic hydrocarbon having 1 to 6 carbon atoms and may be methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl and 2-ethylbutyl. For example, alkyl means C₁-₁₀ alkyl, preferably C₁-₆ alkyl and C₁-₄ alkyl, more preferably C₁-₃ alkyl.

The term "haloalkyl" as used herein means an alkyl group substituted with one or more, for example, 1 to 3, 1 to 4, halogen atoms, and the alkyl group is defined as above. "Halo" refers to F, Cl, Br, or I, and the term is used compatibly with the term "halogen". For example, haloalkyl means fluoromethyl, difluoromethyl, chloromethyl, trifluoromethyl, trifluoroethyl, trifluoropropyl, trifluoroisopropyl, etc.

The term "alkenyl" as used herein refers to a hydrocarbon radical derived from saturated alkyl having at least one double bond. The alkenyl may be an E or Z configuration. For example, alkenyl means C₂-₁₀alkenyl, preferably C₂-₆ alkenyl and C₂-₄ alkenyl, more preferably C₂-₃ alkenyl.

The term "alkynyl" as used herein refers to a hydrocarbon radical derived from saturated alkyl having at least one triple bond. For example, alkynyl means C₂-₁₀ alkynyl, preferably C₂-₆ alkynyl and C₂-₄ alkynyl, more preferably C₂-₃ alkynyl.

The term "alkoxy" as used herein refers to an -O-alkyl or alkyl-O- group, and the alkyl group is defined as described above. For example, it may be methoxy, ethoxy, n-propoxy, isopropoxy, n-butoxy and t-butoxy.

The term "hydroxy" or "hydroxyl" as used herein, means -OH alone or in combination with other terms.

The term "amino" as used herein means -NH₂.

The term "cycloalkyl" as used herein refers to a cyclic alkyl which may be substituted or unsubstituted, for example, C₃-₂₀cycloalkyl refers to a monovalent saturated hydrocarbon ring system having 3 to 20 carbon atoms. A cycloalkyl group may be monocyclic or polycyclic. Examples of cycloalkyl include cyclopropyl, cyclobutyl, cyclopentyl, cyclohexyl, cycloheptyl, cyclooctyl, etc., but are not limited thereto. Preferably, cycloalkyl may be C₃-₈cycloalkyl or C₃-₆cycloalkyl.

The term "cycloalkenyl" as used herein refers to a group which is a cyclic alkene derived from saturated cycloalkyl having one double bond. A cycloalkenyl group may be monocyclic or polycyclic. Examples of cycloalkenyl include cyclopropenyl, cyclobutenyl, cyclopentenyl, cyclohexenyl, cycloheptenyl, cyclooctenyl, etc., but are not limited thereto. Preferably, cycloalkenyl may be C₃-₈cycloalkenyl or C₃-₆cycloalkenyl.

The term "aryl" as used herein refers to a monovalent aromatic hydrocarbon having, for example, 6 to 20 carbon atoms (C₆-₂₀), derived by removing one hydrogen atom from a single carbon atom of a parent aromatic ring. Aryl may include a bicyclic radical containing an aromatic ring fused to a saturated or partially unsaturated ring. An exemplary aryl group may include radicals derived from benzene (phenyl), substituted phenyl, biphenyl, naphthyl, toluyl, naphthalenyl, anthracenyl, indenyl, indanyl, etc. As a specific example, aryl means C₆-₁₂ aryl, preferably C₆-₁₀ aryl.

As used herein, "heterocycle" refers to an aromatic, saturated or partially unsaturated mono-, bi- or poly-ring system containing a specified number of ring atoms, and includes one or more heteroatoms selected from N, O and S. A ring members, wherein a heterocyclic ring may be linked to a base molecule via a ring atom (which may be C or N). A bicyclic system may be linked to 1,1-fusion (spiro), 1,2-fusion (fused), or 1,>2-fusion (bridgehead). Preferably, the 1,1-fused(spiro) may be oxaspiro[3.3]heptanyl, oxaspiro[3.4]octanyl, azaspiro[3.3]heptanyl, azaspiro[3.4]octanyl, etc., but is not limited thereto.

As used herein, "heteroaryl" refers to a monovalent or divalent substituent derived from a monoheterocyclic or polyheterocyclic aromatic hydrocarbon containing at least one, preferably 1 to 4, 1 to 3 or 1 to 2, heteroatoms selected from N, O and S. Examples of heteroaryls include thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxazole, but are not limited thereto. 3,4-Oxadiazolyl, 1,2,4-thiadiazolyl, 1,3,4-thiadiazolyl, triazolyl, tetrazolyl, triazinyl, indolyl, etc. Examples of bicyclic heteroaryl include indolyl, benzothiophenyl, benzofuranyl, benzimidazolyl, benzoxazolyl, benzisoxazolyl, benzthiazolyl, benzthiadiazolyl, quinolinyl, isoquinolinyl, furopyridinyl and similar groups thereof, but are not limited thereto. Unless otherwise defined, heteroaryl is 4-12-membered heteroaryl, preferably 4-10-membered heteroaryl, and more preferably 4-7-membered heteroaryl.

As used herein, "heterocycloalkyl" refers to monocyclic, bicyclic, tricyclic or higher cyclic alkyl having 3 to 10 carbon ring members containing one or more selected from N, O and S, for example 1 to 4, 1 to 3, or 1 to 2 heteroatoms. In addition, the heterocycle according to the present disclosure may also be fused or bridged heterocycloalkyl. Examples of heterocycloalkyl may be azetidinyl, oxetanyl, tetrahydro, tetrahydrofuran, pyrrolidinyl, imidazolinyl, oxazolinyl, oxazolidinyl, piperidinyl, piperazinyl, tetrahydropyranyl, dihydropyranyl, tetrahydropyridinyl, dihydropyridinyl, dihydrothiopyranyl, tetrahydropyrimidinyl, tetrahydropyridazinyl, dihydropyranyl, tetrahydropyranyl, tetrahydrothiopyranyl, tetrahydropyrazolopyridinyl, morpholinyl, indolinyl, azethiomorpholinyl, etc.

Attachment of a heterocycloalkyl substituent may occur via a carbon atom or a heteroatom. The heterocycloalkyl group may be optionally substituted with one or more suitable groups via one or more of the aforementioned group. Unless otherwise defined, heterocycloalkyl refers to 4- to 12-membered heterocycloalkyl, preferably 4- to 10-membered heterocycloalkyl, and more preferably 4- to 7-membered heterocycloalkyl.

The cyclic substituents (e.g., cycloalkyl, cycloalkenyl, aryl, heterocycle, heteroaryl, heterocycloalkyl, etc.) may be unsubstituted or substituted.

### Compound

In order to solve the above problem, the present disclosure provides a compound represented by the following Chemical Formula 1, an optical isomer thereof, a stereoisomer thereof, a solvate thereof, a tautomer thereof, or a pharmaceutically acceptable salt thereof:

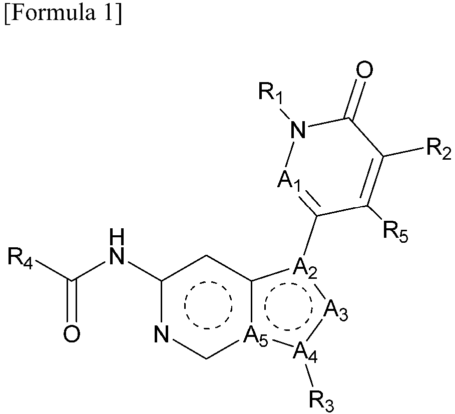

In the above chemical formula,
A₁, A2, A3, A₄ and A₅ may each independently be C, CH, CH₂ or N;
R₁ may be C₁-C₁₀alkyl, C₁-C₁₀haloalkyl, C₃-C₈cycloalkyl, or 4- to 12-membered heterocycloalkyl, wherein the C₁-C₁₀alkyl, the C₁-C₁₀haloalkyl, the C₃-C₈cycloalkyl, or the 4- to 12-membered heterocycloalkyl may be unsubstituted or may be substituted with at least one, preferably 1 to 4 substituents selected from a group consisting of oxo(=O), halo, C₁-C₁₀alkyl, C₁-C₁₀alkoxy and C₁-C₁₀haloalkyl;
R₂ may be H, C₁-C₁₀alkyl, C₁-C₁₀alkoxy, C₃-C₈cycloalkyl, 4-12 membered heterocycloalkyl, OR' or NR'R", wherein the C₁-C₁₀alkyl, the C₁-C₁₀alkoxy or the 4-12-membered heterocycloalkyl may be unsubstituted, or may be substituted with at least one, preferably 1 to 4 substituent selected from a group consisting of halo, cyano, C₁-C₁₀alkyl, C₁-C₁₀alkoxy, C₁-C₁₀alkoxyC₁-C₁₀alkyl, 4-12 membered heterocycloalkyl, C₆-C₁₀aryl and C₃-C₈cycloalkyl;
wherein, R' and R" may each independently be H, C₁-C₁₀alkyl, and in this case, the C₁-C₁₀alkyl may be unsubstituted or may be substituted with 1 or more, preferably 1 to 4 substituents selected from a group consisting of halo, cyano, 4-12-membered heterocycloalkyl, C₆-C₁₀aryl, C₃-C₈cycloalkyl and C₁-C₁₀alkoxy; or
R' and R" may form 4- to 12-membered heterocycloalkyl together with N in the NR'R", wherein, the 4- to 12-membered heterocycloalkyl is unsubstituted or substituted with halo, C₁-C₁₀alkoxy, or C₁-C₁₀alkoxy C₁-C₁₀alkyl;
R₃ may be H, C₁-C₁₀alkyl, C₁-C₁₀deuteroalkyl, C₂-C₁₀alkenyl, C₃-C₈cycloalkyl, -C₁-C₁₀ alkyl-C₃-C₈cycloalkyl, -C(=O)-C₃-C₈cycloalkyl, C₃-C₈cycloalkenyl, 4-to 12-membered heterocycloalkyl, or -C₁-C₁₀alkyl-4-12-membered heterocycloalkyl, wherein, the C₁-C₁₀alkyl, C₁-C₁₀deuteroalkyl, C₂-C₁₀alkenyl, C₃-C₈cycloalkyl, -C₁-C₁₀alkyl-C₃-C₈cycloalkyl, -C(=O)-C₃-C₈cycloalkyl, C₃-C₈cycloalkenyl, 4-12-membered heterocycloalkyl, or -C₁-C₁₀alkyl-4-12-membered heterocycloalkyl may be unsubstituted or substituted with one or more, preferably 1 to 4 substituents, selected from a group consisting of halo, cyano, oxo, C₁-C₁₀alkyl, C₂-C₁₀alkenyl, C₁-C₆alkoxy, C₃-C₈cycloalkyl unsubstituted or substituted with 1 to 3 halo, and 4-12-membered heterocycloalkyl;
R₄ may C₁-C₁₀alkyl, C₁-C₁₀deuteroalkyl, or amino, wherein C₁-C₁₀alkyl, C₁-C₁₀deuteroalkyl, or amino may be unsubstituted or substituted with one or more, preferably 1 to 4 substituents selected from a group consisting of hydroxy and C₁-C₁₀ alkyl;
R₅ may be H, halo, nitro, cyano, amino, hydroxy, -C(=O)R"', -CO(=O)R"', - N(R‴)₂, -C(=O)NR‴, -SO₂R‴, C₁-C₁₀alkyl, C₁-C₁₀deuteroalkyl, C₁-C₁₀haloalkyl, C₁-C₁₀hydroxyalkyl, C₁-C₁₀alkoxy, C₂-C₁₀alkenyl, C₂-C₁₀alkynyl, C₃-C₈cycloalkyl, C₆-C₁₀aryl, C₆-C₁₀arylC₁-C₁₀alkyl, 4-12-membered heterocycloalkyl, 4-12-membered heterocycloalkylC₁-C₁₀alkyl, 4-12-membered heteroaryl or 4-12-membered heteroarylC₁-C₁₀alkyl, wherein, the C₁-C₁₀alkyl, C₁-C₁₀deuteroalkyl, C₁-C₁₀haloalkyl, C₁-C₁₀hydroxyalkyl, C₁-C₁₀alkoxy, C₂-C₁₀alkenyl, C₂-C₁₀alkynyl, C₃-C₈cycloalkyl, C₆-C₁₀aryl, C₆-C₁₀arylC₁-C₁₀alkyl, 4-12-membered heterocycloalkyl, 4-12-membered heterocycloalkylC₁-C₁₀alkyl, 4-12-membered heteroaryl or 4-12-membered heteroarylC₁-C₁₀alkyl is unsubstituted, substituted with one or more, preferably 1 to 4 substituents, selected from a group consisting of halo, cyano, nitro, amino, hydroxy, C₁-C₁₀alkyl, C₂-C₁₀alkenyl, C₁-C₁₀alkoxy, C₆-C₁₀aryl, 4-12-membered heterocycloalkyl, 4-12-membered heteroaryl, and C₃-C₈cycloalkyl, and
the R‴ may be H, halo, C₁-C₁₀alkyl, C₁-C₁₀deuteroalkyl, C₁-C₁₀haloalkyl, C₁-C₁₀hydroxyalkyl, C₁-C₁₀alkoxy, C₂-C₁₀alkenyl, C₂-C₁₀alkynyl, C₃-C₈cycloalkyl, C₆-C₁₀aryl, C₆-C₁₀arylC₁-C₁₀alkyl, 4-12-membered heterocycloalkyl, 4-12-membered heterocycloalkylC₁-C₁₀alkyl, 4-12-membered heteroaryl or 4-12-membered heteroarylC₁-C₁₀alkyl.

In addition, in a specific embodiment of the present disclosure,
the R₁ may be C₁-C₆alkyl, C₁-C₆haloalkyl, C₃-C₆cycloalkyl, or 4- to 10-membered heterocycloalkyl, wherein the C₁-C₆alkyl, the C₁-C₆haloalkyl, the C₃-C₆cycloalkyl, or the 4- to 10-membered heterocycloalkyl may be unsubstituted or substituted with 1 to 4 substituents selected from a group consisting of oxo, fluoro, chloro, bromo, C₁-C₆alkyl, C₁-C₆alkoxy, and C₁-C₆haloalkyl.

In a more specific embodiment, C₃-C₆cycloalkyl of R₁ may be cyclobutyl, cyclopentyl or cyclohexyl, and 4- to 10-membered heterocycloalkyl of R₁ may be oxetanyl, tetrahydrofuranyl or tetrahydropyranyl.

In a more specific embodiment, C₁-C₆alkyl of R₁ may be methyl, ethyl, propyl, isopropyl, butyl, and t-butyl.

The C₁-C₁₀alkyl, C₁-C₁₀haloalkyl,C₃-C₈cycloalkyl, or 4- to 12-membered heterocycloalkyl of the R₁ may be unsubstituted or substituted with 1 to 3 substituents selected from a group consisting of oxo, fluoro, methyl, ethyl, propyl, isopropyl, trifluoromethyl, and methoxy.

In a more specific embodiment, R₁ may be selected from the following bases:

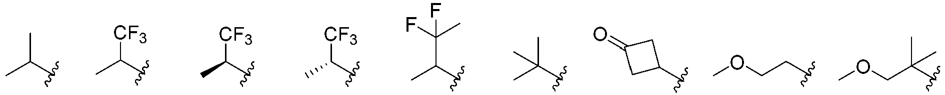

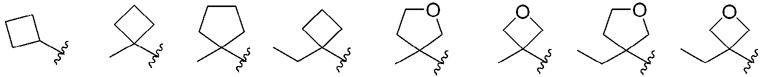

In addition, in a specific embodiment of the present disclosure,
R₂ may be H, C₁-C₆alkyl, C₁-C₆alkoxy, C₃-C₈cycloalkyl, 4-10-membered heterocycloalkyl, OR' or NR'R", and the C₁-C₆alkyl, the C₁-C₆alkoxy or the 4-10-membered heterocycloalkyl may unsubstituted or substituted with 1 to 4 substituents selected from a group consisting of fluoro, chloro, bromo, cyano, C₁-C₆alkyl, C₁-C₆alkoxy, C₁-C₆alkoxyC₁-C₆alkyl, 4-10-membered heterocycloalkyl, C₆-C₁₀aryl, and C₃-C₆cycloalkyl, and
wherein, R' and R" may each independently be H, or C₁-C₆alkyl, and wherein, the C₁-C₆alkyl may be unsubstituted or substituted with 1 to 4 substituents selected from a group consisting of fluoro, chloro, bromo, cyano, 4-10-membered heterocycloalkyl, C₆-C₁₀aryl, C₃-C₆cycloalkyl, and C₁-C₆alkoxy.

Alternatively, R' and R" may form a 4- to 10-membered heterocycloalkyl together with N of the NR'R", wherein the 4- to 10-membered heterocycloalkyl may unsubstituted or substituted with fluoro, chloro, bromo, C₁-C₆alkoxy, or C₁-C₆alkoxyC₁-C₆alkyl.

In a more specific embodiment, R₂ may be selected from the following bases:

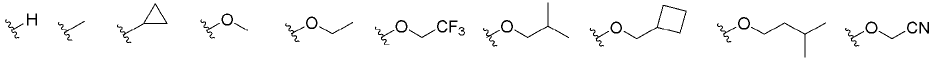

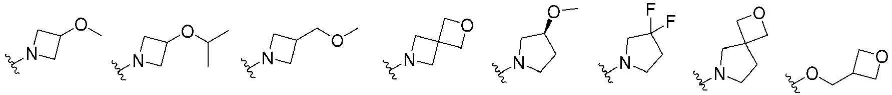

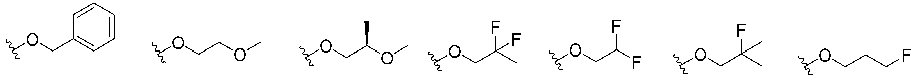

In addition, in a specific embodiment of the present disclosure,
R₃ may H, C₁-C₆alkyl, C₁-C₆deuteroalkyl, C₂-C₆alkenyl, C₃-C₈cycloalkyl, - C₁-C₆alkyl-C₃-C₈cycloalkyl, -C(=O)-C₃-C₈cycloalkyl, C₃-C₈cycloalkenyl, 4-10-membered heterocycloalkyl, or -C₁-C₆ alkyl-4-10-membered heterocycloalkyl, and wherein, the C₁-C₆alkyl, the C₁-C₆deuteroalkyl, the C₂-C₆alkenyl, the C₃-C₈cycloalkyl, the -C₁-C₆alkyl-C₃-C₈cycloalkyl, the -C(=O)-C₃-C₈cycloalkyl, the C₃-C₈cycloalkenyl, the 4-10-membered heterocycloalkyl, or the -C₁-C₆alkyl-4-10-membered heterocycloalkyl may be unsubstituted or substituted with 1 to 4 substituents selected from a group consisting of fluoro, chloro, bromo, cyano, oxo, C₁-C₆alkyl, C₂-C₆alkenyl, C₁-C₆alkoxy, C₃-C₈cycloalkyl unsubstituted or substituted with 1 to 3 halo, and 4-10-membered heterocycloalkyl.

In a more specific embodiment, R₃ may be selected from the following bases:

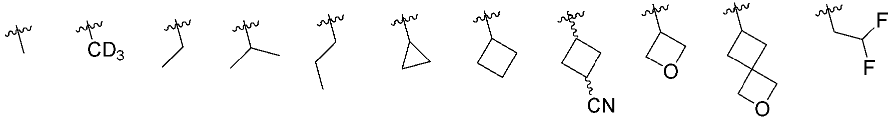

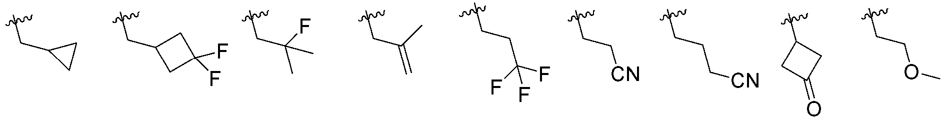

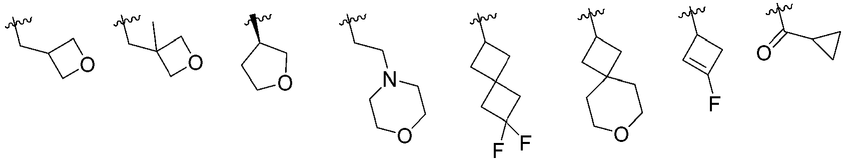

In a specific embodiment of the present disclosure,
R₄ may be C₁-C₆alkyl, C₁-C₆deuteroalkyl, or amino, and wherein the C₁-C₆alkyl, the C₁-C₆deuteroalkyl, or the amino may be unsubstituted or substituted with 1 or 2 substituents selected from a group consisting of hydroxy and C₁-C₆alkyl.

In a more specific embodiment, R₄ may be CH₃, CD₃, CH₂OH, NH₂, or NHCH₃.

In a specific embodiment of the present disclosure,
R₅ may be H, halo, nitro, cyano, amino, hydroxy, -C(=O)R‴, -CO(=O)R"', - N(R‴)₂, -C(=O)NR"', -SO₂R‴, C₁-C₆alkyl, C₁-C₆deuteroalkyl, C₁-C₆haloalkyl, C₁-C₆hydroxyalkyl, C₁-C₆alkoxy, C₂-C₆alkenyl, C₂-C₆alkynyl, C₃-C₆cycloalkyl, C₆-C₁₀aryl, C₆-C₁₀arylC₁-C₆alkyl, 4-10-membered heterocycloalkyl, 4-10-membered heterocycloalkylC₁-C₆alkyl, 4-10-membered heteroaryl or 4-10-membered heteroarylC₁-C₆alkyl, and wherein, the C₁-C₆alkyl, the C₁-C₆deuteroalkyl, the C₁-C₆haloalkyl, the C₁-C₆hydroxyalkyl, the C₁-C₆alkoxy, the C₂-C₆alkenyl, the C₂-C₆alkynyl, the C₃-C₆cycloalkyl, the C₆-C₁₀aryl, the C₆-C₁₀arylC₁-C₆alkyl, the 4-10-membered heterocycloalkyl, the 4-10-membered heterocycloalkylC₁-C₆alkyl, the 4-10-membered heteroaryl or the 4-10-membered heteroarylC₁-C₆alkyl may be unsubstituted or substituted with 1 to 4 substituents selected from a group consisting of halo, cyano, nitro, amino, hydroxy, C₁-C₆alkyl, C₂-C₆alkenyl, C₁-C₆alkoxy, C₆-C₁₀aryl, 4-10-membered heterocycloalkyl, 4-10-membered heteroaryl and C₃-C₆cycloalkyl, and
the R‴ may be H, halo, C₁-C₆alkyl, C₁-C₆deuteroalkyl, C₁-C₆haloalkyl, C₁-C₆hydroxyalkyl, C₁-C₆alkoxy, C₂-C₆alkenyl, C₂-C₆alkynyl, C₃-C₆cycloalkyl, C₆-C₁₀aryl, C₆-C₁₀arylC₁-C₆alkyl, 4-10-membered heterocycloalkyl, 4-10-membered heterocycloalkylC₁-C₆alkyl, 4-10-membered heteroaryl or 4-10-membered heteroarylC₁-C₆alkyl.

In a specific embodiment of the present disclosure, the heterocycloalkyl or the heteroaryl may include one or more, preferably 1 to 4, more preferably 1 to 3 heteroatoms. The heteroatom may be selected from a group consisting of N, O and S.

In a specific embodiment, the compounds of Formula 1 according to the present disclosure may be one or more selected from a group consisting of the following compounds 1 to 117, but is not limited thereto.
Compound 1: N-(1-methyl-3-(1-(3-methyltetrahydrofuran-3-yl)-6-oxo-1,6-dihydropyridazin-3-yl)-1H-pyrrolo[2,3-c]pyridin-5-yl)acetamide,
Compound 2: N-(1-(3-cyanocyclobutyl)-3-(1-(3-methyltetrahydrofuran-3-yl)-6-oxo-1,6-dihydropyridazin-3-yl)-1H-pyrrolo[2,3-c]pyridin-5-yl)acetamide,
Compound 3: N-(1-methyl-3-(1-(3-methyloxetan-3-yl)-6-oxo-1,6-dihydropyridazin-3-yl)-1H-pyrrolo[2,3-c]pyridin-5-yl)acetamide,
Compound 4: 1-(1-methyl-3-(1-(3-methyltetrahydrofuran-3-yl)-6-oxo-1,6-dihydropyridazin-3-yl)-1H-pyrrolo[2,3-c]pyridin-5-yl)urea,
Compound 5: N-(1-(3-cyanocyclobutyl)-3-(5-methyl-1-(3-methyltetrahydrofuran-3-yl)-6-oxo-1,6-dihydropyridazin-3-yl)-1H-pyrrolo[2,3-c]pyridin-5-yl)acetamide,
Compound 6: N-(1-(3-cyanocyclobutyl)-3-(1-(3-ethyltetrahydrofuran-3-yl)-5-methyl-6-oxo-1,6-dihydropyridazin-3-yl)-1H-pyrrolo[2,3-c]pyridin-5-yl)acetamide,
Compound 7: 1-(3-(1-(3-ethyltetrahydrofuran-3-yl)-5-methyl-6-oxo-1,6-dihydropyridazin-3-yl)-1-methyl-1H-pyrrolo[2,3-c]pyridin-5-yl)urea,
Compound 8: N-(1-(3-cyanocyclobutyl)-3-(5-cyclopropyl-1-(3-methyltetrahydrofuran-3-yl)-6-oxo-1,6-dihydropyridazin-3-yl)-1H-pyrrolo[2,3-c]pyridin-5-yl)acetamide,
Compound 9: N-(1-(3-cyanocyclobutyl)-3-(5-ethoxy-1-(3-methyltetrahydrofuran-3-yl)-6-oxo-1,6-dihydropyridazin-3-yl)-1H-pyrrolo[2,3-c]pyridin-5-yl)acetamide,
Compound 10: N-(1-(3-cyanocyclobutyl)-3-(5-(3-methoxyazetidin-1-yl)-1-(3-methyltetrahydrofuran-3-yl)-6-oxo-1,6-dihydropyridazin-3-yl)-1H-pyrrolo[2,3-c]pyridin-5-yl)acetamide,
Compound 11: 1-(3-(5-(3-methoxyazetidin-1-yl)-1-(3-methyltetrahydrofuran-3-yl)-6-oxo-1,6-dihydropyridazin-3-yl)-1-methyl-1H-pyrrolo[2,3-c]pyridin-5-yl)urea,
Compound 12: N-(1-(3-cyanocyclobutyl)-3-(5-cyclopropyl-6-oxo-1-(3-oxocyclobutyl)-1,6-dihydropyridazin-3-yl)-1H-pyrrolo[2,3-c]pyridin-5-yl)acetamide,
Compound 13: N-(1-(3-cyanocyclobutyl)-3-(1-(3-methyltetrahydrofuran-3-yl)-6-oxo-1,6-dihydropyridin-3-yl)-1H-pyrrolo[2,3-c]pyridin-5-yl)acetamide,
Compound 14: 1-(1-methyl-3-(1-(3-methyltetrahydrofuran-3-yl)-6-oxo-1,6-dihydropyridin-3-yl)-1H-pyrrolo[2,3-c]pyridin-5-yl)urea,
Compound 15: N-(3-methyl-1-(1-(3-methyltetrahydrofuran-3-yl)-6-oxo-1,6-dihydropyridazin-3-yl)-1H-pyrrolo[3,2-c]pyridin-6-yl)acetamide,
Compound 16: N-(5-(5-(3-methoxyazetidine-1-yl)-1-(3-methyltetrahydrofuran-3-yl)-6-oxo-1,6-dihydropyridazin-3-yl)-7-methylpyrrolo[1,2-c]pyrimidin-3-yl)acetamide,
Compound 17: N-(3-(5-(3-methoxyazetidin-1-yl)-1-(2-methoxyethyl)-6-oxo-1,6-dihydropyridazin-3-yl)-1-methyl-1H-pyrrolo[2,3-c]pyridin-5-yl)acetamide,
Compound 18: N-(3-(1-(1-methoxy-2-methylpropan-2-yl)-6-oxo-1,6-dihydropyridazin-3-yl)-1-methyl-1H-pyrrolo[2,3-c]pyridin-5-yl)acetamide,
Compound 19: N-(3-(5-isobutoxy-1-isopropyl-6-oxo-1,6-dihydropyridazin-3-yl)-1-methyl-1H-pyrrolo[2,3-c]pyridin-5-yl)acetamide,
Compound 20: N-(3-(1-isopropyl-5-(3-methoxyazetidin-1-yl)-6-oxo-1,6-dihydropyridazin-3-yl)-1-methyl-1H-pyrrolo[2,3-c]pyridin-5-yl)acetamide,
Compound 21: N-(1-cyclopropyl-3-(1-isopropyl-5-(3-methoxyazetidin-1-yl)-6-oxo-1,6-dihydropyridazin-3-yl)-1H-pyrrolo[2,3-c]pyridin-5-yl)acetamide,
Compound 22: N-(1-(3-cyanocyclobutyl)-3-(5-isobutoxy-1-isopropyl-6-oxo-1,6-dihydropyridazin-3-yl)-1H-pyrrolo[2,3-c]pyridin-5-yl)acetamide,
Compound 23: N-(3-(1-cyclobutyl-5-isobutoxy-6-oxo-1,6-dihydropyridazin-3-yl)-1-methyl-1H-pyrrolo[2,3-c]pyridin-5-yl)acetamide,
Compound 24: N-(3-(1-cyclobutyl-5-(3-(methoxymethyl)azetidin-1-yl)-6-oxo-1,6-dihydropyridazin-3-yl)-1-methyl-1H-pyrrolo[2,3-c]pyridin-5-yl)acetamide,
Compound 25: N-(3-(5-(isopentyloxy)-1-isopropyl-6-oxo-1,6-dihydropyridazin-3-yl)-1-methyl-1H-pyrrolo[2,3-c]pyridin-5-yl)acetamide,
Compound 26: N-(3-(1-isopropyl-6-oxo-5-(2,2,2-trifluoroethoxy)-1,6-dihydropyridazin-3-yl)-1-methyl-1H-pyrrolo[2,3-c]pyridin-5-yl)acetamide,
Compound 27: N-(3-(5-(benzyloxy)-1-isopropyl-6-oxo-1,6-dihydropyridazin-3-yl)-1-methyl-1H-pyrrolo[2,3-c]pyridin-5-yl)acetamide,
Compound 28: N-(3-(1-(tert-butyl)-5-isobutoxy-6-oxo-1,6-dihydropyridazin-3-yl)-1-methyl-1H-pyrrolo[2,3-c]pyridin-5-yl)acetamide,
Compound 29: N-(3-(1-(tert-butyl)-5-(3-methoxyazetidin-1-yl)-6-oxo-1,6-dihydropyridazin-3-yl)-1-methyl-1H-pyrrolo[2,3-c]pyridin-5-yl)acetamide,
Compound 30: N-(3-(5-isobutoxy-1-(1-methylcyclopentyl)-6-oxo-1,6-dihydropyridazin-3-yl)-1-methyl-1H-pyrrolo[2,3-c]pyridin-5 -yl)acetamide,
Compound 31: N-(3-(5-(3-methoxyazetidin-1-yl)-1-(1-methylcyclopentyl)-6-oxo-1,6-dihydropyridazin-3-yl)-1-methyl-1H-pyrrolo[2,3-c]pyridin-5-yl)acetamide,
Compound 32: N-(3-(5-isobutoxy-1-(1-methylcyclobutyl)-6-oxo-1,6-dihydropyridazin-3-yl)-1-methyl-1H-pyrrolo[2,3-c]pyridin-5-yl)acetamide,
Compound 33: N-(1-cyclobutyl-3-(5-isobutoxy-1-isopropyl-6-oxo-1,6-dihydropyridazin-3-yl)-1H-pyrrolo[2,3-c]pyridin-5-yl)acetamide,
Compound 34: 1-(3-(5-isobutoxy-1-isopropyl-6-oxo-1,6-dihydropyridazin-3-yl)-1-methyl-1H-pyrrolo[2,3-c]pyridin-5-yl)urea,
Compound 35: 1-(1-cyclobutyl-3-(5-isobutoxy-1-(1-methylcyclobutyl)-6-oxo-1,6-dihydropyridazin-3-yl)-1H-pyrrolo[2,3-c]pyridin-5-yl)urea,
Compound 36: N-(1-cyclobutyl-3-(5-isobutoxy-6-oxo-1-(1,1,1-trifluoropropan-2-yl)-1,6-dihydropyridazin-3-yl)-1H-pyrrolo[2,3-c]pyridin-5-yl)acetamide,
Compound 37: 1-(3-(5-isobutoxy-6-oxo-1-(1,1,1-trifluoropropan-2-yl)-1,6-dihydropyridazin-3-yl)-1-methyl-1H-pyrrolo[2,3-c]pyridin-5-yl)urea,
Compound 38: 1-(1-cyclobutyl-3-(5-isobutoxy-1-isopropyl-6-oxo-1,6-dihydropyridazin-3-yl)-1H-pyrrolo[2,3-c]pyridin-5-yl)urea,
Compound 39: N-(3-(5-(3-methoxyazetidin-1-yl)-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 40: N-(3-(5-(3-methoxyazetidin-1-yl)-1-(1-methylcyclobutyl)-6-oxo-1,6-dihydropyridazin-3-yl)-1-methyl-1H-pyrrolo[2,3-c]pyridin-5-yl)acetamide,
Compound 41: N-(1-cyclobutyl-3-(5-(3-methoxyazetidin-1-yl)-1-(1-methylcyclobutyl)-6-oxo-1,6-dihydropyridazin-3-yl)-1H-pyrrolo[2,3-c]pyridin-5-yl)acetamide,
Compound 42: (S)-N-(3-(5-(3-methoxypyrrolidin-1-yl)-1-(1-methylcyclobutyl)-6-oxo-1,6-dihydropyridazin-3-yl)-1-methyl-1H-pyrrolo[2,3-c]pyridin-5-yl)acetamide,
Compound 43: (S)-N-(1-cyclobutyl-3-(5-(3-methoxypyrrolidin-1-yl)-1-(1-methylcyclobutyl)-6-oxo-1,6-dihydropyridazin-3-yl)-1H-pyrrolo[2,3-c]pyridin-5-yl)acetamide,
Compound 44: N-(1-cyclobutyl-3-(1-(1-methylcyclobutyl)-6-oxo-5-(2-oxa-6-azaspiro[3.4]octan-6-yl)-1,6-dihydropyridazin-3- yl)-1H-pyrrolo[2,3-c]pyridin-5-yl)acetamide,
Compound 45: N-(1-methyl-3-(1-(1-methylcyclobutyl)-6-oxo-5-(2-oxa-6-azaspiro[3.4]octan-6-yl)-1,6-dihydropyridazin-3- yl)-1H-pyrrolo[2,3-c]pyridin-5-yl)acetamide,
Compound 46: N-(1-methyl-3-(1-(1-methylcyclobutyl)-6-oxo-5-(2-oxa-6-azaspiro[3.3]heptan-6-yl)-1,6-dihydropyridazin-3- yl)-1H-pyrrolo[2,3-c]pyridin-5-yl)acetamide,
Compound 47: N-(3-(5-(cyanomethoxy)-1-(1-methylcyclobutyl)-6-oxo-1,6-dihydropyridazin-3-yl)-1-methyl-1H-pyrrolo[2,3-c]pyridin-5-yl)acetamide,
Compound 48: N-(1-methyl-3-(1-(1-methylcyclobutyl)-5-(oxetan-3-ylmethoxy)-6-oxo-1,6-dihydropyridazin-3-yl)-1H-pyrrolo[2,3-c]pyridin-5-yl)acetamide,
Compound 49: N-(1-cyclopropyl-3-(5-isobutoxy-6-oxo-1-(1,1,1-trifluoropropan-2-yl)-1,6-dihydropyridazin-3-yl)-1H-pyrrolo[2,3-c]pyridin-5-yl)acetamide,
Compound 50: (R)-N-(3-(5-isobutoxy-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 51: N-(3-(5-isobutoxy-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 52: N-(1-cyclopropyl-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide,
Compound 53: N-(1-cyclopropyl-3-(1-(1-methylcyclobutyl)-6-oxo-5-(2-oxa-6-azaspiro[3.4]octan-6-yl)-1,6-dihydropyridazin-3-yl)-1H-pyrrolo[2,3-c]pyridin-5-yl)acetamide,
Compound 54: (S)-N-(3-(5-isobutoxy-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 55: N-(3-(5-(cyclobutylmethoxy)-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 56: N-(3-(5-(2,2-difluoropropoxy)-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 57: (R)-N-(3-(1-(1-ethylcyclobutyl)-5-(2-methoxypropoxy)-6-oxo-1,6-dihydropyridazin-3-yl)-1-methyl-1H-pyrrolo[2,3-c]pyridin-5-yl)acetamide,
Compound 58: (S)-N-(1-cyclopropyl-3-(6-oxo-5-(2-oxa-6-azaspiro[3.4]octan-6-yl)-1-(1,1,1-trifluoropropan-2 -yl)-1,6-dihydropyridazin-3-yl)-1H-pyrrolo[2,3-c]pyridin-5-yl)acetamide,
Compound 59: N-(3-(1-(3,3-difluorobutan-2-yl)-5-isobutoxy-6-oxo-1,6-dihydropyridazin-3-yl)-1-methyl-1H-pyrrolo[2,3-c]pyridin-5-yl)acetamide,
Compound 60: N-(3-(1-(1-ethylcyclobutyl)-5-isobutoxy-6-oxo-1,6-dihydropyridazin-3-yl)-1-methyl-1H-pyrrolo[2,3-c]pyridin-5-yl)acetamide,
Compound 61: (S)-N-(3-(5-(3-methoxyazetidin-1-yl)-6-oxo-1-(1,1,1-trifluoropropan-2-yl)-1,6-dihydropyridazin-3- yl)-1-methyl-1H-pyrrolo[2,3-c]pyridin-5-yl)acetamide,
Compound 62: (S)-N-(3-(5-(2,2-difluoropropoxy)-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 63: N-(1-((1S,3R)-3-cyanocyclobutyl)-3-(5-isobutoxy-6-oxo-1-((S)-1,1,1-trifluoropropan-2-yl)-1,6-dihydropyridazin-3-yl)-1H-pyrrolo[2,3-c]pyridin-5-yl)acetamide,
Compound 64: (S)-N-(1-cyclopropyl-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide,
Compound 65: N-(1-cyclopropyl-3-(5-((R)-2-methoxypropoxy)-6-oxo-1-((S)-1,1,1-trifluoropropan-2-yl)-1,6-dihydropyridazin-3-yl)-1H-pyrrolo[2,3-c]pyridin-5-yl)acetamide,
Compound 66: (S)-N-(1-cyclopropyl-3-(5-(2-methoxyethoxy)-6-oxo-1-(1,1,1-trifluoropropan-2-yl)-1,6-dihydropyridazin-3-yl)-1H-pyrrolo[2,3-c]pyridin-5-yl)acetamide,
Compound 67: (S)-N-(3-(5-(3,3-difluoropyrrolidin-1-yl)-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 68: N-(1-((1S,3R)-3-cyanocyclobutyl)-3-(5-(2,2-difluoropropoxy)-6-oxo-1-((S)-1,1,1-trifluoropropan-2-yl)-1,6-dihydropyridazin-3-yl)-1H-pyrrolo[2,3-c]pyridin-5-yl)acetamide,
Compound 69: (S)-N-(3-(5-(3-methoxyazetidin-1-yl)-6-oxo-1-(1,1,1-trifluoropropan-2-yl)-1,6-dihydropyridazin-3-yl)-1-methyl-1H-pyrrolo[2,3-c]pyridin-5-yl)acetamide-2,2,2-d3,
Compound 70: (S)-N-(3-(5-(2,2-difluoroethoxy)-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 71: (S)-N-(1-cyclopropyl-3-(5-(2,2-difluoroethoxy)-6-oxo-1-(1,1,1-trifluoropropan-2-yl)-1,6-dihydropyridazin-3-yl)-1H-pyrrolo[2,3-c]pyridin-5-yl)acetamide,
Compound 72: (S)-N-(3-(5-(2,2-difluoropropoxy)-6-oxo-1-(1,1,1-trifluoropropan-2-yl)-1,6-dihydropyridazin-3-yl)-1-methyl-1H-pyrrolo[2,3-c]pyridin-5-yl)acetamide-2,2,2-d3,
Compound 73: (S)-N-(3-(5-(2,2-difluoropropoxy)-6-oxo-1-(1,1,1-trifluoropropan-2-yl)-1,6-dihydropyridazin-3-yl)-1-(methyl-d3)-1H-pyrrolo[2,3-c]pyridin-5-yl)acetamide,
Compound 74: (S)-N-(1-cyclopropyl-3-(5-(2,2-difluoropropoxy)-6-oxo-1-(1,1,1-trifluoropropan-2-yl)-1,6-dihydropyridazin-3-yl)-1H-pyrrolo[2,3-c]pyridin-5-yl)acetamide,
Compound 75: (S)-N-(3-(5-(3-isopropoxyazetidin-1-yl)-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 76: (S)-N-(1-ethyl-3-(5-(3-isopropoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide,
Compound 77: (S)-N-(1-cyclopropyl-3-(5-(3-isopropoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide,
Compound 78: (S)-N-(3-(5-(2,2-difluoropropoxy)-6-oxo-1-(1,1,1-trifluoropropan-2-yl)-1,6-dihydropyridazin-3-yl)-1-ethyl-1H-pyrrolo[2,3-c]pyridin-5-yl)acetamide,
Compound 79: (S)-N-(3-(5-(2,2-difluoropropoxy)-6-oxo-1-(1,1,1-trifluoropropan-2-yl)-1,6-dihydropyridazin-3-yl)-1-(2-oxaspiro[3.3]heptan-6-yl)-1H-pyrrolo[2,3-c]pyridin-5-yl)acetamide,
Compound 80: (S)-N-(1-(cyclopropyl methyl)-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide,
Compound 81: (S)-N-(1-((3,3-difluorocyclobutyl)methyl)-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide,
Compound 82: (S)-N-(1-(2-fluoro-2-methylpropyl)-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide,
Compound 83: (S)-N-(3-(5-(3-methoxyazetidin-1-yl)-6-oxo-1-(1,1,1-trifluoropropan-2-yl)-1,6-dihydropyridazin-3-yl)-1-(2-methylallyl)-1H-pyrrolo[2,3-c]pyridin-5-yl)acetamide,
Compound 84: (S)-N-(3-(5-(3-methoxyazetidin-1-yl)-6-oxo-1-(1,1,1-trifluoropropan-2-yl)-1,6-dihydropyridazin-3-yl)-1-(3,3,3-trifluoropropyl)-1H-pyrrolo[2,3-c]pyridin-5-yl)acetamide,
Compound 85: (S)-N-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)acetamide,
Compound 86: (S)-N-(3-(5-(2,2-difluoropropoxy)-6-oxo-1-(1,1,1-trifluoropropan-2-yl)-1,6-dihydropyridazin-3-yl)-1-(2-methoxyethyl)-1H-pyrrolo[2,3-c]pyridin-5-yl)acetamide,
Compound 87: (S)-N-(1-(2-cyanoethyl)-3-(5-(2,2-difluoropropoxy)-6-oxo-1-(1,1,1-trifluoropropan-2-yl)-1,6-dihydropyridazin-3-yl)-1H-pyrrolo[2,3-c]pyridin-5-yl)acetamide,
Compound 88: (S)-N-(1-(3-cyanopropyl)-3-(5-(2,2-difluoropropoxy)-6-oxo-1-(1,1,1-trifluoropropan-2-yl)-1,6-dihydropyridazin-3-yl)-1H-pyrrolo[2,3-c]pyridin-5-yl)acetamide,
Compound 89: (S)-N-(3-(5-(2,2-difluoropropoxy)-6-oxo-1-(1,1,1-trifluoropropan-2-yl)-1,6-dihydropyridazin-3-yl)-1-(3-oxocyclobutyl)-1H-pyrrolo[2,3-c]pyridin-5-yl)acetamide,
Compound 90: (S)-N-(3-(5-(2,2-difluoropropoxy)-6-oxo-1-(1,1,1-trifluoropropan-2-yl)-1,6-dihydropyridazin-3-yl)-1-((3-methyloxetan-3-yl)methyl)-1H-pyrrolo[2,3-c]pyridin-5-yl)acetamide,
Compound 91: (S)-N-(3-(5-(2,2-difluoropropoxy)-6-oxo-1-(1,1,1-trifluoropropan-2-yl)-1,6-dihydropyridazin-3-yl)-1-(2-morpholinoethyl)-1H-pyrrolo[2,3-c]pyridin-5-yl)acetamide,
Compound 92: (S)-N-(1-(cyclopropylmethyl)-3-(5-(2,2-difluoropropoxy)-6-oxo-1-(1,1,1-trifluoropropan-2-yl)-1,6-dihydropyridazin-3-yl)-1H-pyrrolo[2,3-c]pyridin-5-yl)acetamide,
Compound 93: (S)-N-(3-(5-(2,2-difluoropropoxy)-6-oxo-1-(1,1,1-trifluoropropan-2-yl)-1,6-dihydropyridazin-3-yl)-1-(oxetan-3-ylmethyl)-1H-pyrrolo[2,3-c]pyridin-5-yl)acetamide,
Compound 94: N-(3-(5-(2,2-difluoropropoxy)-6-oxo-1-((S)-1,1,1-trifluoropropan-2-yl)-1,6-dihydropyridazin-3-yl)-1-((R)-tetrahydrofuran-3-yl)-1H-pyrrolo[2,3-c]pyridin-5-yl)acetamide,
Compound 95: (S)-N-(1-(6,6-difluorospiro[3.3]heptan-2-yl)-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide,
Compound 96: (S)-N-(3-(5-(2,2-difluoropropoxy)-6-oxo-1-(1,1,1-trifluoropropan-2-yl)-1,6-dihydropyridazin-3-yl)-1-propyl-1H-pyrrolo[2,3-c]pyridin-5-yl)acetamide,
Compound 97: (S)-N-(3-(5-(2,2-difluoropropoxy)-6-oxo-1-(1,1,1-trifluoropropan-2-yl)-1,6-dihydropyridazin-3-yl)-1-(7-oxaspiro[3.5]nonan-2-yl)-1H-pyrrolo[2,3-c]pyridin-5-yl)acetamide,
Compound 98: (S)-N-(1-(cyclopropanecarbonyl)-3-(5-(2,2-difluoropropoxy)-6-oxo-1-(1,1,1-trifluoropropan-2-yl)-1,6-dihydropyridazin-3-yl)-1H-pyrrolo[2,3-c]pyridin-5-yl)acetamide,
Compound 99: (S)-N-(1-(2,2-difluoroethyl)-3-(5-(2,2-difluoropropoxy)-6-oxo-1-(1,1,1-trifluoropropan-2-yl)-1,6-dihydropyridazin-3-yl)-1H-pyrrolo[2,3-c]pyridin-5-yl)acetamide,
Compound 100: N-(3-(5-(2,2-difluoropropoxy)-6-oxo-1-((S)-1,1,1-trifluoropropan-2-yl)-1,6-dihydropyridazin-3-yl)-1-(3-fluorocyclobut-2-en-1-yl)-1H-pyrrolo[2,3-c]pyridin-5-yl)acetamide,
Compound 101: (S)-N-(3-(5-(2,2-difluoropropoxy)-6-oxo-1-(1,1,1-trifluoropropan-2-yl)-1,6-dihydropyridazin-3-yl)-1-isopropyl-1H-pyrrolo[2,3-c]pyridin-5-yl)acetamide,
Compound 102: (S)-N-(1-ethyl-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide,
Compound 103: (S)-N-(3-(5-(2-fluoro-2-methylpropoxy)-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 104: (S)-N-(1-methyl-3-(6-oxo-5-(2,2,2-trifluoroethoxy)-1-(1,1,1-trifluoropropan-2-yl)-1,6-dihydropyridazin-3-yl)-1H-pyrrolo[2,3-c]pyridin-5-yl)acetamide,
Compound 105: (S)-N-(1-ethyl-3-(5-(2-fluoro-2-methylpropoxy)-6-oxo-1-(1,1,1-trifluoropropan-2-yl)-1,6-dihydropyridazin-3-yl)-1H-pyrrolo[2,3-c]pyridin-5-yl)acetamide,
Compound 106: (S)-N-(1-ethyl-3-(6-oxo-5-(2,2,2-trifluoroethoxy)-1-(1,1,1-trifluoropropan-2-yl)-1,6-dihydropyridazin-3-yl)-1H-pyrrolo[2,3-c]pyridin-5-yl)acetamide,
Compound 107: (S)-N-(1-cyclopropyl-3-(6-oxo-5-(2,2,2-trifluoroethoxy)-1-(1,1,1-trifluoropropan-2-yl)-1,6-dihydropyridazin-3-yl)-1H-pyrrolo[2,3-c]pyridin-5-yl)acetamide,
Compound 108: (S)-N-(1-ethyl-3-(5-(3-fluoropropoxy)-6-oxo-1-(1,1,1-trifluoropropan-2-yl)-1,6-dihydropyridazin-3-yl)-1H-pyrrolo[2,3-c]pyridin-5-yl)acetamide,
Compound 109: (S)-N-(3-(5-(3-fluoropropoxy)-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 110: (S)-N-(1-cyclopropyl-3-(5-(2-fluoro-2-methylpropoxy)-6-oxo-1-(1,1,1-trifluoropropan-2-yl)-1,6-dihydropyridazin-3-yl)-1H-pyrrolo[2,3-c]pyridin-5-yl)acetamide,
Compound 111: N-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)acetamide,
Compound 112: N-(1-cyclopropyl-3-(5-(2,2-difluoroethoxy)-6-oxo-1-(1,1,1-trifluoropropan-2-yl)-1,6-dihydropyridazin-3-yl)-1H-pyrrolo[2,3-c]pyridin-5-yl)acetamide,
Compound 113: (R)-N-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)acetamide,
Compound 114: (R)-N-(1-cyclopropyl-3-(5-(2,2-difluoroethoxy)-6-oxo-1-(1,1,1-trifluoropropan-2-yl)-1,6-dihydropyridazin-3-yl)-1H-pyrrolo[2,3-c]pyridin-5-yl)acetamide,
Compound 115: (S)-N-(3-(5-(2,2-difluoroethoxy)-6-oxo-1-(1,1,1-trifluoropropan-2-yl)-1,6-dihydropyridazin-3-yl)-1H-pyrrolo[2,3-c]pyridin-5-yl)acetamide,
Compound 116: (S)-N-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)-2-hydroxyacetamide, and
Compound 117: (S)-1-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)-3-methylurea.

Unless otherwise specified, Formulas or names given in the specification and claims encompasses tautomers and all stereoscopic, optical, and geometric isomers (e.g., enantiomers, diastereomers, E/Z isomers, etc.) and racemates thereof, as well as mixtures of individual enantiomers in different ratios, mixtures of diastereomers, or any of the above arbitrary forms including isomers and enantiomers, pharmaceutically acceptable salts thereof, such as hydrates including, for example, solvates and hydrates of free compounds, and solvates and hydrates or salts of such the compounds.

In an embodiment, the compound of the present disclosure may be present in a form of a pharmaceutically acceptable salt. As the salt, an acid addition salt formed by a pharmaceutically acceptable free acid is useful. The term "pharmaceutically acceptable salt" of the present disclosure means any organic or inorganic addition salt of the compound which has a concentration that is relatively non-toxic and harmless to a patient and of which side effects do not degrade a beneficial efficacy of the compound according to the present disclosure.

The acid addition salt is prepared by a conventional method, for example, by dissolving the compound in an excess of aqueous acid solution and precipitating this salt using a water-miscible organic solvent such as methanol, ethanol, acetone or acetonitrile. An acid or alcohol (e.g., glycol monomethyl ether) in the same molar amount of the compound and water may be heated, and then the mixture may be evaporated to dry, or the precipitated salt may be suction filtered.

In this case, an organic acid and an inorganic acid may be used as free acids, hydrochloric acid, phosphoric acid, sulfuric acid, nitric acid, tartaric acid, etc. may be used as the inorganic acid, and methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, etc. may be used as the organic acid, but are not limited thereto.

In addition, a base may be used to prepare a pharmaceutically acceptable metal salt. Alkali metal salt or alkaline earth metal salt is obtained, for example, by dissolving a compound in an excess alkali metal hydroxide or alkaline earth metal hydroxide solution, filtering out undissolved compound salt, and then evaporating and drying the filtrate. In this case, it is pharmaceutically suitable to prepare sodium, potassium, or calcium salts in particular as the metal salt, but is not limited thereto. In addition, a corresponding silver salt may be obtained by reacting alkaline metal or alkaline earth metal salt with an appropriate silver salt (e.g., silver nitrate).

The pharmaceutically acceptable salt of the compounds of the present disclosure includes salts of an acidic or basic group which may be present in the compounds of Formula 1 unless otherwise indicated. For example, the pharmaceutically acceptable salt may include sodium, calcium and potassium salt, etc. of the hydroxy group, and other pharmaceutically acceptable salt of amino group includes hydrobromide, sulfate, hydrogen sulfate, phosphate, hydrogen phosphate, dihydrogen phosphate, acetate, succinate, citrate, tartrate, lactate, mandelate, methanesulfonate (mesylate) and p-toluenesulfonate (tosylate) salts, etc., which may be prepared by a preparing method of salts known in the art. In a specific embodiment, the pharmaceutically acceptable salt of the compound of the present disclosure may be a hydrochloride.

The salt of the compound described in Formula 1 of the present disclosure is a pharmaceutically acceptable salt, and any salt of the compound described in Chemical Formula 1 exhibiting pharmacological activity equivalent to that of the compound of Chemical Formula 1 may be used without limitation.

In addition, the compound described in Formula 1 according to the present disclosure includes, without limitation, not only a pharmaceutically acceptable salt thereof, but also a solvate such as possible hydrates that may be prepared therefrom and all possible stereoisomers. All stereoisomers of the present disclosure, including an enantiomer form and a diastereomer form (e.g., those which may exist due to an asymmetric carbon in various substituents), are included within the scope of the present disclosure. An individual stereoisomer of the compound of the present disclosure may be, for example, substantially other isomers (e.g., as a pure or substantially pure optical isomer having a specific activity), or may be mixed with, for example, a racemate, all other, or other selected stereoisomers. A chiral center of the compound of the present disclosure may have an S or R structure defined by the IUPAC 1974 recommendation. A racemic form may be analyzed by physical methods such as separation by a chiral column chromatography, separation or crystallization of diastereomer derivatives, or fractional crystallization. An individual optical isomer may be obtained from the racemate by any suitable method including crystallization following salt formation with an optically active acid, but is not limited thereto.

A solvate and a stereoisomer of the compound of Chemical Formula 1 may be prepared from the compound using the methods known in the art.

Furthermore, the compound of Chemical Formula 1 according to the present disclosure may be prepared in a crystalline form or in a non-crystalline form and may be optionally hydrated or solvated when prepared in the crystalline form. The present disclosure may include compounds containing various amounts of water as well as a stoichiometric hydrate of the compound described in Formula 1. The solvate of the compound of Chemical Formula 1 according to the present disclosure includes both a stoichiometric solvate and a non-stoichiometric solvate.

In a specific embodiment according to the present disclosure, activities of TYK2 JH2, JAK1 JH1, JAK2 JH1, JAK3 JH1, and TYK2 JH1 enzyme were evaluated for the compound represented by Formula 1 according to the present disclosure, and their excellent activity and selectivity were confirmed. In addition, it showed excellent activity of IC₅₀ < 50 nM in TYK2/JAK1 cell activity.

Therefore, the compound and the pharmaceutically acceptable salt thereof according to the present disclosure may be effectively used for prevention, improvement or treatment of TYK2-related diseases, specifically autoimmune diseases, inflammatory diseases, or cancers.

### Preparing method

In another embodiment, the present disclosure relates to a preparing method of the novel compound.

A compound of Chemical Formula 1 may be synthesized by a synthetic pathway described herein. In certain embodiments, methods well known in the art of chemistry or based on the disclosures disclosed herein may be used. Generally, a starting material is readily prepared using methods which are available from a commercial source or well known to those skilled in the art.

The compounds of the present disclosure may be prepared from a commercially available starting material using a general method exemplified herein. For illustrative purposes, Reaction Schemes 1 to 5 illustrated below provide a pathway for synthesizing major intermediates as well as the compound of Chemical Formula 1. The following Examples will be referred to for a more detailed description of the individual reaction steps. Those skilled in the art will recognize that other synthetic pathways may be available and used. Although specific starting materials and reagents are illustrated and in the Reaction Formulas and described below, other starting materials and reagents may be substituted to provide various derivatives and/or reaction conditions. Additionally, many compounds prepared by the methods described below may be further modified on the basis of the present disclosure using conventional chemicals known to those skilled in the art.

In a specific embodiment, the preparing method includes
a step 1 of preparing a compound represented by Chemical Formula 1 by borylating a compound represented by a following Formula 3 and then performing a Suzuki reaction or Ullmann reaction with a compound corresponding to Chemical Formula 2.

As a specific example, in the step 1, the compound represented by Chemical Formula 3 is dissolved in 1,4-dioxane, and then bis(pinacolato)diboron, potassium acetate, Pd(dba)₂, and 0.6 M PCy₃ in toluene are used as reaction reagents. After stirring at 90~110°C for 1 hour to overnight, filtering is performed. After dissolving the reactant and the compound represented by Formula 2 in 1,4-dioxane and water, Pd(dppf)₂Cl₂·DCM and K₃PO₄ are used as reaction reagents. After stirring at 70~80°C for 1 hour to overnight, a compound of Chemical Formula 1 is prepared.

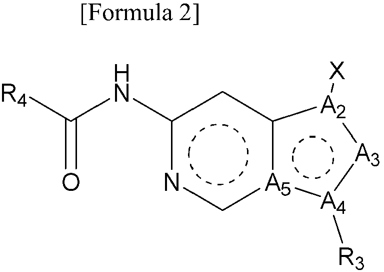

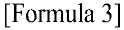

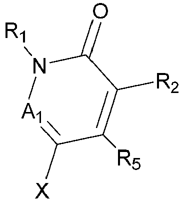

In the above formula,
A₁, A₂, A₃, A₄, A₅, R₁, R₂, R₃, R₄, and R₅ are as defined in Chemical Formula 1,
and X is halo, preferably fluoro, chloro, bromo or iodo.

Specifically, the preparing method may be represented by the following Reaction Scheme 1.

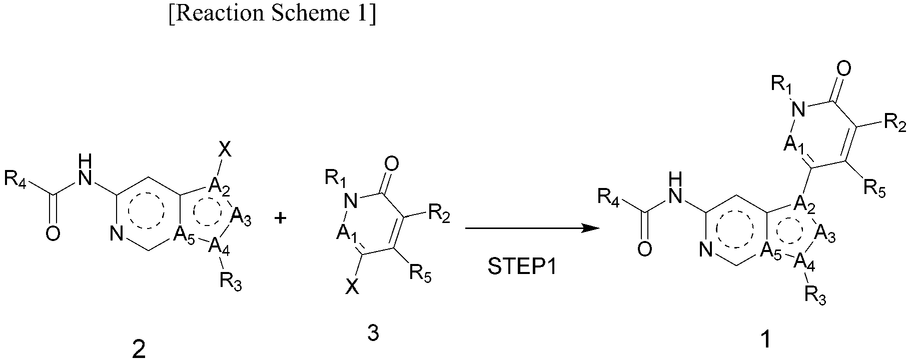

In the Reaction Scheme,
A₁, A₂, A₃, A₄, A₅, R₁, R₂, R₃, R₄, and R₅ are as defined in Chemical Formula 1,
and X is halo, preferably fluoro, chloro, bromo or iodo.

The compound of Chemical Formula 2 may be prepared by a preparing method including the following steps:
a step 2 of preparing a compound represented by a following Chemical Formula 5 by reacting a compound represented by the following Formula 4 with NBS or NIS;
a step 3 of preparing a compound represented by the following Chemical Formula 6 by reacting the compound represented by the following Chemical Formula 5 to S_{N}2 reaction or Chan-Lam reaction;
a step 4 of preparing the compound represented by Chemical Formula 2 by deacetylating the compound represented by a following Chemical Formula 6 with an acid, for example, hydrochloric acid, and then reacting the same with isocyanate or acetyl chloride.

As a specific example, in the step 2, the compound represented by Chemical Formula 4 is dissolved in an appropriate solvent, for example, N,N-dimethylformamide, and then NBS or NIS is added dropwise as a halogen donor and stirred at room temperature overnight.

As a specific example, in the step 3, the compound represented by Formula 5 is dissolved in acetonitrile or N,N-dimethylformamide, and then cesium carbonate is used as a base, and dimethyl sulfate or halogenated alkyl or mesyl alcohol is used as a reaction reagent. The compound of Chemical Formula 6 is prepared by stirring at room temperature or 80°C overnight. Mesyl alcohol is prepared by dissolving alcohol corresponding to R₃ in dimethyl chloride, and then adding triethylamine and methanesulfonyl chloride dropwise at 0°C and stirring at room temperature for 2 to 4 hours. Alternatively, R₃B(OH)₂, 2,2'-bipyridyl, anhydrous copper (II) acetate, and sodium carbonate are used as reagents for the Chan-Lam reaction, and N,N-dimethylformamide is used as a solvent. The compound of Chemical Formula 6 is prepared by stirring at 70°C overnight.

As a specific example, in the step 4, the compound represented by Chemical Formula 6 is dissolved in methanol, and then 4N HCl in 1,4-dioxane is added dropwise and stirred at 80°C for 5 hours. A reactant is concentrated and dissolved in dimethyl chloride, then N,N-diisopropylethylamine and 2,2,2-trichloroacetyl isocyanate are added dropwise at 0°C. The reactant is stirred at room temperature for 30 minutes and then concentrated. After dissolving the reactant in methanol, saturated sodium bicarbonate aqueous solution is added dropwise. The compound of Chemical Formula 2 is prepared by stirring at room temperature for 1 to 4 hours.

Specifically, the preparing method may be represented by the following Reaction Scheme 2.

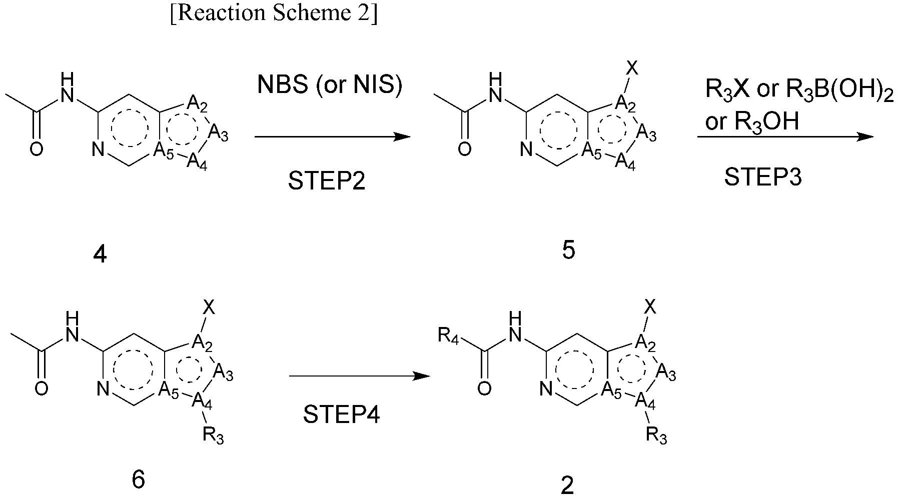

In the above Reaction Scheme,
A₂, A₃, A₄, A₅, R₃ and R₄ are as defined in Chemical Formula 1,
X is halo, preferably fluoro, chloro, bromo or iodo.

As a specific example, the compound of Chemical Formula 3, wherein R₅ is H and R₂ is 4-12 membered heterocycloalkyl, OR' or NR'R", may be prepared by a preparing method including the following steps:
a step 5 of preparing a compound represented by Chemical Formula 8 by the Mitsunobu reaction or S_{N}2 reaction from a compound represented by the following Chemical Formula 7; and
a step 6 of preparing a compound represented by Chemical Formula 3 by reacting a compound represented by the following Chemical Formula 8 with NaHMDS which is a disilicide compound, and an alcohol compound corresponding to R₂, for example, HOR', or an amine compound corresponding to R₂, for example, a HNR'R" compound.

As a specific example, a compound represented by Chemical Formula 7 is dissolved in N,N-dimethylformamide and then cesium carbonate and halogenated alkyl are added dropwise in the fifth step. The compound of Chemical Formula 8 is prepared by stirring at room temperature overnight. Alternatively, the compound represented by Chemical Formula 7 is dissolved in tetrahydrofuran, and then R₁OH, PhOPPh₂ or PPh₃, and DIAD or DEAD are added dropwise and stirred at room temperature overnight to prepare the compound of Chemical Formula 8.

As a specific example, the following step 6 may be performed by dissolving the compound represented by Chemical Formula 8 in 1,4-dioxane or tetrahydrofuran, and then adding 2N NaHMDS (2N NaHMDS in tetrahydrofuran) in tetrahydrofuran and an alcohol (HOR') or amine (HNR'R") corresponding to R₂ dropwise. The compound of Chemical Formula 3 is prepared by stirring at room temperature or 50°C for 1 to 4 hours.

As a specific example, a preparing method of a compound of Chemical Formula 3, in which R⁵ is H and R₂ is 4-12-membered heterocycloalkyl, OR' or NR'R" may be represented by the following Reaction Scheme 3.

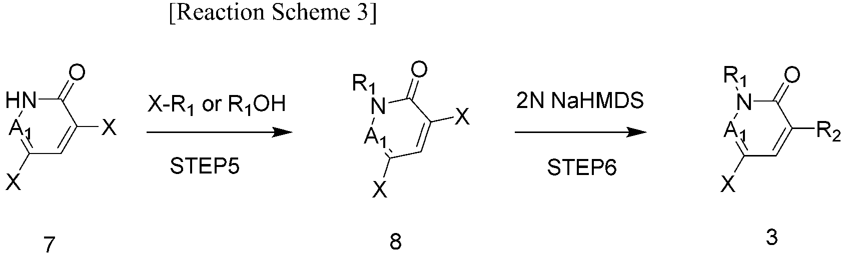

In the above Reaction Scheme,
A₁, R₁ and R₂ are as defined in Chemical Formula 1,
X is halo, preferably fluoro, chloro, bromo or iodo.

As a specific example, the compound of Chemical Formula 3, wherein R₅ is H, R₂ is H, C₁-C₁₀alkyl, C₁-C₁₀alkoxy, or C₃-C₈cycloalkyl, may be prepared by a preparing method including the following steps:
A step 7 of preparing a compound represented by Chemical Formula 8 by Mitsunobu reaction or S_{N}2 reaction from a compound represented by the following Chemical Formula 9.

As a specific example, a compound represented by Chemical Formula 9 is dissolved in N,N-dimethylformamide and then cesium carbonate and halogenated alkyl are added dropwise in the seventh step. The compound of Chemical Formula 8 is prepared by stirring at room temperature overnight. Alternatively, the compound represented by Formula 9 is dissolved in tetrahydrofuran, and then R₁OH, PhOPPh₂ or PPh₃, and DIAD or DEAD are added dropwise and stirred at room temperature overnight to prepare the compound of Chemical Formula 8.

As a specific example, a preparing method of the compound of Chemical Formula 3 in which R₅ is H, R₂ is H, C₁-C₁₀alkyl, C₁-C₁₀alkoxy or C₃-C₈cycloalkyl may be represented by the following Reaction Scheme 4.

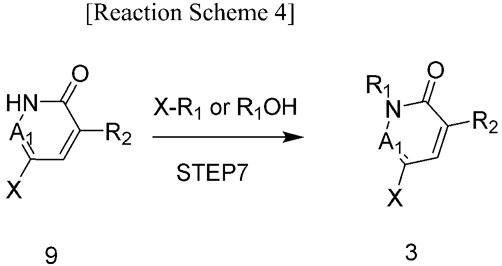

In the above Reaction Scheme,
A₁, R₁ and R₂ are as defined in Chemical Formula 1, and
X is halo, preferably fluoro, chloro, bromo or iodo.

As a specific example, the compound of Chemical Formula 1 may also be manufactured by a preparing method including the following steps:
a step 1' of borylating the compound of Chemical Formula 3, then performing a Suzuki reaction or Ullmann reaction with the compound of Chemical Formula 6 to obtain a compound of Chemical Formula 10;
a step 2' of deacetylating the compound of Chemical Formula 10 with an acid, for example, hydrochloric acid, and then reacting it with acetoxy acetyl chloride or methylamine to obtain a compound of Chemical Formula 1.

As a specific example, in the above step 1', the compound of Chemical Formula 3 is dissolved in 1,4-dioxane, and then bis(pinacolato)diboron, potassium acetate, Pd(dba)₂, and 0.6 M PCy3 in toluene are used as reaction reagents. After stirring at 90~110°C for 1 hour to overnight, filter. After dissolving the reactant and the compound represented by Formula 6 in 1,4-dioxane and water, use Pd(dppf)₂Cl₂.DCM and K₃PO₄ as reaction reagents. After stirring at 70~80°C for 1 hour to overnight, the compound of Chemical Formula 10 is prepared.

As a specific example, in the step 2', the compound of Chemical Formula 10 synthesized in the step 1' is dissolved in methanol, 4N HCl in 1,4-dioxane is added dropwise, and the mixture is stirred at 80°C for 5 hours. The reactant is concentrated and dissolved in dimethyl chloride, and acetoxyacetyl chloride and pyridine are added dropwise. The mixture is stirred at room temperature for 3 hours and concentrated. The reactant is dissolved in methanol, and potassium carbonate is added dropwise. The mixture is stirred at room temperature for 15 minutes to obtain a compound of Chemical Formula 1. Alternatively, the deacetylated reactant is concentrated and dissolved in tetrahydrofuran, and triphosgene is added dropwise at 0°C and stirred for 1 hour. Methylamine is added dropwise to the reactant at room temperature and stirred to obtain a compound of Chemical Formula 1.

Specifically, the above-mentioned manufacturing method may be represented by the following Reaction Scheme 5.

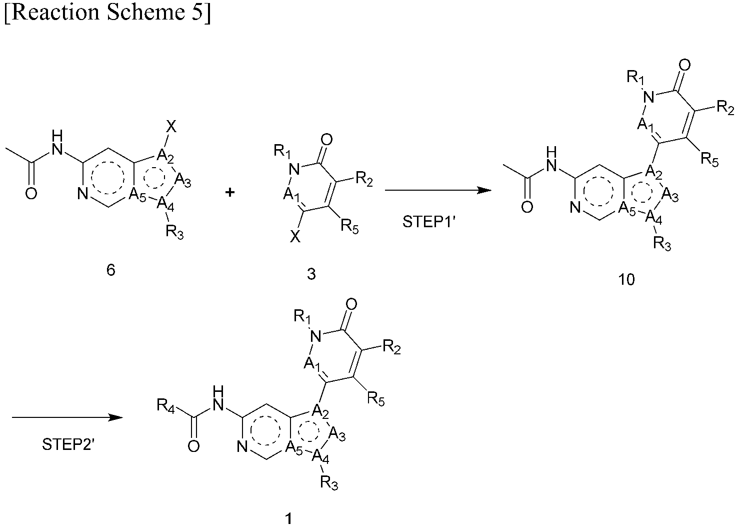

In the above Reaction Scheme,
A₁, A₂, A₃, A₄, A₅, R₁, R₂, R₃, R₄, and R₅ are as defined in the above Chemical Formula 1, and
X is halo, preferably fluoro, chloro, bromo, or iodo.

### Pharmaceutical Composition and Medicinal Use

The compound of the present disclosure, or the pharmaceutically acceptable salt thereof, may be suitable for prevention, improvement, or treatment of various diseases caused by TYK2 activity due to their biological properties.

Therefore, in another embodiment, the present disclosure relates to a pharmaceutical composition for preventing, improving, or treating various diseases related to TYK2 including a compound of Chemical Formula 1, or a pharmaceutically acceptable salt thereof, and a pharmaceutically acceptable carrier.

TYK2-related diseases may include any disease, disorder or condition directly, or indirectly linked to an expression or activity of TYK2 including overexpression and/or abnormal activity level. Abnormal activity level may be determined by comparing an activity level in a normal healthy tissue or cell to an activity level in a diseased cell. Diseases mediated by TYK2 may also include any disease, disorder, or condition that may be prevented, improved, inhibited, or cured by regulating TYK2 activity.

The TYK2-mediated disease that may be treated using a compound of the present disclosure may be an autoimmune disease, an inflammatory disease, cancer, or a neurodegenerative disease.

In a specific embodiment, the autoimmune and the inflammatory diseases may be one or more selected from a group consisting of asthma, alopecia areata, ankylosing spondylitis, lupus, lupus nephritis, crohn's disease, ulcerative colitis, rheumatoid arthritis, psoriasis (including plaque, scalp, and nail psoriasis), sjogren's syndrome, multiple sclerosis, palmar pustulosis, celiac disease, atopic dermatitis, contact dermatitis, delayed hypersensitivity, treatment of eye disease and conditions such as ocular allergies, conjunctivitis, dry keratoconjunctivitis, and vernal conjunctivitis, diseases affecting a nose including allergic rhinitis, interstitial pulmonary fibrosis, psoriatic arthritis, systemic juvenile idiopathic arthritis, cryopyrin-associated periodic syndrome, nephritis, vasculitis, diverticulitis, interstitial cystitis, glomerulonephritis, endometritis, leptospirosis, kidney disease, cardiac hypertrophy, muscle wasting, catabolic disorders, fetal growth retardation, hypercholesterolemia, heart disease, chronic heart failure, mesothelioma, anhidrotic ecodermal dysplasia, Behcet's disease, incontinentia pigmenti, pancreatitis, hereditary periodic fever syndrome, asthma, acute lung injury, acute respiratory distress syndrome, eosinophilia, hypersensitivity, anaphylaxis, nasal sinusitis, ocular allergy, silica-induced disease, chronic obstructive pulmonary disease, cystic fibrosis, acid-induced lung injury, pulmonary hypertension, COVID19, cataracts, muscle inflammation with systemic sclerosis, inclusion body myositis, myasthenia gravis, thyroiditis, addison's disease, lichen planus, type 1 diabetes, or type 2 diabetes, temporal arteritis, tendonitis, dermatomyositis, appendicitis, bronchiolitis, bronchitis, bursitis, cervicitis, cholangitis, cholecystitis, chronic graft rejection, colitis, conjunctivitis, cystitis, dacryoadenitis, encephalitis, endocarditis, endometritis, enteritis, enterocolitis, epicondylitis, epididymitis, fasciitis, fibrositis, gastritis, Henoch-Schonlein purpura, hepatitis, hidradenitis suppurativa, leukocyte adhesion deficiency, immunoglobulin A nephropathy, interstitial lung disease, laryngitis, mastitis, meningitis, myelitis, myocarditis, myositis, nephritis, oophoritis, orchitis, osteitis, otitis, pancreatitis, parotitis, pericarditis, peritonitis, pharyngitis, pleuritis, phlebitis, pneumonia, polymyositis, proctitis, prostatitis, pyelonephritis, rhinitis, salpingitis, sinusitis, stomatitis, synovitis, tendonitis, tonsillitis, uveitis, vaginitis, vasculitis, and vulvitis, but are not limited thereto.

In a specific embodiment, the cancer may be one or more selected from a group consisting of breast cancer, ovarian cancer, cervical cancer, prostate cancer, testicular cancer, penile cancer, genitourinary cancer, seminoma, esophageal cancer, laryngeal cancer, gastric cancer, stomach cancer, gastrointestinal cancer, skin cancer, keratoacanthoma, follicular carcinoma, melanoma, lung cancer, small cell lung carcinoma, non-small cell lung carcinoma (NSCLC), lung adenocarcinoma, lung squamous cell carcinoma, adenomatous polyp, colon cancer, pancreatic cancer, thyroid cancer, papillary cancer, bladder cancer, liver cancer, biliary tract cancer, renal cancer, bone cancer, bone marrow disease, lymphatic disease, trichoblastoma, oral cancer and pharyngeal cancer (oral), lip cancer, tongue cancer, oral cancer, salivary gland cancer, pharyngeal cancer, small intestine cancer, colon cancer, rectal cancer, anal cancer, renal cancer, prostate cancer, vulvar cancer, thyroid cancer, colon cancer, endometrial cancer, cervical cancer, brain cancer, central nervous system cancer, peritoneum cancer, hepatocellular carcinoma, head cancer, neck cancer, hopkins disease and leukemia (acute myeloid leukemia), but is not limited thereto.

In a specific embodiment, the neurodegenerative disease may be one or more diseases selected from a group consisting of alzheimer's disease, parkinson's disease, and amyotrophic lateral sclerosis, but is not limited thereto.

All the above-mentioned cancers characterized by a specific location/organ in a body are meant to include both primary tumors and metastatic tumors derived therefrom. All the above-mentioned cancers may be further distinguished by their histopathological classification:
Terms "subject" or "patient" used compatibly refer to any animal including a mammal, preferably a mouse, rat, other rodent, rabbit, dog, cat, pig, cow, sheep, horse or primate, and most preferably a human.
The term "therapeutically effective amount" refers to an amount of an active compound or a pharmaceutical agent that causes a biological or medical response in a tissue, system, animal, subject, or human being sought by a researcher, veterinarian, doctor, or other clinician.
The term "treating" or "treatment" refers to one or more of (1) inhibiting a disease; for example, inhibiting a disease, condition or disorder in a subject experiencing or exhibiting a pathology or a symptom of a disease, condition or disorder (i.e., preventing additional development of the pathology and/or symptom); and (2) improving a disease; for example, improving a disease, condition or disorder in a subject experiencing or exhibiting a pathology or a symptom of a disease, condition or disorder (i.e., reversing the pathology and/or symptom), for example, reducing severity of the disease. In one embodiment, treating or treatment includes preventing or reducing a risk of developing a disease; for example, preventing or reducing the risk of developing a disease, condition or disorder in an individual who may have a predisposition to the disease, condition, or disorder, but who has not yet experienced or exhibited the pathology or symptom of the disease.

In a specific embodiment, a compound according to the present disclosure, or a pharmaceutically acceptable salt thereof, and a pharmaceutical composition including the same may be used together with non-drug therapies for diseases associated with TYK2 activity known in the art. For example, it may be used for prevention, short-term or long-term treatment of the above-mentioned diseases, in combination with radiotherapy and/or surgery and/or other compounds.

The pharmaceutical composition of the present disclosure may be administered to a mammal such as a rat, a mouse, a livestock, and a human via various routes. In the present disclosure, the administration route of the pharmaceutical composition includes oral, intravenous, intramuscular, intraarterial, intramedullary, intradural, intracardiac, transdermal, subcutaneous, intraperitoneal, intranasal, intestinal, topical, sublingual, or rectal route, but is not limited thereto.

In addition, oral and parenteral administration are preferable as methods of administration of the pharmaceutical composition. As used herein, the term "parenteral" includes techniques of injecting or infusing subcutaneous, intradermal, intravenous, intramuscular, intraarticular, intrabursal, intrasternal, intradural, intralesional, and intracranial.

The pharmaceutical composition according to the present disclosure may further include one or more pharmaceutically acceptable carriers, one or more excipients, and/or a diluent.

Non-limiting examples of pharmaceutically suitable carriers include a solid and/or liquid, such as ethanol, glycerol, water, etc. An amount of carrier in the therapeutic composition may range from about 5 to about 99 wt.% based on a total weight of the therapeutic composition or therapeutic combination. Non-limiting examples of suitable pharmaceutically acceptable excipients and diluents include a non-toxic compatible filler, a binder, a disintegrating agent, a buffer, a preservative, a wetting agent, an extender, an antioxidant, a lubricant, a flavoring agent, a thickening agent, a colorant, a surfactant, an emulsifier, a suspending agent, etc. Such excipients and diluents may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate and mineral oil, but are not limited thereto, and it will be apparent to those skilled in the art that all other pharmaceutically acceptable carriers, excipients and diluents may be used.

A composition including the compound of Chemical Formula 1 of the present disclosure or a salt thereof may be formulated and used in a form of oral formulations such as a tablet, a powder, a granule, a pill, a capsule, a suspension, an emulsion, a solution, an emulsifiable concentrate, a syrup, etc., and an external preparation, a suppository, or a sterile injection solution according to a conventional method.

The pharmaceutical composition according to the present disclosure may be in a form of a sterile injectable preparation as a sterile injectable aqueous or oil-based suspension. The suspension may be formulated according to techniques known in the art using a suitable dispersant or a wetting agent (e.g., Tween 80) and a suspending agent. The sterile injectable preparation may also be a sterile injection solution or a suspension (e.g., a solution in 1,3-butanediol) among a non-toxic parenterally acceptable diluent or solvent. Acceptable vehicles and solvents include mannitol, water, Ringer's solution, or isotonic sodium chloride solution. In addition, a sterile non-volatile oil is commonly used as the solvent or suspending medium. For this purpose, any non-volatile oil having less irritation may be used by including synthetic mono- or diglyceride. Natural oil (e.g., olive oil or castor oil) in which fatty acids such as an oleic acid and a glyceride derivative thereof are pharmaceutically acceptable, and especially polyoxyethylated ones thereof are useful for injection preparations.

The pharmaceutical composition according to the present disclosure may be orally administered in any orally acceptable dosage, including a capsule, a tablet, and an aqueous suspension and solution, but is not limited thereto.

The pharmaceutical composition of the present disclosure may also be administered in a form of a suppository for rectal administration. Such compositions may be prepared by mixing the compound of the present disclosure with a suitable non-irritating excipient that is solid at room temperature but liquid at rectal temperature. Such substances include cocoa butter, beeswax, and polyethylene glycol, but are not limited thereto.

Oral administration of the pharmaceutical composition according to the present disclosure is particularly useful when a desired treatment is related to a site or organ that is easily accessible by topical application. When topically applying to the skin, the pharmaceutical composition should be formulated into a suitable ointment containing an active ingredient that is suspended or dissolved in a carrier. Carriers for topical administration of the compound of the present disclosure include mineral oil, liquid paraffin, white petrolatum, propylene glycol, polyoxyethylene, polyoxypropylene compound, emulsion wax, and water, but are not limited thereto. Alternatively, the pharmaceutical composition may be formulated into a suitable lotion or cream containing an active compound that is suspended or dissolved in a carrier. Suitable carriers include mineral oil, sorbitan monostearate, polysorbate 60, cetyl ester wax, cetearyl alcohol, 2-octyldodecanol, benzyl alcohol, and water, but are not limited thereto. The pharmaceutical composition of the present disclosure may also be topically applied to a lower intestinal tract as a suitable enema by a rectal suppository. A topically applied transdermal patch is also included in the present disclosure.

The pharmaceutical composition of the present disclosure may be administered by nasal aerosol or inhalation. Such compositions may be prepared according to techniques well known in the medicament field and may be prepared as a solution in saline using benzyl alcohol or other suitable preservatives, an absorption promoter for enhancing bioavailability, fluorocarbon, and/or other solubilizers or dispersants known in the art.

The pharmaceutical composition of the present disclosure contains the new compound in a therapeutically effective amount or a prophylactically effective amount. A preferred dosage of the compound according to the present disclosure may vary according to a patient's condition and weight, degree of disease, drug form, administration route and period, but may be appropriately selected by those skilled in the art. However, for a preferable effect, the compound of Chemical Formula 1 of the present disclosure may be administered in a dose of 0.0001 to 1000 mg/kg, preferably 0.01 to 500 mg/kg once or several times a day.

The compound of Chemical Formula 1 among the compositions of the present disclosure may be blended in an amount of 0.0001 to 50 wt.% based on a total weight of the entire composition.

In another embodiment, the present disclosure relates to a method for preventing, improving or treating various diseases caused by an activity of TYK2, including a compound of Chemical Formula 1 or a pharmaceutically acceptable salt thereof, and a pharmaceutically acceptable carrier.

In this case, the compound of Chemical Formula 1 of the present disclosure or the pharmaceutically acceptable salt thereof, is administered in a pharmaceutically effective amount. The term "pharmaceutically effective amount" of the present disclosure means an amount sufficient to treat a disease at a reasonable benefit/risk ratio applicable to medical treatment and not to cause side effects, and an effective dosage level may be determined according to factors including a patient's health condition, type and severity of a disease, activity of a drug, sensitivity to a drug, administration method, time of administration, route of administration, excretion rate, duration of treatment, and drugs blended or used simultaneously, and other factors well known in the medical field. The composition of the present disclosure may be administered as an individual therapeutic agent or in combination with other therapeutic agents, may be administered sequentially or simultaneously with a conventional therapeutic agent, and may be administered in single or in multiple doses. It is important to administer an amount to obtain the maximum effect with a minimum amount without side effects in consideration of all of the factors described above, which may easily be determined by those skilled in the art.

For example, since the dosage may be increased or decreased depending on the route of administration, severity of disease, gender, weight, age, etc., the dosage does not limit the scope of the present disclosure in any way.

A preferred dosage of the compound according to the present disclosure may vary according to a patient's condition and weight, degree of disease, drug form, administration route and period, but may be appropriately selected by those skilled in the art.

### Combination Therapy

The compound of the present disclosure, or a pharmaceutically acceptable salt thereof, and a pharmaceutical composition including the same may be used alone or in combination with one or more additional therapies (e.g., non-drug treatments or therapeutic agents).

The combination therapy may, for example, combine two therapies, or may combine three therapies (e.g., triple therapy of three therapeutic agents) or more. Dosage of one or more of the additional therapies (e.g., non-drug treatments or therapeutic agents) may be reduced from standard dosage when administered alone.

The compound of the present disclosure, or a pharmaceutically acceptable salt thereof, and a pharmaceutical composition including the same may be administered before, after, or simultaneously with one or more of these additional therapies.

In a specific embodiment, the compound of Chemical Formula 1 may be combined with an additional therapeutic compound having an anti-inflammatory or anti-hyperproliferative property, or useful for treatment of an autoimmune disease, an inflammatory disease or cancer. The additional therapeutic agent may be an NSAID or another anti-inflammatory agent. The additional therapeutic agent may be a chemotherapeutic agent.

In a specific embodiment, the compound of the present disclosure is administered with any anti-IBD reagent, including an anti-inflammatory drug such as sulfasalazine, mesalamine or a corticosteroid such as budesonide, prednisone, cortisone or hydrocortisone, an immune inhibitor such as azathioprine, mercaptopurine, infliximab, adalimumab, certolizumab pegol, methotrexate, cyclosporine or natalizumab, an antibiotic such as metronidazole or ciprofloxacin, an antidiarrheal agent such as psyllium powder, loperamide or methylcellulose, lazatib, an analgesic such as an NSAID or acetaminophen, an iron supplement, a vitamin B supplement, a vitamin D supplement and any combination of the above, but is not limited thereto. In another example, the compound of the present disclosure may be administered in combination with other anti-IBD treatments, such as a surgery (e.g., before, during, or after).

In a specific embodiment, the compound of the present disclosure is coadministered with any antipsoriasis agent, including a topical corticosteroid, a vitamin D analog such as calcipotriene or calcitriol, anthralin, a topical retinoid such as tazarotene, a calcineurin inhibitor such as tacrolimus or pimecrolinus, salicylic acid, coal tar, NSAID, moisturizing cream and ointment, oral or injectable retinoid such as acitretin, methotrexate, cyclosporine, hydroxyurea, an immunomodulatory drug such as alefacept, etanercept, infliximab or ustekinumab, thioguanine, and any combination of the medicament, but is not limited thereto. In another example, the compound of the present disclosure may be administered with another antipsoriatic treatment, such as light therapy, sunlight therapy, UVB therapy, narrow-band UVB therapy, Goeckerman therapy, photo chemotherapy, such as psoralen plus ultraviolet A (PUVA), excimer and pulsed dye laser therapy, or any combination (e.g., before, during, or after) of an antipsoriasis agent and antipsoriasis therapy.

In a specific embodiment, the compound of the present disclosure is coadministered with any anti-asthmatic agent including a β2-adrenergic agonist, an inhaled and oral corticosteroid, a leukotriene receptor antagonist, and omalizumab, but is not limited thereto. In another embodiment, the compound of the present disclosure may be coadministered with an antiasthmatic agent selected from a combination of NSAID, fluticasone and salmeterol, a combination of budesonide and formoterol, omalizumab, lebrikizumab and a corticosteroid selected from fluticasone, budesonide, mometasone, flunisolide and beclomethasone.

The compound according to the present disclosure and a pharmaceutically acceptable salt thereof have a TYK2 inhibitory activity with superior cell activity and enzyme selectivity than a compound in the related art compared to a conventional TYK2 inhibitor, and thus it can be effectively used for the prevention, improvement, or treatment of TYK2-related diseases, specifically autoimmune diseases, inflammatory diseases, cancers or neurodegenerative disease.

### DETAILED DESCRIPTION OF THE EMBODIMENTS

Hereinafter, preferred examples are presented for helping understanding of the present disclosure. However, following examples are for illustrative purposes only, and the disclosure is not intended to be limited by these examples.

Various methods for synthesizing the starting materials to synthesize the compounds of the present disclosure have been known, and when available on the market, the starting materials may be purchased from the providers. Reagent suppliers include Sigma-Aldrich, TCI, an-gene, chemspace, chemscene, BLD, Alfa, combi-blocks, and Dae-Jung, but are not limited thereto. In addition, all the materials on the commercial market were used without further purification, unless specified otherwise.

First, the compound used in synthesis in the following examples was prepared according to following Preparation Examples. The following Preparation Examples are examples of the compound represented by Formula 1 of Reaction Formula 1 described earlier and may be appropriately changed according to the structure of the example to be prepared.

### Preparation Example 1) Preparation of N-(3-(5-isobutoxy-1-isopropyl-6-oxo-1,6-dihydropyridazin-3-yl)-1-methyl-1H-pyrrolo[2,3-c]pyridin-5-yl)acetamide (Example 19 compound)

### Step 1) Preparation of 4-bromo-6-chloro-2-isopropylpyridazin-3(2H)-one

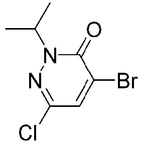

500 mg (2.39 mmol) of 5-bromo-3-chloro-1H-pyridazin-6-one was dissolved in 10 mL of N,N-dimethylformamide, and then 1556 mg (4.77 mmol) of cesium carbonate and 0.360 mL (3.58 mmol) of 2-iodopropane were added dropwise. After stirring for 16 hours at room temperature, 30 mL of water and 30 mL of ethyl acetate were added to separate the layers. The product was extracted with ethyl acetate 200 mL. The organic layer was treated with anhydrous magnesium sulfate and then concentrated. The target compound was obtained by separating using a column chromatography method. (550 mg, 92%)

LC-MS (ESI) calcd. for C₇H₈BrClN₂O: 249.95, m/z found, 250.62[M+H]⁺.

### Step 2) Preparation of 6-chloro-4-isobutoxy-2-isopropyl-pyridazin-3-one

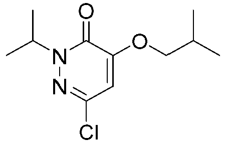

275 mg (1.09 mmol) of 4-bromo-6-chloro-2-isopropylpyridazin-3(2H)-one and 82 mg (1.09 mmol) of 2-methylpropan-1-ol were dissolved in 5 mL of 1,4-dioxane, and then (0.71 mL, 1.42 mmol) of 2M NaHMDS was slowly added dropwise. After stirring for 1 hour at 50°C, 10 mL of water was added to terminate the reaction. The organic layer extracted with 100 mL of ethyl acetate was dried with anhydrous magnesium sulfate, filtered, and then concentrated. The target compound was obtained by separating using a column chromatography method. (179 mg, 67%)

LC-MS (ESI) calcd. for C₁₁H₁₇ClN₂O₂: 244.10, m/z found, 245.10 [M+H]⁺.

### Step 3) Preparation of N-(3-bromo-1H-pyrrolo[2,3-c]pyridin-5-yl)acetamide

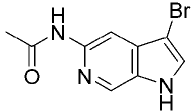

10 g (54.23 mmol) of N-(1H-pyrrolo[2,3-c]pyridin-5-yl)acetamide was dissolved in 50 mL of N,N-dimethylformamide, and then 12.19 g (81.34 mmol) of NBS was added dropwise. After stirring at room temperature until the reaction was completed, 100 mL of water and 100 mL of ethyl acetate were added dropwise. The organic layer extracted with 200 mL of ethyl acetate was dried with anhydrous magnesium sulfate, filtered, and then concentrated. The obtained compound was proceeded to the following reaction without a refining stage.

LC-MS (ESI) calcd. for C₉H₈BrN₃O: 252.99, m/z found, 254.27 [M+H]⁺.

¹H NMR (DMSO-d6, 400 MHz) δ 11.72 (s, 1H), 10.16 (s, 1H), 8.43 (s, 1H), 8.04 (s, 1H), 7.78 (s, 1H), 2.19 (s, 3H).

### Step 4) Preparation of N-(3-bromo-1-methyl-pyrrolo[2,3-c]pyridin-5-yl)acetamide

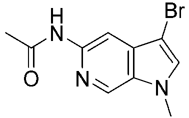

7.4 g (29.13 mmol) of N-(3-bromo-1H-pyrrolo[2,3-c]pyridin-5-yl)acetamide was dissolved in 200 mL of acetonitrile, and then 18.98 g (58.25 mmol) of cesium carbonate and 3.038 mL (32.04 mmol) of dimethyl sulfate were added dropwise. After stirring at room temperature until the reaction was completed, 100 mL of water and 100 mL of ethyl acetate were added dropwise. The organic layer extracted with 200 mL of ethyl acetate was dried with anhydrous magnesium sulfate, filtered, and then concentrated. The obtained compound was proceeded to the following reaction without a refining stage. (7.74 g, 99%)

LC-MS (ESI) calcd. for C₁₀H₁₀BrN₃O: 267.00, m/z found, 268.20 [M+H]⁺.

¹H NMR (DMSO-d6, 400 MHz) δ 10.36 (s, 1H), 8.63 (s, 1H), 8.13 (s, 1H), 7.76 (s, 1H), 3.88 (s, 3H), 2.09 (s, 3H).

### Step 5) Preparation of N-[3-(5-isobutoxy-1-isopropyl-6-oxo-pyridazin-3-yl)-1-methyl-pyrrolo[2,3-c]pyridin-5-yl]acetamide

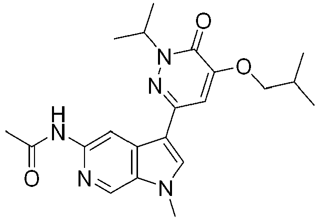

45 mg (0.18 mmol) of 6-chloro-4-isobutoxy-2-isopropyl-pyridazin-3-one, 93 mg (0.37 mmol) of bis(pinacolato)diboron, 36 mg (0.37 mmol) of potassium acetate, 16 mg (0.028 mmol) of Pd(dba)₂, and 0.092 mL (0.055 mmol) of 0.6M PCy₃ in toluene were dissolved in 1 mL of 1,4-dioxane and stirred at 90°C for 2 hours. After lowering the temperature to room temperature, it was filtered using celite. The mixture was dissolved in 1 mL of 1,4-dioxane and 0.1 mL of water, and then 58 mg (0.18 mmol) of N-(3-bromo-1-methyl-pyrrolo[2,3-c]pyridin-5-yl)acetamide, 15 mg (0.018 mmol) of Pd(dppf)₂Cl₂·DCM, and 78 mg (0.37 mmol) of K₃PO₄ were added dropwise and stirred at 80°C for 16 hours. After lowering the temperature to room temperature, it was filtered using celite. The target compound was obtained by separating using Prep-HPLC. (10.5 mg, 14%)

LC-MS (ESI) calcd. for C₂₁H₂₇N₅O₃: 397.21, m/z found, 398.25[M+H]⁺.

¹H NMR (CHLOROFORM-d, 400 MHz) δ 9.08 (s, 1H), 8.45 (s, 1H), 7.95 (br s, 1H), 7.54 (s, 1H), 6.71 (s, 1H), 5.4-5.6 (m, 1H), 3.95 (s, 3H), 3.84 (d, 2H, J=6.6 Hz), 2.2-2.3 (m, 4H), 1.6-1.6 (m, 6H), 1.12 (d, 6H, J=6.8 Hz).

### Preparation Example 2) Preparation of 1-(3-(5-isobutoxy-6-oxo-1-(1,1,1-trifluoropropan-2-yl)-1,6-dihydropyridazin-3-yl)-1-methyl-1H-pyrrolo[2,3-c]pyridin-5-yl)urea (Example 37 compound)

### Step 1) Preparation of 4-bromo-6-chloro-2-(2,2,2-trifluoro-1-methylethyl)pyridazin-3-one

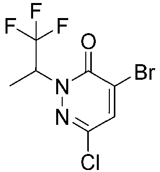

500 mg (2.39 mmol) of 5-bromo-3-chloro-1H-pyridazin-6-one, 409 mg (3.58 mmol) of 1,1,1-trifluoropropan-2-ol, and 1252 mg (4.77 mmol) of PPh3 were dissolved in 10 mL of tetrahydrofuran, and then 0.937 mL (4.77 mmol) of DIAD was slowly added dropwise at 0°C. The temperature was raised to room temperature and stirred for 16 hours, and then 20 mL of water and 20 mL of ethyl acetate were added dropwise. The organic layer extracted with 100 mL of ethyl acetate was dried with anhydrous magnesium sulfate, filtered, and then concentrated. The target compound was obtained by separating using a column chromatography method. (182 mg, 25%)

LC-MS (ESI) calcd. for C₇H₅BrClF₃N₂O: 303.92, m/z found, 305.21[M+H]⁺.

¹H NMR (DMSO-d, 400 MHz) δ 8.31 (s, 1H), 5.73-5.80 (m, 1H), 1.54-1.56 (d, 3H).

### Step 2) Preparation of 6-chloro-4-isobutoxy-2-(2,2,2-trifluoro-1-methylethyl)pyridazin-3-one

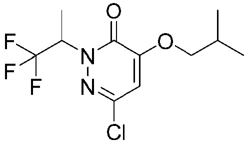

The title compound was prepared by the same method as in Step 1 of Preparation Example 2.

LC-MS (ESI) calcd. for C₁₁H₁₄ClF₃N₂O₂: 298.07, m/z found, 299.28[M+H]⁺.

¹H NMR (DMSO-d, 400 MHz) δ 7.02 (s, 1H), 5.71-5.79 (m, 1H), 3.84-3.92 (m, 2H), 2.02-2.11 (m, 1H), 1.52-1.54 (d, 3H), 0.96-0.97 (d, 6H).

### Step 3) Preparation of (3-bromo-1-methyl-pyrrolo[2,3-c]pyridin-5-yl)urea

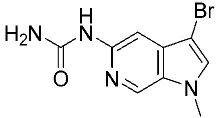

2500 mg (9.32 mmol) of N-(3-bromo-1-methyl-pyrrolo[2,3-c]pyridin-5-yl)acetamide was dissolved in 10 mL of methanol, and 11 mL (46.6 mmol) of 4N HCl was added dropwise. After reaction at 80°C for 1 hour, the mixture was concentrated under reduced pressure. The reaction mixture was dissolved in 10 mL of dimethyl chloride, and then 4.61 mL (27.97 mmol) of N,N-diisopropylethylamine and 2.78 mL (23.31 mmol) of 2,2,2-trichloroacetyl isocyanate were slowly added dropwise at 0°C. After stirring at room temperature until the reaction was completed, the reaction mixture was concentrated under reduced pressure. The reaction mixture was dissolved in 10 mL of methanol, and then 10 mL of saturated sodium bicarbonate aqueous solution was added dropwise and stirred at room temperature for 4 hours. The precipitated solid was filtered and dried to obtain the target compound. (2200 mg, 88%)

¹H NMR (DMSO-d, 400 MHz) δ 8.84 (s, 1H), 8.54 (s, 1H), 7.72 (s, 1H), 6.39-6.62 (m, 2H), 3.84 (s, 3H).

### Step 4) Preparation of 1-(3-(5-isobutoxy-6-oxo-1-(1,1,1-trifluoropropan-2-yl)-1,6-dihydropyridazin-3-yl)-1-methyl-1H-pyrrolo[2,3-c]pyridin-5-yl)urea

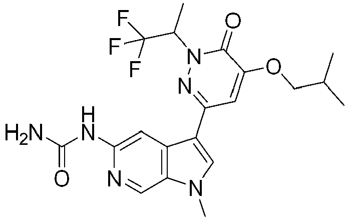

37 mg (0.12 mmol) of 6-chloro-4-isobutoxy-2-(2,2,2-trifluoro-1-methylethyl)pyridazin-3-one, 63 mg (0.25 mmol) of bis(pinacolato)diboron, 24 mg (0.25 mmol) of potassium acetate, 11 mg (0.019 mmol) of Pd(dba)₂, and 0.062 mL (0.037 mmol) of 0.6M PCy₃ in toluene were dissolved in 1,4-dioxane 1 mL and stirred at 90°C for 2 hours. After lowering the temperature to room temperature, it was filtered using celite. The mixture was dissolved in 1 mL of 1,4-dioxane and 0.1 mL of water, and then 33 mg (0.12 mmol) of (3-bromo-1-methyl-pyrrolo[2,3-c]pyridin-5-yl)urea, 10 mg (0.012 mmol) of Pd(dppf)₂Cl₂·DCM, and 53 mg (0.25 mmol) of K₃PO₄ were added dropwise and stirred at 80°C for 16 hours. After lowering the temperature to room temperature, it was filtered using celite. The target compound was obtained using Prep-HPLC. (1.5 mg, 2.6%)

LC-MS (ESI) calcd. for C₂₀H₂₃F₃N₆O₃: 452.18, m/z found, 453.32[M+H]⁺.

¹H NMR (CHLOROFORM-d, 400 MHz) δ 8.4-8.5 (m, 1H), 7.6-7.6 (m, 1H), 7.5-7.6 (m, 1H), 6.9-7.0 (m, 1H), 6.67 (s, 1H), 5.9-6.0 (m, 1H), 3.9-4.0 (m, 3H), 3.8-3.9 (m, 2H), 2.2-2.4 (m, 1H), 1.7-1.8 (m, 3H), 1.0-1.1 (m, 6H).

### Preparation Example 3) Preparation of N-(1-cyclopropyl-3-(1-isopropyl-5-(3-methoxyazetidin-1-yl)-6-oxo-1,6-dihydropyridazin-3-yl)-1H-pyrrolo [2,3-c]pyridin-5-yl)acetamide (Example 21 compound)

### Step 1) Preparing of 4-bromo-6-chloro-2-isopropylpyridazin-3(2H)-one

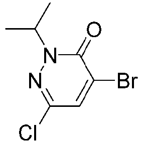

The title compound was obtained by the same method as in Step 1 of Preparation Example 1.

### Step 2) Preparation of 6-chloro-2-isopropyl-4-(3-methoxyazetidin-1-yl)pyridazin-3-one

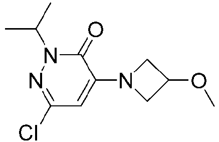

135 mg (1.09 mmol) of 3-methoxyazetidine hydrochloride was dissolved in 5 mL of 1,4-dioxane, and then 0.36 mL (2.19 mmol) of N,N-diisopropylethylamine was added dropwise and stirred at room temperature for 15 minutes. 275 mg (1.09 mmol) of 4-bromo-6-chloro-2-isopropylpyridazin-3(2H)-one and 0.71 mL (1.42 mmol) of 2M NaHMDS were added dropwise to the reaction mixture and stirred at 50°C for 1 hour. After lowering the temperature to room temperature, 10 mL of water was added dropwise and neutralized to pH 7-8 using 1 N HCl aqueous solution. The organic layer extracted with 100 mL of ethyl acetate was dried with anhydrous magnesium sulfate, filtered, and then concentrated. The target compound was obtained by separating using a column chromatography method. (70 mg, 25%)

LC-MS (ESI) calcd. for C₁₁H₁₆ClN₃O₂: 257.09, m/z found, 257.93[M+H]⁺.

### Step 3) Preparation of N-(3-bromo-1-cyclopropyl-pyrrolo[2,3-c]pyridin-5-yl)acetamide

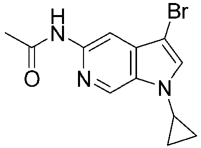

1430 mg (7.87 mmol) of anhydrous copper (II) acetate was dissolved in 12 mL of N,N-dimethylformamide, and then 1229 mg (7.87 mmol) of 2,2'-bipyridyl, 962 mg (7.87 mmol) of sodium carbonate, 1392 mg (7.87 mmol) of 2-cyclopropyl-4,4,5,5-tetramethyl-1,3,2-dioxaborolane, and 1000 mg (3.94 mmol) of N-(3-bromo-1H-pyrrolo[2,3-c]pyridin-5-yl)acetamide were added dropwise in order. It was stirred at 70°C for 16 hours. After lowering the temperature to room temperature, 100 mL of water and 100 mL of ethyl acetate were added dropwise. The organic layer extracted with 200 mL of ethyl acetate was dried with anhydrous magnesium sulfate, filtered, and then concentrated. The target compound was obtained by separating using a column chromatography method. (951 mg, 82%)

LC-MS (ESI) calcd. for C₁₂H₁₂BrN₃O: 293.02, m/z found, 294.30[M+H]⁺.

### Step 4) Preparation of N-(1-cyclopropyl-3-(1-isopropyl-5-(3-methoxyazetidin-1-yl)-6-oxo-1,6-dihydropyridazin-3-yl)-1H-pyrrolo[2,3-c]pyridin-5-yl)acetamide

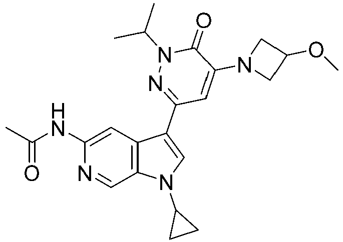

35 mg (0.14 mmol) of 6-chloro-2-isopropyl-4-(3-methoxyazetidin-1-yl)pyridazin-3-one, 69 mg (0.27 mmol) of bis(pinacolato)diboron, 27 mg (0.27 mmol) of potassium acetate, 12 mg (0.020 mmol) of Pd(dba)₂, and 0.068 mL (0.041 mmol) of 0.6M PCy₃ in toluene were dissolved in 1 mL of 1,4-dioxane and stirred at 90°C for 2 hours. After lowering the temperature to room temperature, it was filtered using celite. The mixture was dissolved in 1 mL of 1,4-dioxane and 0.1 mL of water, and then 40 mg (0.14 mmol) of N-(3-bromo-1-cyclopropyl-pyrrolo[2,3-c]pyridin-5-yl)acetamide, 11 mg (0.014 mmol) of Pd(dppf)₂Cl₂·DCM, and 58 mg (0.27 mmol) of K₃PO₄ were added dropwise and stirred at 80°C for 16 hours. After lowering the temperature to room temperature, it was filtered using celite. The target compound was obtained by separating using Prep-HPLC. (2.4 mg, 4%)

LC-MS (ESI) calcd. for C₂₃H₂₈N₆O₃: 436.22, m/z found, 437.32[M+H]⁺.

¹H NMR (CHLOROFORM-d, 400 MHz) δ 9.01 (s, 1H), 8.5-8.7 (m, 1H), 7.99 (br s, 1H), 7.56 (s, 1H), 6.07 (s, 1H), 5.3-5.4 (m, 1H), 4.53 (br s, 2H), 4.35 (s, 1H), 4.1-4.3 (m, 2H), 3.48 (s, 1H), 3.36 (s, 3H), 2.2-2.3 (m, 4H), 1.5-1.6 (m, 6H), 1.2-1.2 (m, 2H), 1.12 (br d, 2H, J=4.4 Hz).

**Preparation Example 4) Preparation of N-(5-(5-(3-methoxyazetidine-1-yl)-1-(3-methyltetrahydrofuran-3-yl)-6-oxo-1,6-dihydropyridazin-3-yl)-7-methylpyrrolo[1,2-c]pyrimidin-3-yl)acetamide (Example 16 compound)**

### Step 1) Preparation of 7-methylpyrrolo[1,2-c]pyrimidin-3-amine

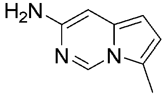

200 mg (0.81 mmol) of tert-butyl N-{7-methylpyrrolo[1,2-c]pyrimidin-3-yl} carbamate was dissolved in 2 mL of dimethyl chloride, and then 0.1 mL of TFA was added dropwise. After stirring at room temperature until the reaction was completed, the reaction mixture was concentrated under reduced pressure. The obtained compound was proceeded to the following reaction without a refining stage.

LC-MS (ESI) calcd. for C₈H₉N₃: 147.08, m/z found, 147.72[M+H]⁺.

### Step 2) Preparation of N-(7-methylpyrrolo[1,2-c]pyrimidin-3-yl)acetamide

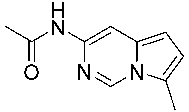

118 mg (0.80 mmol) of 7-methylpyrrolo[1,2-c]pyrimidin-3-amine was dissolved in 2 mL of tetrahydrofuran, and then 0.091 mL (0.96 mmol) of acetic anhydride and 1.39 mL (8.00 mmol) of N,N-diisopropylethylamine were added dropwise. After stirring at room temperature until the reaction was completed, 10 mL of water and 10 mL of ethyl acetate were added dropwise. The organic layer extracted with 100 mL of ethyl acetate was dried with anhydrous magnesium sulfate, filtered, and then concentrated. The obtained compound was proceeded to the following reaction without a refining stage.

LC-MS (ESI) calcd. for C₁₀H₁₁N₃O: 189.09, m/z found, 190.13[M+H]⁺.

### Step 3) Preparation of N-(5-bromo-7-methyl-pyrrolo[1,2-c]pyrimidin-3-yl)acetamide

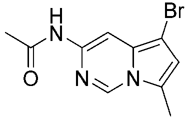

60 mg (0.32 mmol) of N-(7-methylpyrrolo[1,2-c]pyrimidin-3-yl)acetamide was dissolved in 2 mL of dimethyl chloride, and then 85 mg (0.48 mmol) of NBS was added dropwise. After stirring at room temperature until the reaction was completed, 10 mL of water and 10 mL of ethyl acetate were added dropwise. The organic layer extracted with 100 mL of ethyl acetate was dried with anhydrous magnesium sulfate, filtered, and then concentrated. The target compound was obtained by separating using a column chromatography method. (40 mg, 47%)

LC-MS (ESI) calcd. for C₁₀H₁₀BrN₃O: 267.00, m/z found, 268.01[M+H]⁺.

### Step 4) Preparation of N-[5-[5-(3-methoxyazetidin-1-yl)-1-(3-methyltetrahydrofuran-3-yl)-6-oxo-pyridazin-3-yl]-7-methyl-pyrrolo[1,2-c]pyrimidin-3-yl]acetamide

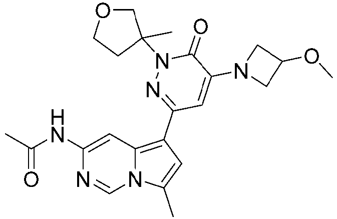

50 mg (0.17 mmol) of 6-chloro-4-(3-methoxyazetidin-1-yl)-2-(3-methyltetrahydrofuran-3-yl)pyridazin-3-one, 86 mg (0.33 mmol) of bis(pinacolato)diboron, 33 mg (0.33 mmol) of potassium acetate, 14 mg (0.025 mmol) of Pd(dba)2, 0.083 mL (0.051 mmol) of 0.6 M PCy₃ in toluene were dissolved in 1 mL of 1,4-dioxane and stirred at 130°C for 4 hours. After lowering the temperature to room temperature, it was filtered using celite. The mixture was dissolved in 1 mL of 1,4-dioxane and 0.1 mL of water, and then 40 mg (0.15 mmol) of N-(5-bromo-7-methyl-pyrrolo[1,2-c]pyrimidin-3-yl)acetamide, 14 mg (0.017 mmol) of Pd(dppf)₂Cl₂·DCM, and 71 mg (0.33 mmol) of K₃PO₄ were added dropwise and stirred at 80°C for 16 hours. After lowering the temperature to room temperature, it was filtered using celite. The target compound was obtained using Prep-HPLC. (1 mg, 1%)

LC-MS (ESI) calcd. for C₂₃H₂₈N₆O₄: 452.22, m/z found, 453.45[M+H]⁺

¹H NMR (CHLOROFORM-d, 400 MHz) δ 8.6-8.7 (m, 1H), 8.4-8.5 (m, 1H), 7.5-7.6 (m, 1H), 6.8-6.8 (m, 1H), 6.10 (s, 1H), 4.4-4.6 (m, 3H), 4.3-4.4 (m, 1H), 4.2-4.3 (m, 1H), 4.1-4.2 (m, 2H), 4.0-4.1 (m, 2H), 3.3 (s, 3H), 2.8-2.9 (m, 1H), 2.6-2.7 (m, 1H), 2.5 (s, 3H), 2.2 (s, 3H), 1.7 (s, 3H).

### Preparation Example 5) Preparation of N-(3-methyl-1-(1-(3-methyltetrahydrofuran-3-yl)-6-oxo-1,6-dihydropyridazin-3-yl)-1H-pyrrolo[3,2-c]pyridin-6-yl)acetamide (Example 15 compound)

### Step 1) Preparation of 6-(6-chloro-3-methyl-pyrrolo[3,2-c]pyridin-1-yl)-2-(3-methyltetrahydrofuran-3-yl)pyridazin-3-one

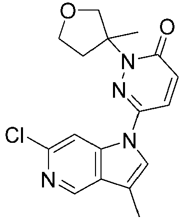

68 mg (0.26 mmol) of 6-bromo-2-(3-methyltetrahydrofuran-3-yl)pyridazin-3-one was dissolved in 2 mL of N,N-dimethylformamide, and then 69 mg (0.39 mmol) of 6-chloro-3-methyl-1H-pyrrolo[3,2-c]pyridine, 5.0 mg (0.026 mmol) of CuI, 5.6 µL (0.053 mmol) of N,N'-dimethylethylenediamine, and 108 mg (0.79 mmol) of potassium carbonate were added dropwise and stirred at 130°C for 16 hours. After lowering the temperature to room temperature, it was filtered using celite. The target compound was obtained using Prep-HPLC. (10 mg, 11%)

LC-MS (ESI) calcd. for C₁₇H₁₇ClN₄O₂: 344.10, m/z found, 345.07[M+H]⁺.

### Step 2) Preparation of N-(3-methyl-1-(1-(3-methyltetrahydrofuran-3-yl)-6-oxo-1,6-dihydropyridazin-3-yl)-1H-pyrrolo[3,2-c]pyridin-6-yl)acetamide

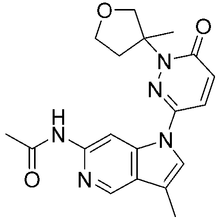

10 mg (0.029 mmol) of 6-(6-chloro-3-methyl-pyrrolo[3,2-c]pyridin-1-yl)-2-(3-methyltetrahydrofuran-3-yl)pyridazin-3-one, 3.5 mg (0.058 mmol) of acetamide, 2.7 mg (0.0029 mmol) of Brettphos Pd(II) G3, and 28 mg (0.087 mmol) of cesium carbonate were dissolved in 2 mL of *tert*-butanol and stirred at 130°C for 3 hours. After lowering the temperature to room temperature, it was filtered using celite. The target compound was obtained using Prep-HPLC. (1.5 mg, 14%)

LC-MS (ESI) calcd. for C₁₉H₂₁N₅O₃: 367.16, m/z found, 368.26[M+H]⁺.

¹H NMR (CHLOROFORM-d, 400 MHz) δ 8.68 (s, 1H), 8.56 (s, 1H), 8.09 (br s, 1H), 7.56 (d, 1H, J=9.9 Hz), 7.1-7.2 (m, 2H), 4.2-4.4 (m, 2H), 4.0-4.1 (m, 2H), 2.8-2.9 (m, 1H), 2.5-2.6 (m, 1H), 2.4 (s, 3H), 2.2 (s, 3H), 1.8 (s, 3H).

### Preparation Example 6) Preparation of N-(3-methyl-1-(1-(3-methyltetrahydrofuran-3-yl)-6-oxo-1,6-dihydropyridazin-3-yl)-1H-pyrrolo [3,2-c]pyridin-6-yl)acetamide (Example 5 compound)

### Step 1) Preparation of 6-chloro-4-methyl-2-(3-methyltetrahydrofuran-3-yl)pyridazin-3-one

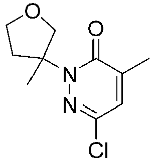

100 mg (0.692 mmol) of 3-chloro-5-methyl-1H-pyridazin-6-one, 141 mg (1.38 mmol) of tetrahydro-3-methyl-3-furanol, and 417 mg (1.59 mmol) of PPh₃ were dissolved in 5 mL of tetrahydrofuran, and then 0.312 mL (1.59 mmol) of DIAD was slowly added dropwise at 0°C. The temperature was raised to room temperature and stirred for 16 hours, and then 20 mL of water and 20 mL of ethyl acetate were added dropwise. The organic layer extracted with 100 mL of ethyl acetate was dried with anhydrous magnesium sulfate, filtered, and then concentrated. The target compound was obtained by separating using a column chromatography method. (30 mg, 19%)

LC-MS (ESI) calcd. for C₁₀H₁₃ClN₂O₂: 228.68, m/z found, 229.13[M+H]⁺.

### Step 2) Preparation of N-(3-iodo-1H-pyrrolo[2,3-c]pyridin-5-yl)acetamide

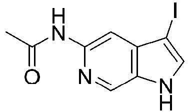

5 g (28.54 mmol) of N-(1H-pyrrolo[2,3-c]pyridin-5-yl)acetamide was dissolved in 20 mL of N,N-dimethylformamide, and then 7.7 g (34.25 mmol) of NIS was added dropwise. After stirring at room temperature until the reaction was completed, 100 mL of water and 100 mL of ethyl acetate were added dropwise. The organic layer extracted with 200 mL of ethyl acetate was dried with anhydrous magnesium sulfate, filtered, and then concentrated. The obtained compound was proceeded to the following reaction without a refining stage.

LC-MS (ESI) calcd. for C₉H₈IN₃O: 300.97, m/z found, 301.67 [M+H]⁺.

### Step 3) Preparation of N-(1-(3-cyanocyclobutyl)-3-iodo-1H-pyrrolo[2,3-c]pyridin-5-yl)acetamide

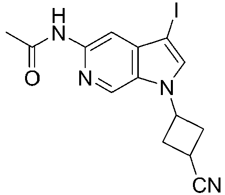

631 mg (6.50 mmol) of 3-hydroxycyclobutanecarbonitrile was dissolved in 7 mL of dimethyl chloride, and then 1.09 mL (7.80 mmol) of triethylamine and 0.550 mL (7.15 mmol) of methanesulfonyl chloride were slowly added dropwise at -10°C. After stirring at 0°C for 4 hours, 20 mL of water and 20 mL of ethyl acetate were added dropwise, and washed with saturated hydrogen carbonate sodium aqueous solution. The organic layer extracted with 200 mL of ethyl acetate was dried with anhydrous magnesium sulfate, filtered, and then concentrated. The obtained compound was proceeded to the following reaction without a refining stage. 1505 mg (5.0 mmol) of N-(3-iodo-1H-pyrrolo[2,3-c]pyridin-5-yl)acetamide and 3258 mg (10.0 mmol) of cesium carbonate were dissolved in 20 mL of N,N-dimethylformamide, and then the (3-cyanocyclobutyl)methanesulfonate prepared above was added dropwise. After stirring overnight at 80°C, 100 mL of water and 100 mL of ethyl acetate were added dropwise. The organic layer extracted with 200 mL of ethyl acetate was dried with anhydrous magnesium sulfate, filtered, and then concentrated. The target compound was obtained by separating using a column chromatography method. (200 mg, 11%)

LC-MS (ESI) calcd. for C₁₄H₁₃IN₄O: 380.01, m/z found, 381.06 [M+H]⁺.

### Step 4) Preparation of N-(1-(3-cyanocyclobutyl)-3-(5-methyl-1-(3-methyltetrahydrofuran-3-yl)-6-oxo-1,6-dihydropyridazin-3-yl)-1H-pyrrolo[2,3-c]pyridin-5-yl)acetamide

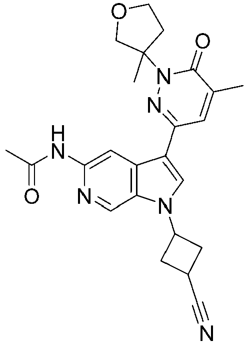

30 mg (0.13 mmol) of 6-chloro-4-methyl-2-(3-methyltetrahydrofuran-3-yl)pyridazin-3-one, 66 mg (0.26 mmol) of bis(pinacolato)diboron, 26 mg (0.26 mmol) of potassium acetate, and 11 mg (0.013 mmol) of Pd(dppf)₂Cl₂·DCM were dissolved in 1 mL of 1,4-dioxane and stirred at 100°C for 2 hours. After lowering the temperature to room temperature, it was filtered using celite. The mixture was dissolved in 1 mL of 1,4-dioxane and 0.1 mL of water, and then 49 mg (0.13 mmol) of N-[1-(3-cyanocyclobutyl)-3-iodo-pyrrolo[2,3-c]pyridin-5-yl]acetamide, 11 mg (0.013 mmol) of Pd(dppf)₂Cl₂·DCM, and 55 mg (0.26 mmol) of K₃PO₄ were added dropwise and stirred at 80°C for 16 hours. After lowering the temperature to room temperature, it was filtered using celite. The target compound was obtained by separating using Prep-HPLC. (13 mg, 22%)

LC-MS (ESI) calcd. for C₂₄H₂₆N₆O₃: 446.21, m/z found, 447.27 [M+H]⁺.

¹H NMR (CHLOROFORM-d, 400 MHz) δ 8.96 (s, 1H), 8.43 (s, 1H), 8.09 (s, 1H), 7.71 (s, 1H), 7.4-7.5 (m, 1H), 4.97 (br d, 1H, J=9.0 Hz), 4.50 (d, 1H, J=9.3 Hz), 4.30 (d, 1H, J=9.4 Hz), 4.0-4.1 (m, 2H), 3.1-3.2 (m, 3H), 2.8-3.0 (m, 3H), 2.72 (br d, 1H, J=6.1 Hz), 2.3-2.3 (m, 3H), 2.24 (s, 3H), 1.77 (s, 3H).

### Preparation Example 7) Preparation of N-(3-(5-(3-methoxyazetidin-1-yl)-6-oxo-1-(1,1,1-trifluoropropan-2-yl)-1,6-dihydropyridazin-3-yl)-1-methyl-1H-pyrrolo[2,3-c]pyridin-5-yl)acetamide(Example 39 compound)

### Step 1) Preparation of 4-bromo-6-chloro-2-(2,2,2-trifluoro-1-methylethyl)pyridazin-3-one

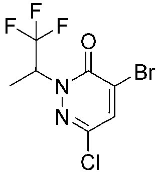

The title compound was obtained by the same method as in Step 1 of Preparation Example 2.

### Step 2) Preparation of 6-chloro-4-(3-methoxyazetidine-1-yl)-2-(1,1,1-trifluoropropan-2-yl)pyridazin-3(2H)-one

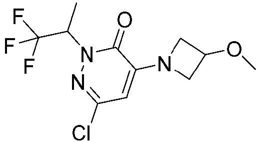

97 mg (0.79 mmol) of 3-methoxyazetidine hydrochloride was dissolved in 1 mL of 1,4-dioxane, and then 0.22 mL (1.31 mmol) of N,N-diisopropylethylamine was added dropwise and stirred at room temperature for 15 minutes. 200 mg (0.65 mmol) of 4-bromo-6-chloro-2-(2,2,2-trifluoro-1-methyl-ethyl)pyridazin-3-one and 0.42 mL (0.85 mmol) of 2M NaHMDS were added dropwise to the reaction mixture and stirred at 50°C for 1 hour. After lowering the temperature to room temperature, 10 mL of water was added dropwise and neutralized to pH 7-8 using 1 N HCl aqueous solution. The organic layer extracted with 100 mL of ethyl acetate was dried with anhydrous magnesium sulfate, filtered, and then concentrated. The target compound was obtained by separating using a column chromatography method. (56 mg, 27%)

LC-MS (ESI) calcd. for C₁₁H₁₃ClF₃N₃O₂: 311.06, m/z found, 311.95[M+H]⁺.

### Step 3) Preparation of N-(3-(5-(3-methoxyazetidin-1-yl)-6-oxo-1-(1,1,1-trifluoropropan-2-yl)-1,6-dihydropyridazin-3-yl)-1-methyl-1H-pyrrolo[2,3-c]pyridin-5-yl)acetamide

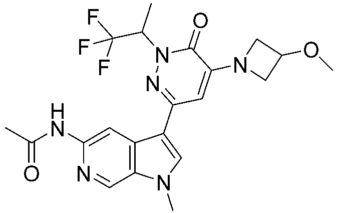

62 mg (0.20 mmol) of 6-chloro-4-(3-methoxyazetidine-1-yl)-2-(1,1,1-trifluoropropan-2-yl)pyridazin-3(2H)-one, 101 mg (0.40 mmol) of bis(pinacolato)diboron, 39 mg (0.40 mmol) of potassium acetate, and 16 mg (0.020 mmol) of Pd(dppf)₂Cl₂·DCM were dissolved in 1 mL of 1,4-dioxane and stirred at 100°C for 2 hours. After lowering the temperature to room temperature, it was filtered using celite. The mixture was dissolved in 1 mL of 1,4-dioxane and 0.1 mL of water, and then 63 mg (0.20 mmol) of N-(3-iodo-1-methyl-pyrrolo[2,3-c]pyridin-5-yl)acetamide, 16 mg (0.020 mmol) of Pd(dppf)₂Cl₂·DCM, and 84 mg (0.40 mmol) of K₃PO₄ were added dropwise and stirred at 80°C overnight. After lowering the temperature to room temperature, it was filtered using celite. The target compound was obtained by separating using Prep-HPLC. (8 mg, 9%)

LC-MS (ESI) calcd. for C₂₁H₂₃F₃N₆O₃: 464.18, m/z found, 465.54 [M+H]⁺.

¹H NMR (CHLOROFORM-d, 400 MHz) δ 8.91 (s, 1H), 8.42 (s, 1H), 7.9-8.0 (m, 1H), 7.53 (s, 1H), 6.1-6.1 (m, 1H), 5.8-5.9 (m, 1H), 4.5-4.6 (m, 2H), 4.3-4.4 (m, 1H), 4.2-4.2 (m, 2H), 3.9-4.0 (m, 3H), 3.9-4.0 (m, 4H), 3.37 (s, 3H), 2.25 (s, 4H), 1.8-1.9 (m, 3H)

### Preparation Example 8) Preparation of (S)-N-(3-(5-(2,2-difluoroethoxy)-6-oxo-1-(1,1,1-trifluoropropan-2-yl)-1,6-dihydropyridazin-3-yl)-1H-pyrrolo[2,3-c]pyridin-5-yl)acetamide (Example 115 compound)

### Step 1) Preparation of N-(3-bromo-1H-pyrrolo[2,3-c]pyridin-5-yl)acetamide

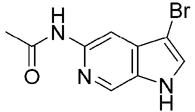

The title compound was obtained by the same method as in Step 3 of Preparation Example 1.

### Step 2) Preparation of 4-(2,2-difluoroethoxy)-6-(4,4,5,5-tetramethyl-1,3,2-dioxaborolan-2-yl)-2-[(2S)-1,1,1-trifluoropropan-2-yl]pyridazin-3-one

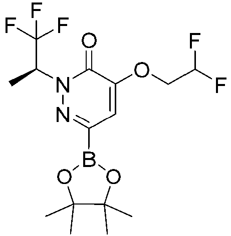

1000 mg (3.26 mmol) of 6-chloro-4-(2,2-difluoroethoxy)-2-[(2S)-1,1,1-trifluoropropan-2-yl]pyridazin-3-one, 1004 mg (3.91 mmol) of bis(pinacolato)diboron, 14.6 mg (0.065 mmol) of Pd(OAc)₂, 22.9 mg (0.065 mmol) of CyJohnPhos, and 384 mg (3.91 mmol) of potassium acetate were dissolved in monoglyme and stirred at 60°C for 1 hour. 15 mL of ethyl acetate and 7 mL of water were added, it was stirred and filtered using celite. After drying with anhydrous magnesium sulfate and filtering, it was concentrated, 5 mL of diisopropyl ether was added dropwise and stirred overnight. The target compound was obtained by filtering the crystal. (800 mg, 62%)

¹H NMR (DMSO-d6, 400 MHz) δ 6.88 (s, 1H), 6.3-6.6 (m, 1H), 5.8-5.9 (m, 1H), 4.4-4.5 (m, 2H), 1.57 (d, 3H, J=7.0 Hz), 1.31 (s, 12H)

### Step 3) Preparation of (S)-N-(3-(5-(2,2-difluoroethoxy)-6-oxo-1-(1,1,1-trifluoropropan-2-yl)-1,6-dihydropyridazin-3-yl)-1H-pyrrolo[2,3-c]pyridin-5-yl)acetamide

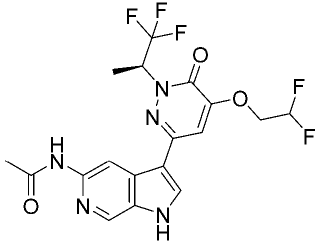

50 mg (0.20 mmol) of N-(3-bromo-1H-pyrrolo[2,3-c]pyridin-5-yl)acetamide, 94 mg (0.24 mmol) of 4-(2,2-difluoroethoxy)-6-(4,4,5,5-tetramethyl-1,3,2-dioxaborolan-2-yl)-2-[(2S)-1,1,1-trifluoropropan-2-yl]pyridazin-3-one, 16 mg (0.020 mmol) of Pd(dppf)₂Cl₂·DCM, and 84 mg (0.39 mmol) of K₃PO₄ were dissolved in 2 mL of 1,4-dioxane and 0.4 mL of water and stirred at 80°C for 2 hours. After lowering the temperature to room temperature, it was filtered using celite. The target compound was obtained using Prep-HPLC. (11.8 mg, 13%)

LC-MS (ESI) calcd. for C₁₈H₁₆F₅N₅O₃: 445.34, m/z found, 446.49 [M+H]⁺.

¹H NMR (CHLOROFORM-d, 400 MHz) δ 9.0 (s, 1H), 8.5-8.7 (m, 2H), 7.9 (s, 1H), 7.7 (s, 1H), 6.94 (br s, 1H), 6.2-6.3 (m, 1H), 5.9-6.0 (m, 1H), 4.2-4.5 (m, 4H), 2.26 (br s, 3H), 1.8-2.0 (m, 3H)

### Preparation Example 9) Preparation of (S)-N-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)-2-hydroxyacetamide (Example 116 compound)

### Step 1) Preparation of (S)-6-(5-amino-1-ethyl-1H-pyrrolo[2,3-c]pyridin-3-yl)-4-(2,2-difluoroethoxy)-2-(1,1,1-trifluoropropan-2-yl)pyridazin-3(2H)-one

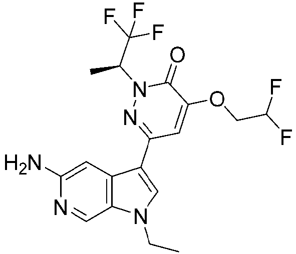

150 mg (0.32 mmol) of (S)-N-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)acetamide (Example 85) was dissolved in 1 mL of dimethyl chloride, and then 0.3 mL of 4N HCl in 1,4-dioxane was added dropwise and stirred at 80°C overnight. After lowering the temperature to room temperature, it was concentrated and the target compound was obtained using Prep-HPLC. (130 mg, 95%)

LC-MS (ESI) calcd. for C₁₈H₁₈F₅N₅O₂: 431.36, m/z found, 432.12 [M+H]⁺.

¹H NMR (METHANOL-d4, 400 MHz) δ 8.27 (br s, 1H), 8.09 (br s, 1H), 7.46 (br s, 1H), 7.30 (br s, 1H), 6.46 (br s, 1H), 6.32 (br s, 1H), 6.19 (br s, 1H), 5.9-6.0 (m, 1H), 4.48 (br t, 2H, J=12.6 Hz), 4.24 (br d, 2H, J=6.6 Hz), 1.80 (br d, 3H, J=6.3 Hz), 1.51 (br t, 3H, J=6.3 Hz)

### Step 2) Preparation of (S)-N-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)-2-hydroxyacetamide

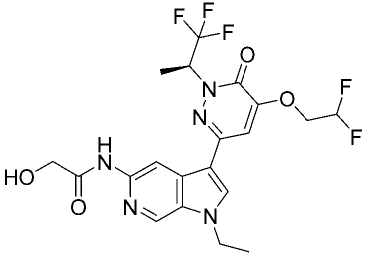

30 mg (0.0695 mmol) of (S)-6-(5-amino-1-ethyl-1H-pyrrolo[2,3-c]pyridin-3-yl)-4-(2,2-difluoroethoxy)-2-(1,1,1-trifluoropropan-2-yl)pyridazin-3(2H)-one was dissolved in 1 mL of dimethyl chloride, and then 14.2 mg (0.104 mmol) of acetoxyacetyl chloride and 0.028 mL (0.348 mmol) of pyridine were added dropwise and stirred at room temperature for 3 hours. The reactant was concentrated under reduced pressure, dissolved in 1 mL of methanol, and then 19.2 mg (0.139 mmol) of potassium carbonate was added dropwise and stirred at room temperature for 15 minutes. After concentration under reduced pressure, the target compound was obtained using Prep-HPLC. (10.7 mg, 31%)

LC-MS (ESI) calcd. for C₂₀H₂₀F₅N₅O₄: 489.40, m/z found, 490.21 [M+H]⁺.

¹H NMR (CHLOROFORM-d, 400 MHz) δ 9.31 (br s, 1H), 9.02 (br s, 1H), 8.46 (s, 1H), 7.61 (s, 1H), 6.88 (s, 1H), 6.1-6.4 (m, 1H), 5.94 (br s, 1H), 5.32 (s, 6H), 4.2-4.5 (m, 6H), 1.89 (br d, 3H, J=6.5 Hz), 1.5-1.6 (m, 3H)

### Preparation Example 10) Preparation of (S)-1-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)-3-methylurea (Example 117 compound)

### Step 1) Preparation of (S)-6-(5-amino-1-ethyl-1H-pyrrolo[2,3-c]pyridin-3-yl)-4-(2,2-difluoroethoxy)-2-(1,1,1-trifluoropropan-2-yl)pyridazin-3(2H)-one

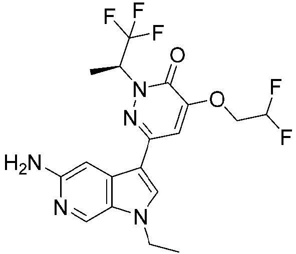

The title compound was obtained by the same method as in Step 1 of Preparation Example 9.

### Step 2) Preparation of (S)-1-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)-3-methylurea

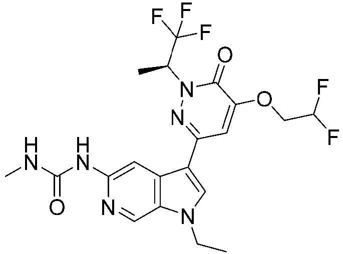

30 mg (0.0695 mmol) of (S)-6-(5-amino-1-ethyl-1H-pyrrolo[2,3-c]pyridin-3-yl)-4-(2,2-difluoroethoxy)-2-(1,1,1-trifluoropropan-2-yl)pyridazin-3(2H)-one was dissolved in 1 mL of tetrahydrofuran, and then 21 mg (0.0695 mmol) of triphosgene was added dropwise at 0°C and stirred for 1 hour. After raising the temperature to room temperature, 0.278 mL (0.556 mmol) of 2M methylamine in tetrahydrofuran was added dropwise and stirred for 2 hours. After concentration under reduced pressure, the target compound was obtained using Prep-HPLC. (7 mg, 21%)

¹H NMR (METHANOL-d4, 400 MHz) δ 8.54 (d, 1H, J=0.9 Hz), 8.2 (s, 1H), 8.1 (s, 1H), 7.84 (d, 1H, J=0.9 Hz), 7.37 (s, 1H), 6.2-6.5 (m, 1H), 5.9-6.0 (m, 1H), 4.50 (dt, 2H, J=3.9, 13.3 Hz), 4.3-4.4 (m, 2H), 3.13 (br s, 3H), 1.83 (d, 3H, J=7.0 Hz), 1.5-1.6 (m, 3H)

The following examples were synthesized with reference to the above Preparation Examples.

### Example 1: Synthesis of N-(1-methyl-3-(1-(3-methyltetrahydrofuran-3-yl)-6-oxo-1,6-dihydropyridazin-3-yl)-1H-pyrrolo[2,3-c]pyridin-5-yl)acetamide

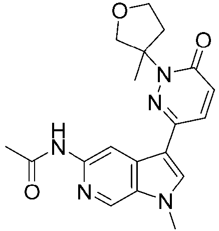

The compound was obtained from the method of Preparation Example 7.

¹H NMR (CHLOROFORM-d, 400 MHz) δ 8.91 (s, 1H), 8.43 (s, 1H), 7.9-8.0 (m, 1H), 7.5-7.6 (m, 2H), 6.94 (d, 1H, J=9.6 Hz), 4.4-4.5 (m, 1H), 4.30 (d, 1H, J=9.5 Hz), 4.0-4.1 (m, 2H), 3.9 (s, 3H), 2.8-2.9 (m, 1H), 2.6-2.7 (m, 1H), 2.2 (s, 3H), 1.7 (s, 3H)

### Example 2: Synthesis of N-(1-(3-cyanocyclobutyl)-3-(1-(3-methyltetrahydrofuran-3-yl)-6-oxo-1,6-dihydropyridazin-3-yl)-1H-pyrrolo[2,3-c]pyridin-5-yl)acetamide

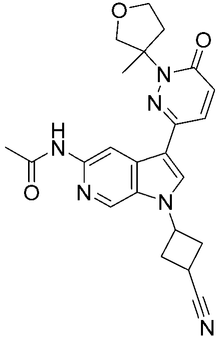

The compound was obtained from the method of Preparation Example 6.

¹H NMR (CHLOROFORM-d, 400 MHz) δ 8.9-9.0 (m, 1H), 8.4-8.5 (m, 1H), 7.9-7.9 (m, 1H), 7.7-7.7 (m, 1H), 7.6-7.7 (m, 1H), 6.9-7.1 (m, 1H), 4.9-5.0 (m, 1H), 4.4-4.5 (m, 1H), 4.2-4.4 (m, 1H), 4.0-4.2 (m, 2H), 3.1-3.3 (m, 3H), 2.8-3.0 (m, 3H), 2.7-2.8 (m, 1H), 2.2 (s, 3H), 1.7-1.8 (s, 3H)

### Example 3: Synthesis of N-(1-methyl-3-(1-(3-methyloxetan-3-yl)-6-oxo-1,6-dihydropyridazin-3-yl)-1H-pyrrolo[2,3-c]pyridin-5-yl)acetamide

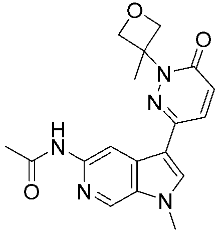

The compound was obtained from the method of Preparation Example 7.

¹H NMR (CHLOROFORM-d, 400 MHz) δ 8.9-8.9 (m, 1H), 8.46 (s, 1H), 8.0-8.0 (m, 1H), 7.5-7.6 (m, 2H), 6.9-7.0 (m, 1H), 6.3-6.4 (m, 2H), 5.2-5.3 (m, 2H), 4.9-5.1 (m, 3H), 4.7-4.8 (m, 2H), 3.9-4.1 (m, 5H), 2.2-2.3 (m, 6H), 2.0-2.0 (s, 3H)

### Example 4: Synthesis of 1-(1-methyl-3-(1-(3-methyltetrahydrofuran-3-yl)-6-oxo-1,6-dihydropyridazin-3-yl)-1H-pyrrolo[2,3-c]pyridin-5-yl)urea

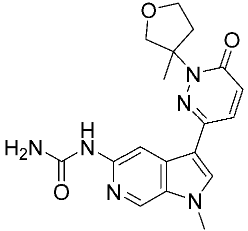

The compound was obtained from the method of Preparation Example 2.

¹H NMR (METHANOL-d4, 400 MHz) δ 8.55 (s, 1H), 8.1-8.1 (m, 1H), 8.05 (s, 1H), 7.90 (s, 1H), 6.99 (d, 1H, J=9.6 Hz), 4.4-4.4 (m, 1H), 4.2-4.3 (m, 1H), 4.0-4.1 (m, 2H), 3.9-4.0 (s, 3H), 3.59 (d, 1H, J=4.8 Hz), 3.5-3.6 (m, 1H), 2.8-2.9 (m, 1H), 2.7-2.7 (m, 1H), 2.0-2.1 (m, 3H)

### Example 5: Synthesis of N-(1-(3-cyanocyclobutyl)-3-(5-methyl-1-(3-methyltetrahydrofuran-3-yl)-6-oxo-1,6-dihydropyridazin-3-yl)-1H-pyrrolo[2,3-c]pyridin-5-yl)acetamide

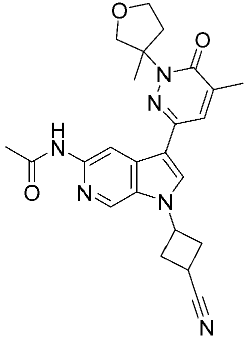

The compound was obtained from the method of Preparation Example 6.

¹H NMR (CHLOROFORM-d, 400 MHz) δ 8.96 (s, 1H), 8.43 (s, 1H), 8.09 (s, 1H), 7.71 (s, 1H), 7.4-7.5 (m, 1H), 4.97 (br d, 1H, J=9.0 Hz), 4.50 (d, 1H, J=9.3 Hz), 4.30 (d, 1H, J=9.4 Hz), 4.0-4.1 (m, 2H), 3.1-3.2 (m, 3H), 2.8-3.0 (m, 3H), 2.72 (br d, 1H, J=6.1 Hz), 2.3-2.3 (m, 3H), 2.24 (s, 3H), 1.77 (s, 3H)

### Example 6: Synthesis of N-(1-(3-cyanocyclobutyl)-3-(1-(3-ethyltetrahydrofuran-3-yl)-5-methyl-6-oxo-1,6-dihydropyridazin-3-yl)-1H-pyrrolo[2,3-c]pyridin-5-yl)acetamide

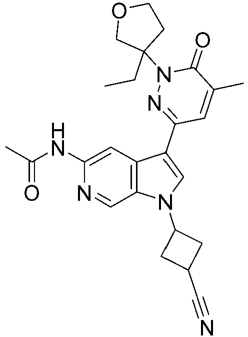

The compound was obtained from the method of Preparation Example 6.

¹H NMR (DMSO-d6, 400 MHz) δ 10.2-10.3 (m, 1H), 8.8-8.9 (m, 1H), 8.6-8.7 (m, 2H), 8.0-8.0 (m, 1H), 5.1-5.3 (m, 1H), 4.3-4.4 (m, 1H), 4.0-4.1 (m, 1H), 3.8-3.9 (m, 2H), 2.8-3.0 (m, 4H), 2.7-2.8 (m, 1H), 2.6-2.7 (m, 1H), 2.1-2.2 (m, 3H), 2.1-2.1 (s, 3H), 1.9-2.0 (m, 1H), 0.9 (s, 3H)

### Example 7: Synthesis of 1-(3-(1-(3-ethyltetrahydrofuran-3-yl)-5-methyl-6-oxo-1,6-dihydropyridazin-3-yl)-1-methyl-1H-pyrrolo [2,3-c] pyridin-5-yl)urea

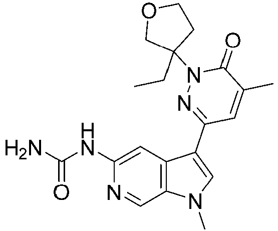

The compound was obtained from the method of Preparation Example 2.

¹H NMR (DMSO-d6, 400 MHz) δ 8.9-8.9 (m, 1H), 8.54 (d, 1H, J=0.8 Hz), 8.3-8.4 (m, 1H), 8.17 (s, 1H), 7.8-7.9 (m, 1H), 6.4-6.6 (m, 2H), 4.3-4.4 (m, 1H), 4.0-4.1 (m, 1H), 3.8-3.9 (m, 5H), 2.6-2.7 (m, 3H), 2.1-2.2 (m, 4H), 1.9-2.0 (m, 1H), 0.8 (s, 3H)

### Example 8: Synthesis of N-(1-(3-cyanocyclobutyl)-3-(5-cyclopropyl-1-(3-methyltetrahydrofuran-3-yl)-6-oxo-1,6-dihydropyridazin-3-yl)-1H-pyrrolo[2,3-c]pyridin-5-yl)acetamide

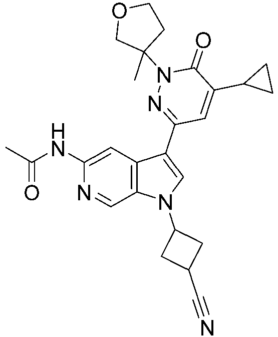

The compound was obtained from the method of Preparation Example 6.

¹H NMR (400 MHz, METHANOL- d4) δ 8.74 (s, 1 H) 8.59 - 8.66 (s, 1 H) 8.40 - 8.44 (s, 1 H) 7.50 - 7.55 (m, 1 H) 4.38 - 4.44 (m, 1 H) 3.95 - 4.06 (m, 3 H) 3.07 - 3.17 (m, 2 H) 2.92 - 3.04 (m, 2 H) 2.69 - 2.79 (m, 1 H) 2.25 - 2.33 (m, 1 H) 2.21 - 2.23 (s, 3 H) 2.17 - 2.20 (m, 1 H) 1.92 - 1.94 (m, 1 H) 1.90 - 1.92 (s, 3 H) 1.17 - 1.21 (m, 2 H) 1.01 - 1.07 (m, 2 H)

### Example 9: Synthesis of N-(1-(3-cyanocyclobutyl)-3-(5-ethoxy-1-(3-methyltetrahydrofuran-3-yl)-6-oxo-1,6-dihydropyridazin-3-yl)-1H-pyrrolo[2,3-c]pyridin-5-yl)acetamide

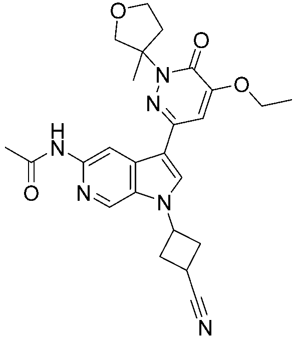

The compound was obtained from the method of Preparation Example 6.

¹H NMR (400 MHz, METHANOL- d4) δ 8.84 - 8.89 (s, 1 H) 8.57 - 8.60 (s, 1 H) 8.53 - 8.56 (s, 1 H) 7.34 - 7.38 (s, 1 H) 5.13 - 5.22 (m, 1 H) 4.40 - 4.45 (m, 1 H) 4.23 - 4.32 (m, 3 H) 4.01 - 4.08 (m, 2 H) 3.36 - 3.38 (m, 2 H) 3.07 - 3.16 (m, 2 H) 2.96 - 3.03 (m, 2 H) 2.86 - 2.95 (m, 1 H) 2.68 - 2.75 (m, 1 H) 2.21 - 2.23 (s, 3 H) 1.71 - 1.74 (s, 3 H) 1.52 - 1.58 (t, 3 H)

### Example 10: Synthesis of N-(1-(3-cyanocyclobutyl)-3-(5-(3-methoxyazetidin-1-yl)-1-(3-methyltetrahydrofuran-3-yl)-6-oxo-1,6-dihydropyridazin-3-yl)-1H-pyrrolo[2,3-c]pyridin-5-yl)acetamide

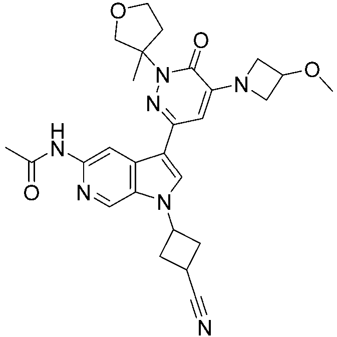

The compound was obtained from the method of Preparation Example 6.

¹H NMR (METHANOL-d4, 400 MHz) δ 8.83 (s, 1H), 8.56 (s, 1H), 8.41 (s, 1H), 6.58 (s, 1H), 5.1-5.2 (m, 1H), 4.5-4.5 (m, 1H), 4.3-4.5 (m, 2H), 4.22 (br d, 1H, J=9.8 Hz), 4.12 (br s, 1H), 4.02 (br d, 2H, J=7.5 Hz), 3.50 (br s, 1H), 2.8-3.2 (m, 5H), 2.6-2.7 (m, 1H), 2.21 (s, 2H), 1.69 (s, 2H)

### Example 11: Synthesis of 1-(3-(5-(3-methoxyazetidin-1-yl)-1-(3-methyltetrahydrofuran-3-yl)-6-oxo-1,6-dihydropyridazin-3-yl)-1-methyl-1H-pyrrolo[2,3-c]pyridin-5-yl)urea

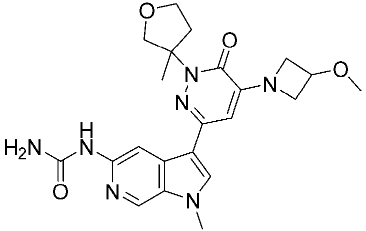

The compound was obtained from the method of Preparation Example 2.

¹H NMR (DMSO-d6, 400 MHz) δ 8.89 (br s, 1H), 8.51 (s, 1H), 8.22 (br s, 1H), 8.14 (s, 1H), 6.38 (s, 1H), 4.38 (br s, 2H), 4.27 (br d, 2H, J=9.1 Hz), 3.9-4.1 (m, 3H), 3.8-3.9 (m, 4H), 3.2-3.3 (m, 1H), 2.61 (br d, 2H, J=18.3 Hz), 1.54 (s, 3H)

### Example 12: Synthesis of N-(1-(3-cyanocyclobutyl)-3-(5-cyclopropyl-6-oxo-1-(3-oxocyclobutyl)-1,6-dihydropyridazin-3-yl)-1H-pyrrolo[2,3-c]pyridin-5-yl)acetamide

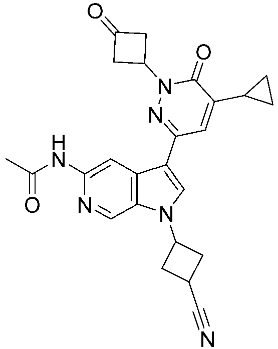

The compound was obtained from the method of Preparation Example 6.

¹H NMR (400 MHz, DMSO- d6) δ 10.23 - 10.31 (br s, 1 H) 8.84 - 8.93 (s, 1 H) 8.61 - 8.69 (m, 2 H) 7.45 - 7.52 (s, 1 H) 5.50 - 5.63 (s, 1 H) 5.10 - 5.25 (m, 1 H) 3.92 - 3.95 (m, 1 H) 3.74 - 3.83 (m, 2 H) 3.47 - 3.50 (m, 2 H) 2.96 - 3.05 (m, 2 H) 2.85 - 2.92 (m, 2 H) 2.21 - 2.28 (m, 1 H) 2.06 - 2.09 (s, 3 H) 1.10 - 1.14 (m, 2 H) 1.00 - 1.04 (m, 2 H)

### Example 13: Synthesis of N-(1-(3-cyanocyclobutyl)-3-(1-(3-methyltetrahydrofuran-3-yl)-6-oxo-1,6-dihydropyridin-3-yl)-1H-pyrrolo[2,3-c]pyridin-5-yl)acetamide

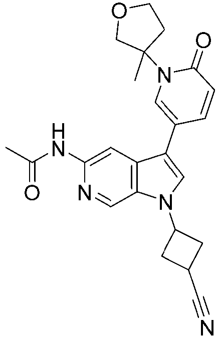

The compound was obtained from the method of Preparation Example 6.

¹H NMR (CHLOROFORM-d, 400 MHz) δ 8.61 (s, 1H), 8.44 (s, 1H), 8.39 (s, 1H), 8.02 (s, 1H), 7.82 (br d, 1H, J=8.5 Hz), 7.50 (s, 1H), 6.82 (d, 1H, J=8.5 Hz), 4.97 (br t, 1H, J=7.9 Hz), 4.29 (d, 1H, J=9.8 Hz), 3.9-4.0 (m, 3H), 3.1-3.2 (m, 3H), 2.9-3.0 (m, 2H), 2.6-2.7 (m, 1H), 2.24 (s, 3H), 2.1-2.2 (m, 1H), 1.82 (s, 3H)

### Example 14: Synthesis of 1-(1-methyl-3-(1-(3-methyltetrahydrofuran-3-yl)-6-oxo-1,6-dihydropyridin-3-yl)-1H-pyrrolo[2,3-c]pyridin-5-yl)urea

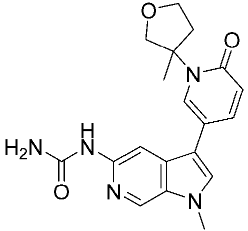

The compound was obtained from the method of Preparation Example 2.

¹H NMR (METHANOL-d4, 400 MHz) δ 8.5-8.6 (m, 1H), 8.4-8.4 (m, 1H), 7.9-8.0 (m, 1H), 7.68 (s, 3H), 6.8-6.9 (m, 1H), 4.2-4.3 (m, 1H), 3.9-4.0 (m, 6H), 2.6-2.7 (m, 1H), 2.1-2.2 (m, 1H), 1.8 (s, 3H)

### Example 15: Synthesis of N-(3-methyl-1-(1-(3-methyltetrahydrofuran-3-yl)-6-oxo-1,6-dihydropyridazin-3-yl)-1H-pyrrolo[3,2-c]pyridin-6-yl)acetamide

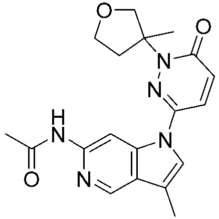

The compound was obtained from the method of Preparation Example 5.

¹H NMR (CHLOROFORM-d, 400 MHz) δ 8.68 (s, 1H), 8.56 (s, 1H), 8.09 (br s, 1H), 7.56 (d, 1H, J=9.9 Hz), 7.1-7.2 (m, 2H), 4.2-4.4 (m, 2H), 4.0-4.1 (m, 2H), 2.8-2.9 (m, 1H), 2.5-2.6 (m, 1H), 2.4 (s, 3H), 2.2 (s, 3H), 1.8 (s, 3H)

### Example 16: Synthesis of N-(5-(5-(3-methoxyazetidine-1-yl)-1-(3-methyltetrahydrofuran-3-yl)-6-oxo-1,6-dihydropyridazin-3-yl)-7-methylpyrrolo[1,2-c]pyrimidin-3-yl)acetamide

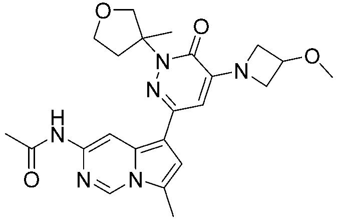

The compound was obtained from the method of Preparation Example 4.

¹H NMR (CHLOROFORM-d, 400 MHz) δ 8.6-8.7 (m, 1H), 8.4-8.5 (m, 1H), 7.5-7.6 (m, 1H), 6.8-6.8 (m, 1H), 6.10 (s, 1H), 4.4-4.6 (m, 3H), 4.3-4.4 (m, 1H), 4.2-4.3 (m, 1H), 4.1-4.2 (m, 2H), 4.0-4.1 (m, 2H), 3.3 (s, 3H), 2.8-2.9 (m, 1H), 2.6-2.7 (m, 1H), 2.5 (s, 3H), 2.2 (s, 3H), 1.7 (s, 3H)

### Example 17: Synthesis of N-(3-(5-(3-methoxyazetidin-1-yl)-1-(2-methoxyethyl)-6-oxo-1,6-dihydropyridazin-3-yl)-1-methyl-1H-pyrrolo[2,3-c]pyridin-5-yl)acetamide

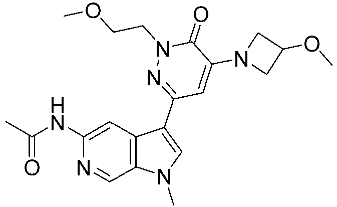

The compound was obtained from the method of Preparation Example 1.

¹H NMR (400 MHz, METHANOL- d4) δ 8.54 (s, 1 H) 8.85 (s, 1 H) 6.37 (s, 1 H) 7.96 (s, 1 H) 4.45 - 4.52 (m, 2 H) 4.37 - 4.42 (t, 2 H) 4.32 - 4.37 (m, 1 H) 4.07 - 4.15 (m, 2 H) 3.97 - 4.02 (m, 2 H) 3.95 (s, 3 H) 3.41 (s, 3 H) 3.37 (s, 3 H) 2.22 (s, 3 H)

### Example 18: Synthesis of N-(3-(1-(1-methoxy-2-methylpropan-2-yl)-6-oxo-1,6-dihydropyridazin-3-yl)-1-methyl-1H-pyrrolo[2,3-c]pyridin-5-yl)acetamide

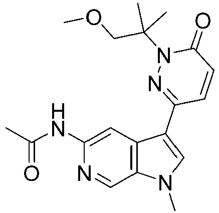

The compound was obtained from the method of Preparation Example 1.

¹H NMR (CHLOROFORM-d, 400 MHz) δ 8.77 (s, 1H), 8.48 (s, 1H), 8.0-8.1 (m, 2H), 7.8-7.9 (m, 1H), 7.02 (d, 1H, J=9.1 Hz), 3.9-4.0 (m, 3H), 3.8-3.9 (m, 2H), 3.4-3.5 (m, 3H), 2.2-2.3 (m, 4H), 1.70 (s, 6H)

### Example 19: Synthesis of N-(3-(5-isobutoxy-1-isopropyl-6-oxo-1,6-dihydropyridazin-3-yl)-1-methyl-1H-pyrrolo[2,3-c]pyridin-5-yl)acetamide

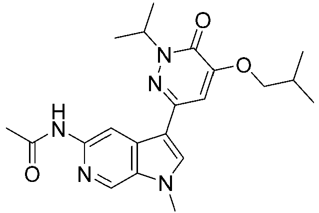

The compound was obtained from the method of Preparation Example 1.

¹H NMR (CHLOROFORM-d, 400 MHz) δ 9.08 (s, 1H), 8.45 (s, 1H), 7.95 (br s, 1H), 7.54 (s, 1H), 6.71 (s, 1H), 5.4-5.6 (m, 1H), 3.95 (s, 3H), 3.84 (d, 2H, J=6.6 Hz), 2.2-2.3 (m, 4H), 1.6-1.6 (m, 6H), 1.12 (d, 6H, J=6.8 Hz)

### Example 20: Synthesis of N-(3-(1-isopropyl-5-(3-methoxyazetidin-1-yl)-6-oxo-1,6-dihydropyridazin-3-yl)-1-methyl-1H-pyrrolo[2,3-c]pyridin-5-yl)acetamide

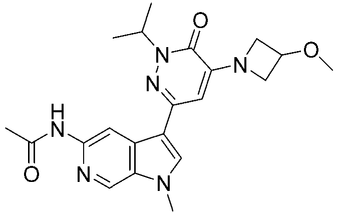

The compound was obtained from the method of Preparation Example 1.

¹H NMR (CHLOROFORM-d, 400 MHz) δ 9.03 (s, 1H), 8.4-8.5 (m, 1H), 7.9-8.0 (m, 1H), 7.51 (s, 1H), 6.09 (s, 1H), 5.3-5.4 (m, 1H), 4.53 (br s, 2H), 4.3-4.4 (m, 1H), 4.17 (br d, 2H, J=6.6 Hz), 3.92 (s, 3H), 3.36 (s, 3H), 2.25 (s, 3H), 1.5-1.6 (m, 6H)

### Example 21: Synthesis of N-(1-cyclopropyl-3-(1-isopropyl-5-(3-methoxyazetidin-1-yl)-6-oxo-1,6-dihydropyridazin-3-yl)-1H-pyrrolo[2,3-c]pyridin-5-yl)acetamide

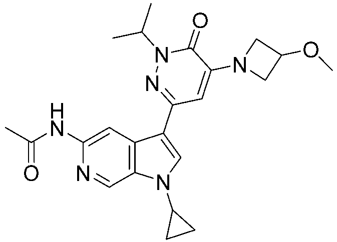

The compound was obtained from the method of Preparation Example 3.

¹H NMR (CHLOROFORM-d, 400 MHz) δ 9.01 (s, 1H), 8.5-8.7 (m, 1H), 7.99 (br s, 1H), 7.56 (s, 1H), 6.07 (s, 1H), 5.3-5.4 (m, 1H), 4.53 (br s, 2H), 4.35 (s, 1H), 4.1-4.3 (m, 2H), 3.48 (s, 1H), 3.36 (s, 3H), 2.2-2.3 (m, 4H), 1.5-1.6 (m, 6H), 1.2-1.2 (m, 2H), 1.12 (br d, 2H, J=4.4 Hz)

### Example 22: Synthesis of N-(1-(3-cyanocyclobutyl)-3-(5-isobutoxy-1-isopropyl-6-oxo-1,6-dihydropyridazin-3-yl)-1H-pyrrolo[2,3-c]pyridin-5-yl)acetamide

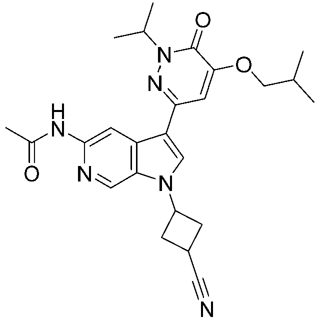

The compound was obtained from the method of Preparation Example 6.

¹H NMR (CHLOROFORM-d, 400 MHz) δ 9.11 (s, 1H), 8.43 (s, 1H), 7.97 (s, 1H), 7.70 (s, 1H), 6.74 (s, 1H), 5.4-5.6 (m, 1H), 5.00 (s, 1H), 3.90 (d, 2H, J=6.6 Hz), 3.1-3.2 (m, 3H), 2.9-3.1 (m, 2H), 2.3-2.4 (m, 1H), 2.26 (s, 3H), 1.5-1.6 (m, 6H), 1.14 (d, 6H, J=6.8 Hz)

### Example 23: Synthesis of N-(3-(1-cyclobutyl-5-isobutoxy-6-oxo-1,6-dihydropyridazin-3-yl)-1-methyl-1H-pyrrolo[2,3-c]pyridin-5-yl)acetamide

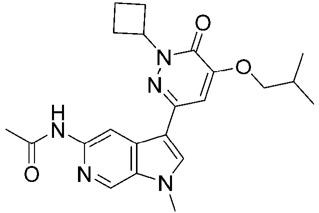

The compound was obtained from the method of Preparation Example 7.

¹H NMR (CHLOROFORM-d, 400 MHz) δ 9.12 (s, 1H), 8.46 (s, 1H), 7.9-8.0 (m, 1H), 7.5-7.6 (m, 1H), 6.72 (s, 1H), 5.6-5.7 (m, 1H), 3.9 (s, 3H), 3.8-3.9 (m, 2H), 2.7-2.9 (m, 2H), 2.4-2.6 (m, 2H), 2.2-2.3 (m, 4H), 2.0-2.2 (m, 1H), 1.8-2.0 (m, 1H), 1.0-1.2 (m, 6H)

"

### Example 24: Synthesis of N-(3-(1-cyclobutyl-5-(3-(methoxymethyl)azetidin-1-yl)-6-oxo-1,6-dihydropyridazin-3-yl)-1-methyl-1H-pyrrolo[2,3-c]pyridin-5-yl)acetamide

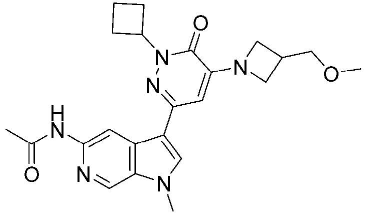

The compound was obtained from the method of Preparation Example 7.

¹H NMR (CHLOROFORM-d, 400 MHz) δ 9.05 (s, 1H), 8.42 (s, 1H), 7.99 (br s, 1H), 7.52 (s, 1H), 6.05 (s, 1H), 5.50 (t, 1H, J=8.4 Hz), 4.39 (br s, 2H), 4.08 (br s, 2H), 3.92 (s, 3H), 3.62 (d, 2H, J=6.6 Hz), 3.41 (s, 3H), 2.97 (br s, 1H), 2.80 (br t, 2H, J=10.4 Hz), 2.41 (br d, 2H, J=8.5 Hz), 2.24 (s, 3H), 2.05 (br d, 1H, J=9.9 Hz), 1.7-1.9 (m, 1H)

### Example 25: Synthesis of N-(3-(5-(isopentyloxy)-1-isopropyl-6-oxo-1,6-dihydropyridazin-3-yl)-1-methyl-1H-pyrrolo[2,3-c]pyridin-5-yl)acetamide

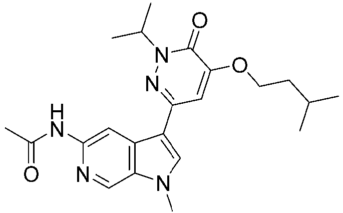

The compound was obtained from the method of Preparation Example 1.

¹H NMR (CHLOROFORM-d, 400 MHz) δ 9.08 (s, 1H), 8.45 (s, 1H), 8.06 (s, 1H), 7.54 (s, 1H), 6.72 (s, 1H), 5.4-5.5 (m, 1H), 4.12 (t, 2H, J=6.6 Hz), 3.94 (s, 3H), 2.26 (s, 3H), 1.8-2.0 (m, 4H), 1.57 (d, 6H, J=6.6 Hz), 1.01 (d, 6H, J=6.5 Hz)

### Example 26: Synthesis of N-(3-(1-isopropyl-6-oxo-5-(2,2,2-trifluoroethoxy)-1,6-dihydropyridazin-3-yl)-1-methyl-1H-pyrrolo[2,3-c]pyridin-5-yl)acetamide

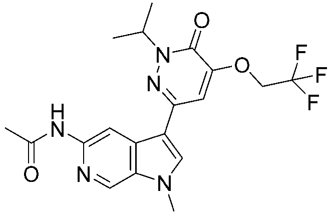

The compound was obtained from the method of Preparation Example 1.

¹H NMR (CHLOROFORM-d, 400 MHz) δ 9.08 (s, 1H), 8.46 (s, 1H), 7.99 (br s, 1H), 7.52 (s, 1H), 7.01 (s, 1H), 5.4-5.5 (m, 1H), 4.78 (q, 2H, J=8.2 Hz), 3.95 (s, 3H), 2.27 (s, 3H), 1.60 (d, 6H, J=6.6 Hz)

### Example 27: Synthesis of N-(3-(5-(benzyloxy)-1-isopropyl-6-oxo-1,6-dihydropyridazin-3-yl)-1-methyl-1H-pyrrolo[2,3-c]pyridin-5-yl)acetamide

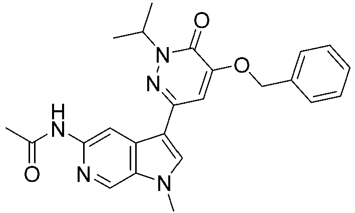

The compound was obtained from the method of Preparation Example 1.

¹H NMR (CHLOROFORM-d, 400 MHz) δ 9.05 (s, 1H), 8.43 (s, 1H), 8.08 (br s, 1H), 7.52 (d, 2H, J=7.3 Hz), 7.3-7.5 (m, 4H), 6.78 (s, 1H), 5.4-5.6 (m, 1H), 5.27 (s, 2H), 3.92 (s, 3H), 2.26 (s, 3H), 1.57 (d, 6H, J=6.6 Hz)

### Example 28: Synthesis of N-(3-(1-(tert-butyl)-5-isobutoxy-6-oxo-1,6-dihydropyridazin-3-yl)-1-methyl-1H-pyrrolo[2,3-c]pyridin-5-yl)acetamide

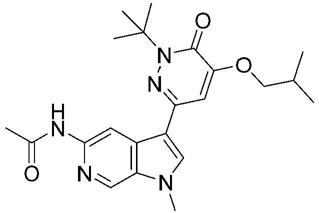

The compound was obtained from the method of Preparation Example 7.

¹H NMR (DMSO-d6, 400 MHz) δ 10.20 (s, 1H), 8.85 (s, 1H), 8.63 (d, 1H, J=1.0 Hz), 8.30 (s, 1H), 7.15 (s, 1H), 3.92 (s, 3H), 3.84 (d, 2H, J=6.6 Hz), 2.1-2.1 (m, 4H), 1.72 (s, 9H), 1.03 (d, 6H, J=6.8 Hz)

### Example 29: Synthesis of N-(3-(1-(tert-butyl)-5-(3-methoxyazetidin-1-yl)-6-oxo-1,6-dihydropyridazin-3-yl)-1-methyl-1H-pyrrolo[2,3-c]pyridin-5-yl)acetamide

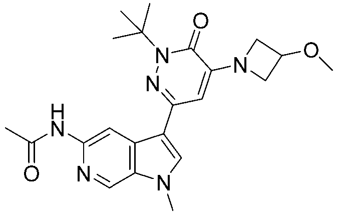

The compound was obtained from the method of Preparation Example 7.

¹H NMR (DMSO-d6, 400 MHz) δ 10.17 (s, 1H), 8.81 (s, 1H), 8.59 (d, 1H, J=0.9 Hz), 8.12 (s, 1H), 6.38 (s, 1H), 4.3-4.4 (m, 2H), 4.3-4.3 (m, 1H), 3.9-4.0 (m, 2H), 3.90 (s, 3H), 3.25 (s, 3H), 2.07 (s, 3H), 1.68 (s, 9H)

### Example 30: Synthesis of N-(3-(5-isobutoxy-1-(1-methylcyclopentyl)-6-oxo-1,6-dihydropyridazin-3-yl)-1-methyl-1H-pyrrolo[2,3-c]pyridin-5 -yl)acetamide

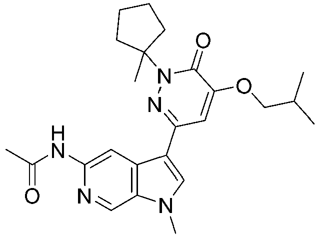

The compound was obtained from the method of Preparation Example 7.

¹H NMR (DMSO-d6, 400 MHz) δ 10.19 (s, 1H), 8.85 (s, 1H), 8.62 (d, 1H, J=0.9 Hz), 8.30 (s, 1H), 7.15 (s, 1H), 3.92 (s, 3H), 3.85 (d, 2H, J=6.6 Hz), 2.4-2.4 (m, 2H), 2.2-2.3 (m, 2H), 2.1-2.2 (m, 4H), 1.6-1.7 (m, 4H), 1.53 (s, 3H), 1.03 (d, 6H, J=6.8 Hz)

### Example 31: Synthesis of N-(3-(5-(3-methoxyazetidin-1-yl)-1-(1-methylcyclopentyl)-6-oxo-1,6-dihydropyridazin-3-yl)-1-methyl-1H-pyrrolo[2,3-c]pyridin-5-yl)acetamide

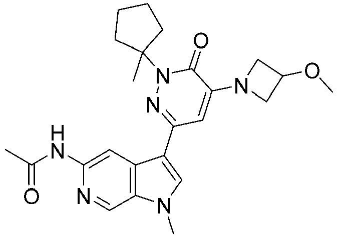

The compound was obtained from the method of Preparation Example 7.

¹H NMR (DMSO-d6, 400 MHz) δ 10.15 (s, 1H), 8.81 (s, 1H), 8.59 (d, 1H, J=1.0 Hz), 8.13 (s, 1H), 6.37 (s, 1H), 4.3-4.4 (m, 2H), 4.2-4.3 (m, 1H), 3.9-4.0 (m, 2H), 3.90 (s, 3H), 3.25 (s, 3H), 2.4-2.4 (m, 2H), 2.2-2.2 (m, 2H), 2.08 (s, 3H), 1.6-1.7 (m, 4H), 1.51 (s, 3H)

### Example 32: Synthesis of N-(3-(5-isobutoxy-1-(1-methylcyclobutyl)-6-oxo-1,6-dihydropyridazin-3-yl)-1-methyl-1H-pyrrolo[2,3-c]pyridin-5-yl)acetamide

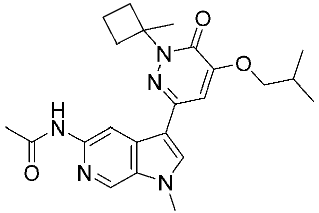

The compound was obtained from the method of Preparation Example 7.

¹H NMR (CHLOROFORM-d, 400 MHz) δ 8.9-9.0 (m, 1H), 8.4-8.5 (m, 1H), 8.1-8.1 (m, 1H), 7.53 (s, 1H), 6.70 (s, 1H), 5.32 (s, 12H), 3.94 (s, 3H), 3.82 (d, 2H, J=6.8 Hz), 3.51 (s, 3H), 2.7-2.8 (m, 2H), 2.5-2.6 (m, 2H), 2.25 (s, 3H), 1.9-2.0 (m, 1H), 1.8-1.9 (m, 1H), 1.11 (d, 7H, J=6.8 Hz)

### Example 33: Synthesis of N-(1-cyclobutyl-3-(5-isobutoxy-1-isopropyl-6-oxo-1,6-dihydropyridazin-3-yl)-1H-pyrrolo[2,3-c]pyridin-5-yl)acetamide

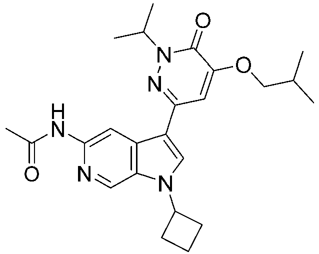

The compound was obtained from the method of Preparation Example 1.

¹H NMR (DMSO-d6, 400 MHz) δ 10.22 (s, 1H), 9.06 (s, 1H), 8.66 (d, 1H, J=0.9 Hz), 8.55 (s, 1H), 7.28 (s, 1H), 5.26 (quin, 1H, J=6.7 Hz), 5.14 (quin, 1H, J=8.4 Hz), 3.90 (d, 2H, J=6.5 Hz), 2.6-2.6 (m, 1H), 2.6-2.6 (m, 1H), 2.1-2.2 (m, 1H), 2.10 (s, 3H), 1.9-2.0 (m, 2H), 1.43 (d, 6H, J=6.6 Hz), 1.05 (d, 6H, J=6.8 Hz)

### Example 34: Synthesis of 1-(3-(5-isobutoxy-1-isopropyl-6-oxo-1,6-dihydropyridazin-3-yl)-1-methyl-1H-pyrrolo[2,3-c]pyridin-5-yl)urea

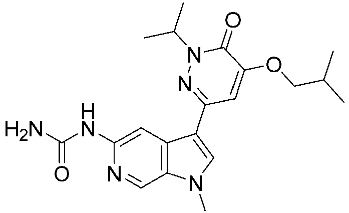

The compound was obtained from the method of Preparation Example 2.

¹H NMR (DMSO-d6, 400 MHz) δ 8.93 (s, 1H), 8.54 (d, 1H, J=1.0 Hz), 8.48 (br s, 1H), 8.29 (s, 1H), 7.14 (s, 1H), 6.5-6.6 (m, 2H), 5.25 (td, 1H, J=6.6, 13.4 Hz), 3.89 (s, 5H), 2.1-2.2 (m, 2H), 2.0-2.0 (m, 1H), 1.42 (d, 6H, J=6.6 Hz), 1.03 (d, 6H, J=6.8 Hz)

### Example 35: Synthesis of 1-(1-cyclobutyl-3-(5-isobutoxy-1-(1-methylcyclobutyl)-6-oxo-1,6-dihydropyridazin-3-yl)-1H-pyrrolo [2,3-c] pyridin-5-yl)urea

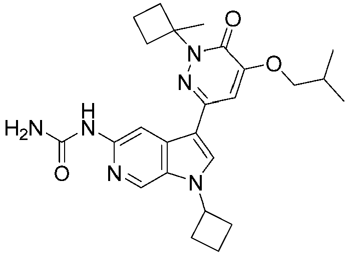

The compound was obtained from the method of Preparation Example 2.

¹H NMR (CHLOROFORM-d, 400 MHz) δ 8.45 (s, 1H), 7.5-7.7 (m, 2H), 6.99 (s, 1H), 6.65 (s, 1H), 4.92 (s, 1H), 3.82 (d, 2H, J=6.8 Hz), 2.6-2.8 (m, 4H), 2.55 (dt, 2H, J=2.4, 9.5 Hz), 2.4-2.5 (m, 2H), 2.2-2.4 (m, 1H), 1.9-2.1 (m, 3H), 1.88 (br s, 1H), 1.73 (s, 3H), 1.13 (d, 6H, J=6.6 Hz)

### Example 36: Synthesis of N-(1-cyclobutyl-3-(5-isobutoxy-6-oxo-1-(1,1,1-trifluoropropan-2-yl)-1,6-dihydropyridazin-3-yl)-1H-pyrrolo[2,3-c]pyridin-5-yl)acetamide

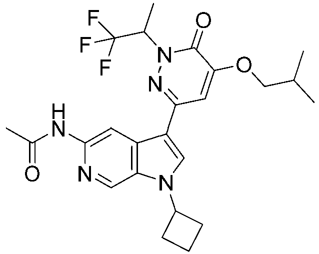

The compound was obtained from the method of Preparation Example 7.

¹H NMR (CHLOROFORM-d, 400 MHz) δ 8.95 (s, 1H), 8.47 (s, 1H), 8.04 (s, 1H), 7.69 (s, 1H), 6.77 (s, 1H), 5.9-6.1 (m, 1H), 4.94 (s, 1H), 4.23 (s, 1H), 3.89 (br d, 1H, J=3.3 Hz), 3.88 (br d, 1H, J=3.5 Hz), 2.6-2.8 (m, 2H), 2.5-2.6 (m, 2H), 2.2-2.3 (m, 4H), 2.05 (td, 2H, J=5.0, 9.9 Hz), 1.89 (d, 3H, J=7.1 Hz), 1.27 (s, 2H), 1.12 (d, 6H, J=6.8 Hz)

### Example 37: Synthesis of 1-(3-(5-isobutoxy-6-oxo-1-(1,1,1-trifluoropropan-2-yl)-1,6-dihydropyridazin-3-yl)-1-methyl-1H-pyrrolo[2,3-c]pyridin-5-yl)urea

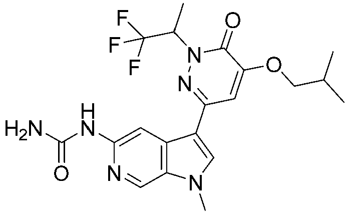

The compound was obtained from the method of Preparation Example 2.

¹H NMR (CHLOROFORM-d, 400 MHz) δ 8.4-8.5 (m, 1H), 7.6-7.6 (m, 1H), 7.5-7.6 (m, 1H), 6.9-7.0 (m, 1H), 6.67 (s, 1H), 5.9-6.0 (m, 1H), 3.9-4.0 (m, 3H), 3.8-3.9 (m, 2H), 2.2-2.4 (m, 1H), 1.7-1.8 (m, 3H), 1.0-1.1 (m, 6H)

### Example 38: Synthesis of 1-(1-cyclobutyl-3-(5-isobutoxy-1-isopropyl-6-oxo-1,6-dihydropyridazin-3-yl)-1H-pyrrolo[2,3-c]pyridin-5-yl)urea

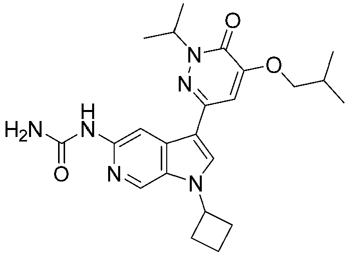

The compound was obtained from the method of Preparation Example 2.

¹H NMR (CHLOROFORM-d, 400 MHz) δ 8.4-8.5 (m, 1H), 7.6-7.7 (m, 2H), 7.0-7.0 (m, 1H), 6.6-6.7 (m, 1H), 5.4-5.5 (m, 1H), 4.9-5.0 (m, 1H), 3.8-3.9 (m, 2H), 2.6-2.8 (m, 2H), 2.5-2.6 (m, 2H), 2.3-2.4 (m, 1H), 2.0-2.1 (m, 2H), 1.50 (d, 6H, J=6.6 Hz), 1.13 (d, 6H, J=6.8 Hz)

### Example 39: Synthesis of N-(3-(5-(3-methoxyazetidin-1-yl)-6-oxo-1-(1,1,1-trifluoropropan-2-yl)-1,6-dihydropyridazin-3-yl)-1-methyl-1H-pyrrolo[2,3-c]pyridin-5-yl)acetamide

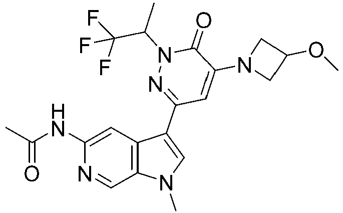

The compound was obtained from the method of Preparation Example 7.

¹H NMR (CHLOROFORM-d, 400 MHz) δ 8.91 (s, 1H), 8.42 (s, 1H), 7.9-8.0 (m, 1H), 7.53 (s, 1H), 6.1-6.1 (m, 1H), 5.8-5.9 (m, 1H), 4.5-4.6 (m, 2H), 4.3-4.4 (m, 1H), 4.2-4.2 (m, 2H), 3.9-4.0 (m, 3H), 3.9-4.0 (m, 4H), 3.37 (s, 3H), 2.25 (s, 4H), 1.8-1.9 (m, 3H)

### Example 40: Synthesis of N-(3-(5-(3-methoxyazetidin-1-yl)-1-(1-methylcyclobutyl)-6-oxo-1,6-dihydropyridazin-3-yl)-1-methyl-1H-pyrrolo[2,3-c]pyridin-5-yl)acetamide

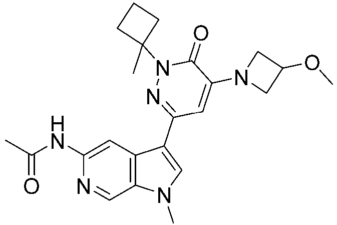

The compound was obtained from the method of Preparation Example 7.

¹H NMR (CHLOROFORM-d, 400 MHz) δ 8.91 (s, 1H), 8.41 (s, 1H), 8.02 (br s, 1H), 7.48 (s, 1H), 6.08 (s, 1H), 4.51 (br s, 2H), 4.3-4.4 (m, 1H), 4.0-4.2 (m, 2H), 3.91 (s, 3H), 3.3-3.4 (m, 3H), 2.6-2.8 (m, 2H), 2.47 (br t, 2H, J=9.1 Hz), 2.24 (s, 3H), 1.95 (br d, 1H, J=10.3 Hz), 1.8-1.9 (m, 2H)

### Example 41: Synthesis of N-(1-cyclobutyl-3-(5-(3-methoxyazetidin-1-yl)-1-(1-methylcyclobutyl)-6-oxo-1,6-dihydropyridazin-3-yl)-1H-pyrrolo[2,3-c]pyridin-5-yl)acetamide

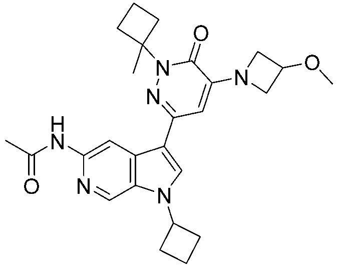

The compound was obtained from the method of Preparation Example 7.

¹H NMR (CHLOROFORM-d, 400 MHz) δ 8.90 (s, 1H), 8.43 (s, 1H), 8.12 (s, 1H), 7.62 (s, 1H), 6.10 (s, 1H), 4.91 (s, 1H), 4.52 (br s, 2H), 4.34 (s, 1H), 4.16 (br d, 2H, J=6.1 Hz), 3.34 (s, 3H), 2.7-2.8 (m, 2H), 2.66 (br dd, 2H, J=3.0, 5.6 Hz), 2.4-2.6 (m, 4H), 2.23 (s, 3H), 1.9-2.1 (m, 4H), 1.85 (br s, 1H), 1.79 (br s, 2H)

### Example 42: Synthesis of (S)-N-(3-(5-(3-methoxypyrrolidin-1-yl)-1-(1-methylcyclobutyl)-6-oxo-1,6-dihydropyridazin-3-yl)-1-methyl-1H-pyrrolo[2,3-c]pyridin-5-yl)acetamide

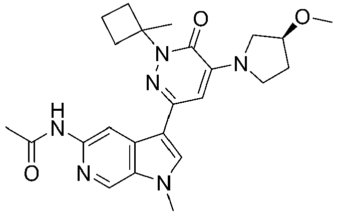

The compound was obtained from the method of Preparation Example 7.

¹H NMR (CHLOROFORM-d, 400 MHz) δ 8.9-8.9 (m, 1H), 8.4-8.5 (m, 1H), 7.9-7.9 (m, 1H), 7.51 (s, 1H), 6.2-6.2 (m, 1H), 4.0-4.1 (m, 3H), 3.9-4.0 (m, 3H), 3.7-3.8 (m, 2H), 3.3-3.4 (m, 3H), 2.7-2.8 (m, 2H), 2.4-2.5 (m, 2H), 2.2-2.3 (m, 2H), 2.2-2.3 (m, 1H), 1.7-1.8 (m, 3H), 0.8-0.9 (m, 4H)

### Example 43: Synthesis of (S)-N-(1-cyclobutyl-3-(5-(3-methoxypyrrolidin-1-yl)-1-(1-methylcyclobutyl)-6-oxo-1,6-dihydropyridazin-3-yl)-1H-pyrrolo[2,3-c]pyridin-5-yl)acetamide

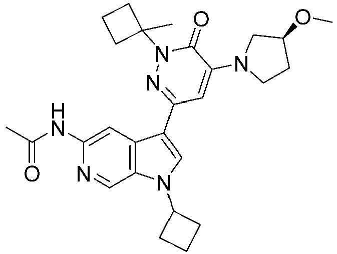

The compound was obtained from the method of Preparation Example 7.

¹H NMR (CHLOROFORM-d, 400 MHz) δ 8.90 (s, 1H), 8.43 (s, 1H), 7.91 (br s, 1H), 7.64 (s, 1H), 6.21 (s, 1H), 4.92 (s, 1H), 4.0-4.1 (m, 3H), 3.6-3.8 (m, 2H), 3.38 (s, 3H), 2.75 (br d, 2H, J=10.4 Hz), 2.6-2.7 (m, 2H), 2.4-2.6 (m, 4H), 2.24 (s, 3H), 2.03 (dt, 4H, J=4.9, 10.3 Hz), 1.85 (s, 1H), 1.73 (s, 3H)

### Example 44: Synthesis of N-(1-cyclobutyl-3-(1-(1-methylcyclobutyl)-6-oxo-5-(2-oxa-6-azaspiro[3.4]octan-6-yl)-1,6-dihydropyridazin-3- yl)-1H-pyrrolo[2,3-c]pyridin-5-yl)acetamide

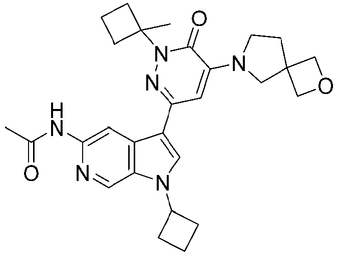

The compound was obtained from the method of Preparation Example 7.

¹H NMR (CHLOROFORM-d, 400 MHz) δ 8.90 (s, 1H), 8.44 (s, 1H), 8.02 (s, 1H), 7.64 (s, 1H), 6.21 (s, 1H), 4.92 (br t, 1H, J=8.4 Hz), 4.6-4.8 (m, 5H), 4.12 (s, 2H), 3.70 (br t, 2H, J=6.8 Hz), 2.6-2.8 (m, 4H), 2.4-2.6 (m, 4H), 2.2-2.3 (m, 5H), 1.9-2.1 (m, 4H), 1.8-1.9 (m, 1H), 1.34 (d, 1H, J=6.6 Hz)

### Example 45: Synthesis of N-(1-methyl-3-(1-(1-methylcyclobutyl)-6-oxo-5-(2-oxa-6-azaspiro[3.4]octan-6-yl)-1,6-dihydropyridazin-3- yl)-1H-pyrrolo[2,3-c]pyridin-5-yl)acetamide

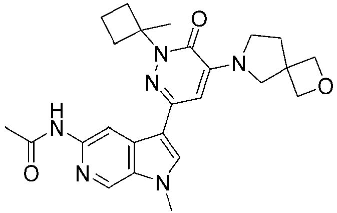

The compound was obtained from the method of Preparation Example 7.

¹H NMR (CHLOROFORM-d, 400 MHz) δ 8.91 (s, 1H), 8.42 (s, 1H), 7.96 (br s, 1H), 7.51 (s, 1H), 6.21 (s, 1H), 5.32 (s, 1H), 4.74 (d, 2H, J=6.1 Hz), 4.64 (d, 2H, J=6.0 Hz), 4.11 (s, 2H), 3.92 (s, 3H), 3.69 (br t, 2H, J=6.8 Hz), 2.7-2.8 (m, 2H), 2.4-2.6 (m, 2H), 2.2-2.3 (m, 5H), 1.95 (br d, 1H, J=10.5 Hz), 1.84 (br d, 1H, J=10.5 Hz), 1.73 (s, 3H), 1.28 (s, 1H)

### Example 46: Synthesis of N-(1-methyl-3-(1-(1-methylcyclobutyl)-6-oxo-5-(2-oxa-6-azaspiro[3.3]heptan-6-yl)-1,6-dihydropyridazin-3-yl)-1H-pyrrolo[2,3-c]pyridin-5-yl)acetamide

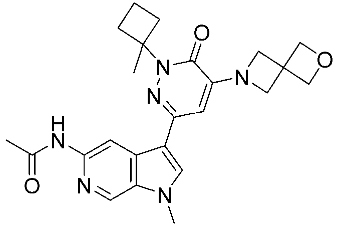

The compound was obtained from the method of Preparation Example 7.

¹H NMR (DMSO-d6, 400 MHz) δ 10.15 (s, 1H), 8.85 (s, 1H), 8.58 (d, 1H, J=1.0 Hz), 8.15 (s, 1H), 6.34 (s, 1H), 4.72 (s, 4H), 4.33 (br s, 3H), 3.90 (s, 3H), 2.07 (s, 3H), 2.0-2.0 (m, 3H), 1.8-1.9 (m, 1H), 1.7-1.7 (m, 1H), 1.55 (s, 3H), 1.4-1.5 (m, 2H)

### Example 47: Synthesis of N-(3-(5-(cyanomethoxy)-1-(1-methylcyclobutyl)-6-oxo-1,6-dihydropyridazin-3-yl)-1-methyl-1H-pyrrolo[2,3-c]pyridin-5-yl)acetamide

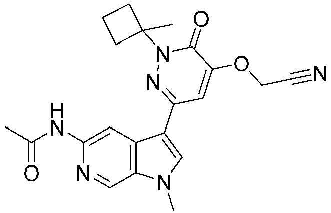

The compound was obtained from the method of Preparation Example 7.

¹H NMR (DMSO-d6, 400 MHz) δ 10.22 (s, 1H), 8.89 (s, 1H), 8.65 (d, 1H, J=1.0 Hz), 8.26 (s, 1H), 7.44 (s, 1H), 5.31 (s, 2H), 3.94 (s, 3H), 2.6-2.6 (m, 2H), 2.4-2.4 (m, 2H), 2.09 (s, 3H), 1.8-1.9 (m, 1H), 1.7-1.8 (m, 1H), 1.60 (s, 3H)

### Example 48: Synthesis of N-(1-methyl-3-(1-(1-methylcyclobutyl)-5-(oxetan-3-ylmethoxy)-6-oxo-1,6-dihydropyridazin-3-yl)-1H-pyrrolo[2,3-c]pyridin-5-yl)acetamide

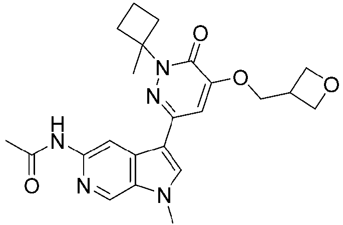

The compound was obtained from the method of Preparation Example 7.

¹H NMR (400 MHz, CHLOROFORM- d) δ 8.96 (s, 1 H) 8.45 (s, 1 H) 7.58 (s, 1 H) 6.82 (s, 1 H) 4.93 - 5.01 (m, 2 H) 4.55 - 4.61 (m, 2 H) 4.38 (d, J=7.25 Hz, 2 H) 3.93 - 3.97 (m, 3 H) 3.58 - 3.67 (m, 1 H) 2.68 - 2.80 (m, 2 H) 2.46 - 2.56 (m, 2 H) 1.84 - 2.00 (m, 2 H) 1.75 (s, 3 H) 1.28 (s, 3 H)

### Example 49: Synthesis of N-(1-cyclopropyl-3-(5-isobutoxy-6-oxo-1-(1,1,1-trifluoropropan-2-yl)-1,6-dihydropyridazin-3-yl)-1H-pyrrolo[2,3-c]pyridin-5-yl)acetamide

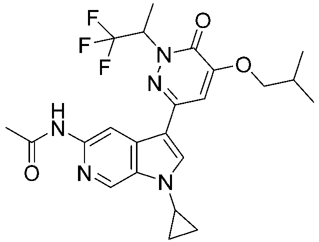

The compound was obtained from the method of Preparation Example 3.

¹H NMR (DMSO-d6, 400 MHz) δ 10.25 (s, 1H), 8.95 (s, 1H), 8.69 (d, 1H, J=1.1 Hz), 8.43 (s, 1H), 7.32 (s, 1H), 5.8-5.9 (m, 1H), 3.9-4.0 (m, 2H), 3.6-3.7 (m, 1H), 2.1-2.2 (m, 1H), 2.09 (s, 3H), 1.81 (d, 3H, J=7.1 Hz), 1.1-1.2 (m, 2H), 1.1-1.1 (m, 2H), 1.04 (d, 6H, J=6.6 Hz)

### Example 50: Synthesis of (R)-N-(3-(5-isobutoxy-6-oxo-1-(1,1,1-trifluoropropan-2-yl)-1,6-dihydropyridazin-3-yl)-1-methyl-1H-pyrrolo[2,3-c]pyridin-5-yl)acetamide

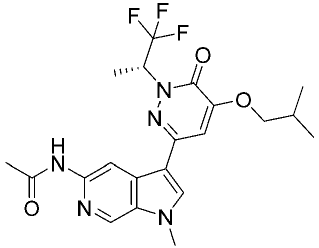

The compound was obtained from the method of Preparation Example 7.

¹H NMR (DMSO-d6, 400 MHz) δ 10.22 (s, 1H), 8.94 (s, 1H), 8.65 (d, 1H, J=1.0 Hz), 8.40 (s, 1H), 7.25 (s, 1H), 5.8-5.9 (m, 1H), 3.9-4.0 (m, 5H), 2.1-2.2 (m, 4H), 2.0-2.0 (m, 1H), 1.81 (d, 3H, J=7.1 Hz), 1.04 (d, 6H, J=6.6 Hz)

### Example 51: Synthesis of N-(3-(5-isobutoxy-6-oxo-1-(1,1,1-trifluoropropan-2-yl)-1,6-dihydropyridazin-3-yl)-1-methyl-1H-pyrrolo[2,3-c]pyridin-5-yl)acetamide

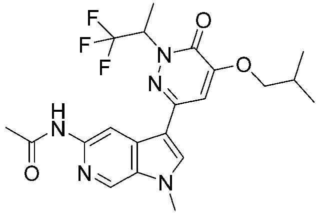

The compound was obtained from the method of Preparation Example 7.

¹H NMR (DMSO-d6, 400 MHz) δ 10.22 (s, 1H), 8.94 (s, 1H), 8.65 (d, 1H, J=1.0 Hz), 8.40 (s, 1H), 7.25 (s, 1H), 5.85 (quin, 1H, J=7.3 Hz), 3.9-4.0 (m, 5H), 2.1-2.2 (m, 4H), 1.9-2.1 (m, 1H), 1.81 (d, 3H, J=7.1 Hz), 1.04 (d, 6H, J=6.6 Hz)

### Example 52: Synthesis of N-(1-cyclopropyl-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide

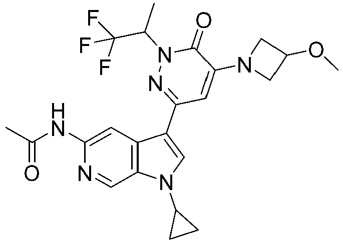

The compound was obtained from the method of Preparation Example 3.

¹H NMR (CHLOROFORM-d, 400 MHz) δ 8.90 (s, 1H), 8.62 (s, 1H), 8.04 (br s, 1H), 7.57 (s, 1H), 6.08 (s, 1H), 5.84 (br d, 1H, J=7.3 Hz), 5.32 (s, 2H), 4.5-4.7 (m, 2H), 4.35 (s, 1H), 4.20 (br s, 2H), 3.47 (s, 1H), 3.36 (s, 3H), 2.25 (s, 3H), 1.8-1.9 (m, 3H), 1.2-1.4 (m, 5H), 1.0-1.2 (m, 5H), 0.90 (br s, 2H)

### Example 53: Synthesis of N-(1-cyclopropyl-3-(1-(1-methylcyclobutyl)-6-oxo-5-(2-oxa-6-azaspiro[3.4]octan-6-yl)-1,6-dihydropyridazin-3-yl)-1H-pyrrolo[2,3-c]pyridin-5-yl)acetamide

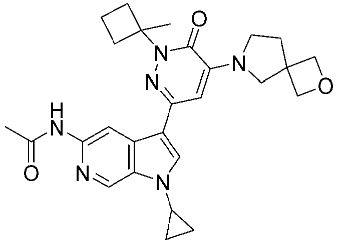

The compound was obtained from the method of Preparation Example 3.

¹H NMR (CHLOROFORM-d, 400 MHz) δ 8.89 (s, 1H), 8.61 (s, 1H), 7.93 (br s, 1H), 7.56 (s, 1H), 6.18 (s, 1H), 4.75 (d, 2H, J=6.1 Hz), 4.64 (d, 2H, J=6.1 Hz), 4.11 (s, 2H), 3.69 (br t, 2H, J=6.8 Hz), 3.4-3.6 (m, 1H), 2.73 (br d, 2H, J=12.1 Hz), 2.4-2.6 (m, 2H), 2.2-2.3 (m, 5H), 1.95 (br d, 1H, J=10.4 Hz), 1.84 (br d, 1H, J=9.8 Hz), 1.73 (s, 3H), 1.1-1.2 (m, 4H)

### Example 54: Synthesis of (S)-N-(3-(5-isobutoxy-6-oxo-1-(1,1,1-trifluoropropan-2-yl)-1,6-dihydropyridazin-3-yl)-1-methyl-1H-pyrrolo[2,3-c]pyridin-5-yl)acetamide

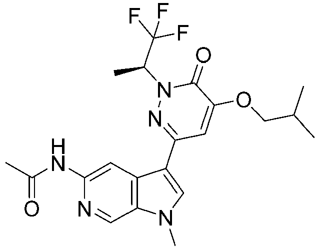

The compound was obtained from the method of Preparation Example 7.

¹H NMR (DMSO-d6, 400 MHz) δ 10.22 (s, 1H), 8.94 (s, 1H), 8.65 (d, 1H, J=0.9 Hz), 8.40 (s, 1H), 7.25 (s, 1H), 5.8-5.9 (m, 1H), 3.9-4.0 (m, 5H), 2.1-2.2 (m, 4H), 1.81 (d, 3H, J=7.1 Hz), 1.03 (d, 6H, J=6.6 Hz)

### Example 55: Synthesis of N-(3-(5-(cyclobutylmethoxy)-6-oxo-1-(1,1,1-trifluoropropan-2-yl)-1,6-dihydropyridazin-3-yl)-1-methyl-1H-pyrrolo[2,3-c]pyridin-5-yl)acetamide

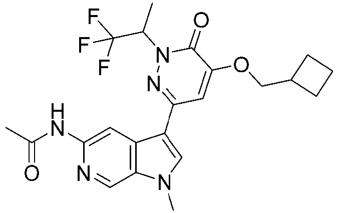

The compound was obtained from the method of Preparation Example 7.

¹H NMR (DMSO-d6, 400 MHz) δ 10.22 (s, 1H), 8.94 (s, 1H), 8.65 (d, 1H, J=1.0 Hz), 8.40 (s, 1H), 7.27 (s, 1H), 5.84 (td, 1H, J=7.1, 14.6 Hz), 4.1-4.2 (m, 2H), 3.94 (s, 3H), 2.8-2.9 (m, 1H), 2.1-2.2 (m, 5H), 1.8-2.0 (m, 7H)

### Example 56: Synthesis of N-(3-(5-(2,2-difluoropropoxy)-6-oxo-1-(1,1,1-trifluoropropan-2-yl)-1,6-dihydropyridazin-3-yl)-1-methyl-1H-pyrrolo[2,3-c]pyridin-5-yl)acetamide

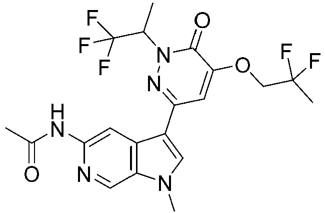

The compound was obtained from the method of Preparation Example 7.

¹H NMR (DMSO-d6, 400 MHz) δ 10.24 (s, 1H), 8.95 (s, 1H), 8.66 (d, 1H, J=1.0 Hz), 8.37 (s, 1H), 7.42 (s, 1H), 5.86 (quin, 1H, J=7.3 Hz), 4.49 (t, 2H, J=12.3 Hz), 3.94 (s, 3H), 2.10 (s, 3H), 1.7-1.9 (m, 6H)

### Example 57: Synthesis of (R)-N-(3-(1-(1-ethylcyclobutyl)-5-(2-methoxypropoxy)-6-oxo-1,6-dihydropyridazin-3-yl)-1-methyl-1H-pyrrolo[2,3-c]pyridin-5-yl)acetamide

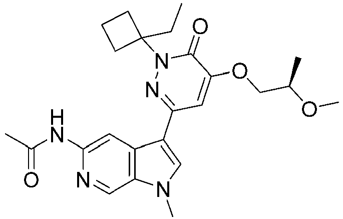

The compound was obtained from the method of Preparation Example 7.

¹H NMR (CHLOROFORM-d, 400 MHz) δ 9.00 (s, 1H), 8.41 (s, 2H), 7.51 (s, 1H), 6.76 (s, 1H), 4.0-4.2 (m, 1H), 3.9-4.0 (m, 5H), 3.5-3.5 (m, 3H), 2.7-2.8 (m, 2H), 2.5-2.6 (m, 2H), 2.2-2.3 (m, 5H), 1.8-2.0 (m, 3H), 1.3-1.4 (m, 5H), 0.8-0.9 (m, 6H)

### Example 58: Synthesis of (S)-N-(1-cyclopropyl-3-(6-oxo-5-(2-oxa-6-azaspiro [3.4] octan-6-yl)-1-(1,1,1-trifluoropropan-2 -yl)-1,6-dihydropyridazin-3-yl)-1H-pyrrolo[2,3-c]pyridin-5-yl)acetamide

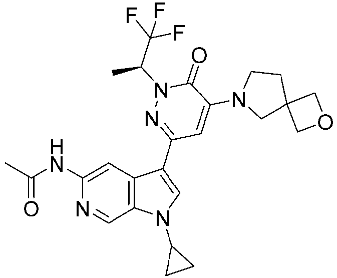

The compound was obtained from the method of Preparation Example 3.

¹H NMR (CHLOROFORM-d, 400 MHz) δ 8.8-9.0 (m, 1H), 8.6-8.7 (m, 1H), 7.9-8.0 (m, 1H), 7.5-7.7 (m, 1H), 6.2-6.3 (m, 1H), 5.8-6.0 (m, 1H), 4.6-4.8 (m, 4H), 4.0-4.2 (m, 2H), 3.7-3.9 (m, 2H), 3.4-3.6 (m, 1H), 2.2-2.4 (m, 5H), 1.8-1.9 (m, 3H), 1.1-1.3 (m, 4H)

### Example 59: Synthesis of N-(3-(1-(3,3-difluorobutan-2-yl)-5-isobutoxy-6-oxo-1,6-dihydropyridazin-3-yl)-1-methyl-1H-pyrrolo[2,3-c]pyridin-5-yl)acetamide

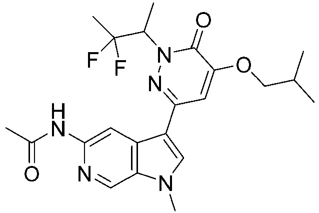

The compound was obtained from the method of Preparation Example 7.

¹H NMR (DMSO-d6, 400 MHz) δ 10.26 (s, 1H), 9.00 (s, 1H), 8.65 (d, 1H, J=1.0 Hz), 8.39 (s, 1H), 7.22 (s, 1H), 5.5-5.6 (m, 1H), 3.8-4.0 (m, 5H), 2.1-2.2 (m, 4H), 1.7-1.8 (m, 6H), 1.03 (d, 6H, J=6.8 Hz)

### Example 60: Synthesis of N-(3-(1-(1-ethylcyclobutyl)-5-isobutoxy-6-oxo-1,6-dihydropyridazin-3-yl)-1-methyl-1H-pyrrolo[2,3-c]pyridin-5-yl)acetamide

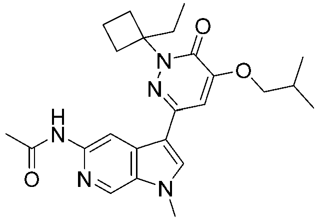

The compound was obtained from the method of Preparation Example 7.

¹H NMR (DMSO-d6, 400 MHz) δ 10.18 (s, 1H), 8.91 (s, 1H), 8.61 (d, 1H, J=1.1 Hz), 8.30 (s, 1H), 7.16 (s, 1H), 3.91 (s, 3H), 3.85 (d, 2H, J=6.6 Hz), 2.5-2.6 (m, 2H), 2.4-2.5 (m, 2H), 2.0-2.2 (m, 6H), 1.7-1.9 (m, 2H), 1.03 (d, 6H, J=6.6 Hz), 0.79 (t, 3H, J=7.4 Hz)

### Example 61: Synthesis of (S)-N-(3-(5-(3-methoxyazetidin-1-yl)-6-oxo-1-(1,1,1-trifluoropropan-2-yl)-1,6-dihydropyridazin-3-yl)-1-methyl-1H-pyrrolo[2,3-c]pyridin-5-yl)acetamide

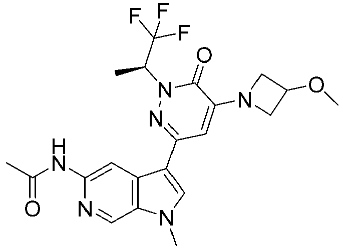

The compound was obtained from the method of Preparation Example 7.

¹H NMR (DMSO-d6, 400 MHz) δ 10.18 (s, 1H), 8.90 (s, 1H), 8.61 (d, 1H, J=1.0 Hz), 8.22 (s, 1H), 6.43 (s, 1H), 5.7-5.8 (m, 1H), 4.4-4.5 (m, 2H), 4.3-4.3 (m, 1H), 4.02 (br d, 2H, J=2.3 Hz), 3.91 (s, 3H), 3.26 (s, 3H), 2.08 (s, 3H), 1.77 (d, 3H, J=7.0 Hz)

### Example 62: Synthesis of (S)-N-(3-(5-(2,2-difluoropropoxy)-6-oxo-1-(1,1,1-trifluoropropan-2-yl)-1,6-dihydropyridazin-3-yl)-1-methyl-1H-pyrrolo[2,3-c]pyridin-5-yl)acetamide

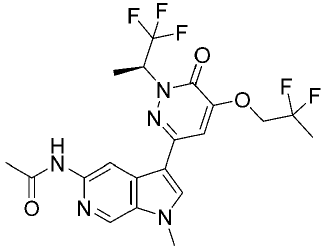

The compound was obtained from the method of Preparation Example 7.

¹H NMR (DMSO-d6, 400 MHz) δ 10.24 (s, 1H), 8.95 (s, 1H), 8.66 (d, 1H, J=1.0 Hz), 8.37 (s, 1H), 7.42 (s, 1H), 5.8-5.9 (m, 1H), 4.49 (t, 2H, J=12.3 Hz), 3.94 (s, 3H), 2.10 (s, 3H), 1.7-1.9 (m, 6H)

### Example 63: Synthesis of N-(1-((1S,3R)-3-cyanocyclobutyl)-3-(5-isobutoxy-6-oxo-1-((S)-1,1,1-trifluoropropan-2-yl)-1,6-dihydropyridazin-3-yl)-1H-pyrrolo[2,3-c]pyridin-5-yl)acetamide

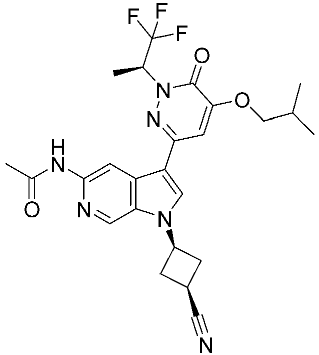

The compound was obtained from the method of Preparation Example 6.

¹H NMR (DMSO-d6, 400 MHz) δ 10.24 (s, 1H), 8.96 (s, 1H), 8.68 (s, 1H), 8.65 (d, 1H, J=0.9 Hz), 7.35 (s, 1H), 5.86 (quin, 1H, J=7.3 Hz), 5.1-5.2 (m, 1H), 3.97 (dq, 2H, J=6.6, 9.4 Hz), 3.2-3.3 (m, 1H), 3.01 (dq, 2H, J=2.4, 8.0 Hz), 2.8-3.0 (m, 2H), 2.16 (td, 1H, J=6.6, 13.3 Hz), 2.09 (s, 3H), 1.82 (d, 3H, J=7.0 Hz), 1.07 (d, 6H, J=6.8 Hz)

### Example 64: Synthesis of (S)-N-(1-cyclopropyl-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide

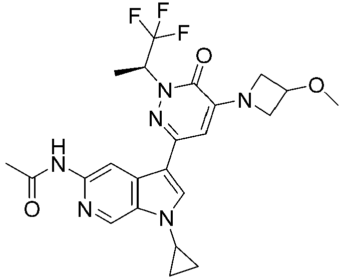

The compound was obtained from the method of Preparation Example 3.

¹H NMR (CHLOROFORM-d, 400 MHz) δ 8.89 (s, 1H), 8.62 (s, 1H), 8.00 (br s, 1H), 7.57 (s, 1H), 6.08 (s, 1H), 5.8-5.9 (m, 1H), 4.5-4.6 (m, 2H), 4.3-4.4 (m, 1H), 4.20 (br s, 2H), 3.48 (br d, 1H, J=3.8 Hz), 3.36 (s, 3H), 2.25 (s, 3H), 1.7-1.9 (m, 3H), 1.37 (s, 1H), 1.1-1.3 (m, 5H)

### Example 65: Synthesis of N-(1-cyclopropyl-3-(5-((R)-2-methoxypropoxy)-6-oxo-1-((S)-1,1,1-trifluoropropan-2-yl)-1,6-dihydropyridazin-3-yl)-1H-pyrrolo[2,3-c]pyridin-5-yl)acetamide

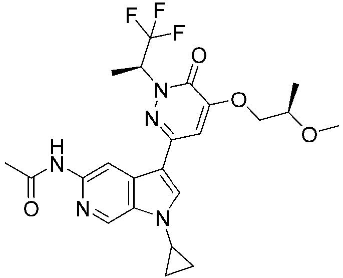

The compound was obtained from the method of Preparation Example 3.

¹H NMR (CHLOROFORM-d, 400 MHz) δ 8.95 (s, 1H), 8.65 (s, 1H), 8.00 (br s, 1H), 7.61 (s, 1H), 6.83 (s, 1H), 5.9-6.0 (m, 1H), 4.17 (dd, 1H, J=6.2, 9.7 Hz), 4.01 (dd, 1H, J=4.8, 9.8 Hz), 3.91 (br d, 1H, J=5.4 Hz), 3.50 (s, 4H), 2.25 (s, 3H), 1.7-1.9 (m, 3H), 1.35 (d, 3H, J=6.3 Hz), 1.2-1.3 (m, 2H), 1.1-1.2 (m, 2H)

### Example 66: Synthesis of (S)-N-(1-cyclopropyl-3-(5-(2-methoxyethoxy)-6-oxo-1-(1,1,1-trifluoropropan-2-yl)-1,6-dihydropyridazin-3-yl)-1H-pyrrolo[2,3-c]pyridin-5-yl)acetamide

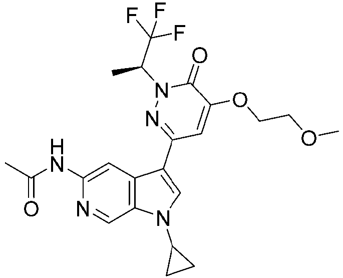

The compound was obtained from the method of Preparation Example 3.

¹H NMR (CHLOROFORM-d, 400 MHz) δ 8.94 (s, 1H), 8.64 (s, 1H), 8.19 (br s, 1H), 7.61 (s, 1H), 6.87 (s, 1H), 5.9-6.0 (m, 1H), 4.31 (t, 2H, J=4.8 Hz), 4.14 (d, 1H, J=7.1 Hz), 3.8-4.0 (m, 2H), 3.5-3.5 (m, 4H), 2.25 (s, 3H), 2.06 (s, 1H), 1.87 (d, 3H, J=7.1 Hz), 1.2-1.3 (m, 3H), 1.12 (br d, 2H, J=3.3 Hz), 0.90 (s, 2H)

### Example 67: Synthesis of (S)-N-(3-(5-(3,3-difluoropyrrolidin-1-yl)-6-oxo-1-(1,1,1-trifluoropropan-2-yl)-1,6-dihydropyridazin-3-yl)-1-methyl-1H-pyrrolo[2,3-c]pyridin-5-yl)acetamide

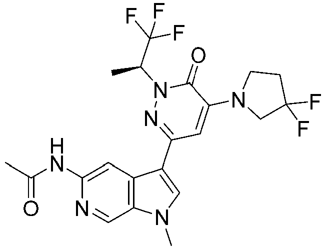

The compound was obtained from the method of Preparation Example 7.

¹H NMR (CHLOROFORM-d, 400 MHz) δ 8.90 (s, 1H), 8.43 (s, 1H), 8.00 (br s, 1H), 7.54 (s, 1H), 6.28 (s, 1H), 5.8-6.0 (m, 1H), 4.29 (br t, 2H, J=13.5 Hz), 3.8-3.9 (m, 5H), 2.4-2.6 (m, 2H), 2.25 (s, 3H), 2.03 (s, 1H), 1.7-1.9 (m, 3H)

### Example 68: Synthesis of N-(1-((1S,3R)-3-cyanocyclobutyl)-3-(5-(2,2-difluoropropoxy)-6-oxo-1-((S)-1,1,1-trifluoropropan-2-yl)-1,6-dihydropyridazin-3-yl)-1H-pyrrolo[2,3-c]pyridin-5-yl)acetamide

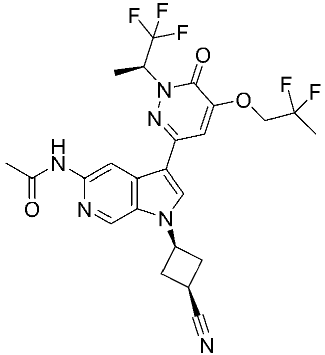

The compound was obtained from the method of Preparation Example 6.

¹H NMR (DMSO-d6, 400 MHz) δ 10.25 (s, 1H), 8.96 (s, 1H), 8.66 (s, 2H), 7.49 (s, 1H), 5.8-5.9 (m, 1H), 5.1-5.2 (m, 1H), 4.52 (t, 2H, J=12.0 Hz), 3.0-3.1 (m, 1H), 2.8-2.9 (m, 1H), 2.09 (s, 3H), 2.01 (q, 1H, J=7.1 Hz), 1.8-1.9 (m, 6H)

### Example 69: Synthesis of (S)-N-(3-(5-(3-methoxyazetidin-1-yl)-6-oxo-1-(1,1,1-trifluoropropan-2-yl)-1,6-dihydropyridazin-3-yl)-1-methyl-1H-pyrrolo[2,3-c]pyridin-5-yl)acetamide-2,2,2-d3

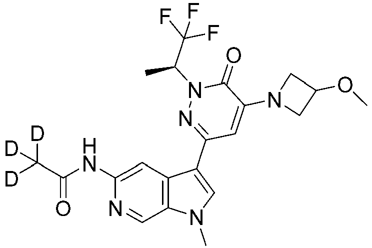

The compound was obtained from the method of Preparation Example 7.

¹H NMR (CHLOROFORM-d, 400 MHz) δ 8.90 (s, 1H), 8.39 (s, 1H), 8.00 (br s, 1H), 7.49 (s, 1H), 6.07 (s, 1H), 5.8-6.0 (m, 1H), 4.53 (br s, 2H), 4.3-4.4 (m, 1H), 4.19 (br s, 2H), 3.89 (s, 3H), 3.36 (s, 3H), 1.7-1.9 (m, 3H)

### Example 70: Synthesis of (S)-N-(3-(5-(2,2-difluoroethoxy)-6-oxo-1-(1,1,1-trifluoropropan-2-yl)-1,6-dihydropyridazin-3-yl)-1-methyl-1H-pyrrolo[2,3-c]pyridin-5-yl)acetamide

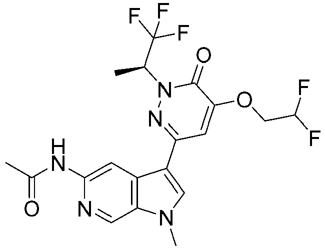

The compound was obtained from the method of Preparation Example 7.

¹H NMR (CHLOROFORM-d, 400 MHz) δ 8.9-9.1 (m, 1H), 8.8-8.9 (m, 1H), 8.4-8.5 (m, 1H), 7.9-8.0 (m, 1H), 7.5-7.6 (m, 1H), 6.8-7.0 (m, 1H), 6.1-6.4 (m, 1H), 5.9-6.0 (m, 1H), 5.9-5.9 (m, 1H), 4.3-4.5 (m, 2H), 3.8-4.0 (m, 3H), 2.2-2.3 (m, 3H), 1.8-2.0 (m, 3H)

### Example 71: Synthesis of (S)-N-(1-cyclopropyl-3-(5-(2,2-difluoroethoxy)-6-oxo-1-(1,1,1-trifluoropropan-2-yl)-1,6-dihydropyridazin-3-yl)-1H-pyrrolo[2,3-c]pyridin-5-yl)acetamide

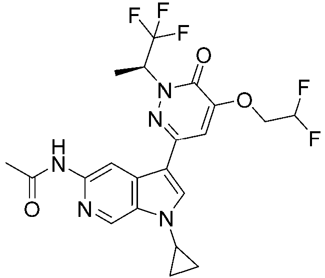

The compound was obtained from the method of Preparation Example 3.

¹H NMR (CHLOROFORM-d, 400 MHz) δ 8.9-9.0 (m, 1H), 8.6-8.7 (m, 1H), 7.8-8.0 (m, 1H), 7.5-7.7 (m, 1H), 6.8-7.0 (m, 1H), 6.1-6.4 (m, 1H), 5.9-6.0 (m, 1H), 4.4-4.5 (m, 2H), 3.4-3.6 (m, 1H), 2.1-2.3 (m, 3H), 1.8-2.0 (m, 3H), 1.0-1.3 (m, 4H)

### Example 72: Synthesis of (S)-N-(3-(5-(2,2-difluoropropoxy)-6-oxo-1-(1,1,1-trifluoropropan-2-yl)-1,6-dihydropyridazin-3-yl)-1-methyl-1H-pyrrolo[2,3-c]pyridin-5-yl)acetamide-2,2,2-d3

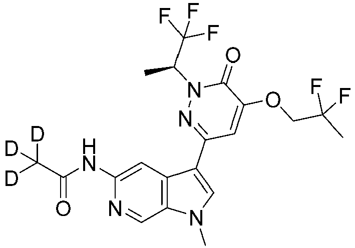

The compound was obtained from the method of Preparation Example 7.

¹H NMR (DMSO-d6, 400 MHz) δ 10.24 (s, 1H), 8.95 (s, 1H), 8.66 (d, 1H, J=1.0 Hz), 8.37 (s, 1H), 7.42 (s, 1H), 5.86 (quin, 1H, J=7.3 Hz), 4.49 (t, 2H, J=12.3 Hz), 3.94 (s, 3H), 1.7-1.9 (m, 6H)

### Example 73: Synthesis of (S)-N-(3-(5-(2,2-difluoropropoxy)-6-oxo-1-(1,1,1-trifluoropropan-2-yl)-1,6-dihydropyridazin-3-yl)-1-(methyl-d3)-1H-pyrrolo[2,3-c]pyridin-5-yl)acetamide

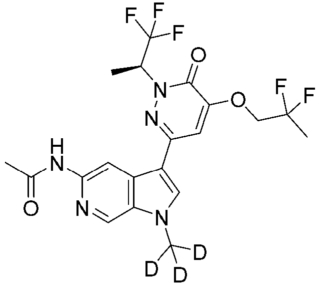

The compound was obtained from the method of Preparation Example 7.

¹H NMR (DMSO-d6, 400 MHz) δ 10.24 (s, 1H), 8.95 (s, 1H), 8.66 (d, 1H, J=1.0 Hz), 8.37 (s, 1H), 7.42 (s, 1H), 5.8-5.9 (m, 1H), 4.49 (t, 2H, J=12.3 Hz), 2.10 (s, 3H), 1.7-1.9 (m, 6H)

### Example 74: Synthesis of (S)-N-(1-cyclopropyl-3-(5-(2,2-difluoropropoxy)-6-oxo-1-(1,1,1-trifluoropropan-2-yl)-1,6-dihydropyridazin-3-yl)-1H-pyrrolo[2,3-c]pyridin-5-yl)acetamide

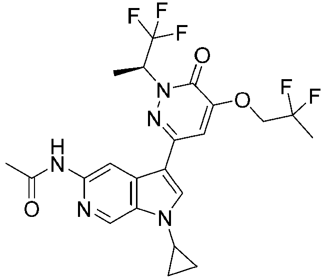

The compound was obtained from the method of Preparation Example 3.

¹H NMR (DMSO-d6, 400 MHz) δ 10.27 (s, 1H), 8.95 (s, 1H), 8.70 (d, 1H, J=1.0 Hz), 8.42 (s, 1H), 7.47 (s, 1H), 5.85 (quin, 1H, J=7.4 Hz), 4.49 (t, 2H, J=12.3 Hz), 3.68 (tt, 1H, J=3.7, 7.1 Hz), 2.10 (s, 3H), 1.7-1.9 (m, 6H), 1.2-1.3 (m, 1H), 1.1-1.2 (m, 2H), 1.0-1.1 (m, 2H)

### Example 75: Synthesis of (S)-N-(3-(5-(3-isopropoxyazetidin-1-yl)-6-oxo-1-(1,1,1-trifluoropropan-2-yl)-1,6-dihydropyridazin-3-yl)-1-methyl-1H-pyrrolo[2,3-c]pyridin-5-yl)acetamide

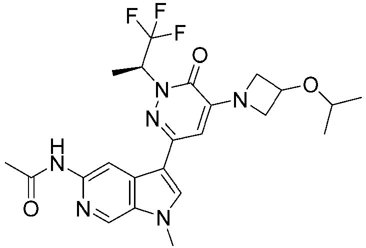

The compound was obtained from the method of Preparation Example 7.

¹H NMR (CHLOROFORM-d, 400 MHz) δ 8.91 (s, 1H), 8.41 (s, 1H), 7.93 (br s, 1H), 7.51 (s, 1H), 6.09 (s, 1H), 5.86 (s, 1H), 4.51 (br d, 3H, J=6.3 Hz), 4.1-4.3 (m, 2H), 3.91 (s, 3H), 3.6-3.8 (m, 1H), 2.25 (s, 3H), 1.8-1.9 (m, 3H), 1.20 (d, 6H, J=6.1 Hz)

### Example 76: Synthesis of (S)-N-(1-ethyl-3-(5-(3-isopropoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide

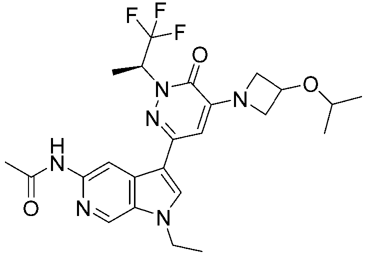

The compound was obtained from the method of Preparation Example 7.

¹H NMR (CHLOROFORM-d, 400 MHz) δ 8.91 (s, 1H), 8.45 (s, 1H), 7.92 (br s, 1H), 7.58 (s, 1H), 6.10 (s, 1H), 5.85 (br d, 1H, J=7.3 Hz), 4.4-4.6 (m, 3H), 4.28 (q, 4H, J=7.4 Hz), 3.6-3.7 (m, 1H), 2.25 (s, 3H), 1.8-1.9 (m, 3H), 1.5-1.6 (m, 3H), 1.2-1.2 (m, 6H)

### Example 77: Synthesis of (S)-N-(1-cyclopropyl-3-(5-(3-isopropoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide

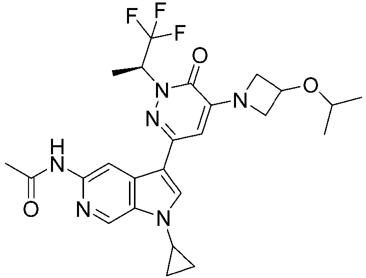

The compound was obtained from the method of Preparation Example 3.

¹H NMR (CHLOROFORM-d, 400 MHz) δ 8.8-8.9 (m, 1H), 8.6-8.7 (m, 1H), 7.9-8.0 (m, 1H), 7.57 (s, 1H), 6.0-6.1 (m, 1H), 5.8-5.9 (m, 1H), 4.4-4.7 (m, 3H), 4.23 (s, 2H), 3.6-3.7 (m, 1H), 3.4-3.5 (m, 1H), 2.2-2.3 (m, 3H), 1.8-1.9 (m, 3H), 1.0-1.3 (m, 10H)

### Example 78: Synthesis of (S)-N-(3-(5-(2,2-difluoropropoxy)-6-oxo-1-(1,1,1-trifluoropropan-2-yl)-1,6-dihydropyridazin-3-yl)-1-ethyl-1H-pyrrolo[2,3-c]pyridin-5-yl)acetamide

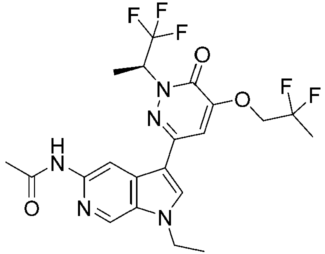

The compound was obtained from the method of Preparation Example 7.

¹H NMR (DMSO-d6, 400 MHz) δ 10.23 (s, 1H), 8.95 (s, 1H), 8.71 (d, 1H, J=1.0 Hz), 8.45 (s, 1H), 7.43 (s, 1H), 5.86 (quin, 1H, J=7.2 Hz), 4.49 (t, 2H, J=12.3 Hz), 4.35 (q, 2H, J=7.3 Hz), 2.10 (s, 3H), 1.7-1.9 (m, 6H), 1.48 (t, 3H, J=7.3 Hz)

### Example 79: Synthesis of (S)-N-(3-(5-(2,2-difluoropropoxy)-6-oxo-1-(1,1,1-trifluoropropan-2-yl)-1,6-dihydropyridazin-3-yl)-1-(2-oxaspiro [3.3] heptan-6-yl)-1H-pyrrolo[2,3-c]pyridin-5-yl)acetamide

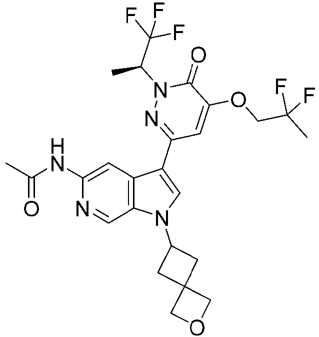

The compound was obtained from the method of Preparation Example 7.

¹H NMR (DMSO-d6, 400 MHz) δ 10.25 (s, 1H), 8.94 (s, 1H), 8.65 (d, 1H, J=1.0 Hz), 8.51 (s, 1H), 7.46 (s, 1H), 5.8-5.9 (m, 1H), 5.0-5.1 (m, 1H), 4.79 (s, 2H), 4.62 (s, 2H), 4.50 (t, 2H, J=12.0 Hz), 2.9-3.0 (m, 2H), 2.7-2.7 (m, 1H), 2.6-2.7 (m, 1H), 2.09 (s, 3H), 1.7-1.9 (m, 6H)

### Example 80: Synthesis of (S)-N-(1-(cyclopropyl methyl)-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide

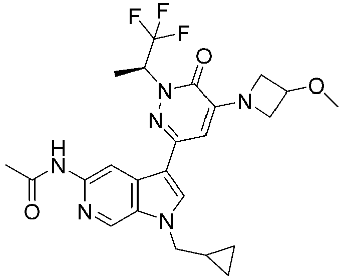

The compound was obtained from the method of Preparation Example 7.

¹H NMR (CHLOROFORM-d, 400 MHz) δ 8.92 (s, 1H), 8.46 (s, 1H), 7.93 (br s, 1H), 7.65 (s, 1H), 6.12 (s, 1H), 5.86 (s, 1H), 4.5-4.6 (m, 2H), 4.3-4.4 (m, 1H), 4.1-4.3 (m, 2H), 4.0-4.1 (m, 1H), 4.08 (d, 3H, J=6.9 Hz), 3.37 (s, 3H), 2.25 (s, 3H), 1.8-1.9 (m, 3H), 1.3-1.4 (m, 1H), 0.7-0.8 (m, 2H), 0.45 (d, 2H, J=5.8 Hz)

### Example 81: Synthesis of (S)-N-(1-((3,3-difluorocyclobutyl)methyl)-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide

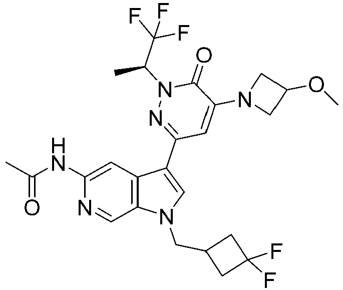

The compound was obtained from the method of Preparation Example 7.

¹H NMR (CHLOROFORM-d, 400 MHz) δ 8.93 (s, 1H), 8.44 (s, 1H), 7.8-8.0 (m, 1H), 7.4-7.6 (m, 1H), 6.0-6.2 (m, 1H), 5.7-6.0 (m, 1H), 4.4-4.7 (m, 2H), 4.1-4.4 (m, 5H), 3.3-3.4 (m, 3H), 2.6-2.9 (m, 3H), 2.3-2.6 (m, 2H), 2.1-2.3 (m, 3H), 1.7-1.9 (m, 3H)

### Example 82: Synthesis of (S)-N-(1-(2-fluoro-2-methylpropyl)-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide

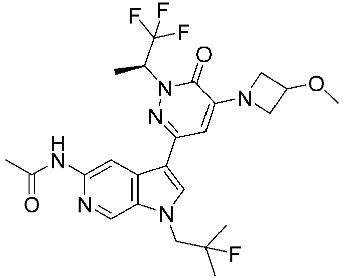

The compound was obtained from the method of Preparation Example 7.

¹H NMR (CHLOROFORM-d, 400 MHz) δ 8.95 (s, 1H), 8.42 (s, 1H), 7.9-8.0 (m, 1H), 7.58 (d, 1H, J=1.3 Hz), 6.10 (s, 1H), 5.8-6.0 (m, 1H), 4.4-4.6 (m, 2H), 4.1-4.4 (m, 5H), 3.36 (s, 3H), 2.25 (s, 3H), 1.86 (d, 3H, J=7.0 Hz), 1.3-1.5 (m, 6H)

### Example 83: Synthesis of (S)-N-(3-(5-(3-methoxyazetidin-1-yl)-6-oxo-1-(1,1,1-trifluoropropan-2-yl)-1,6-dihydropyridazin-3-yl)-1-(2-methylallyl)-1H-pyrrolo[2,3-c]pyridin-5-yl)acetamide

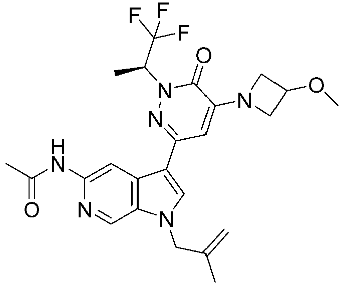

The compound was obtained from the method of Preparation Example 7.

¹H NMR (CHLOROFORM-d, 400 MHz) δ 8.9-9.0 (m, 1H), 8.4-8.5 (m, 1H), 7.8-7.9 (m, 1H), 7.54 (s, 1H), 6.1-6.1 (m, 1H), 5.8-6.0 (m, 1H), 5.0-5.1 (m, 1H), 4.8-4.9 (m, 1H), 4.74 (s, 2H), 4.5-4.6 (m, 2H), 4.3-4.4 (m, 1H), 4.1-4.3 (m, 2H), 3.3-3.4 (m, 3H), 2.2-2.3 (m, 3H), 2.1-2.3 (m, 3H), 1.85 (br d, 3H, J=7.0 Hz), 1.7-1.8 (m, 3H)

### Example 84: Synthesis of (S)-N-(3-(5-(3-methoxyazetidin-1-yl)-6-oxo-1-(1,1,1-trifluoropropan-2-yl)-1,6-dihydropyridazin-3-yl)-1-(3,3,3-trifluoropropyl)-1H-pyrrolo[2,3-c]pyridin-5-yl)acetamide

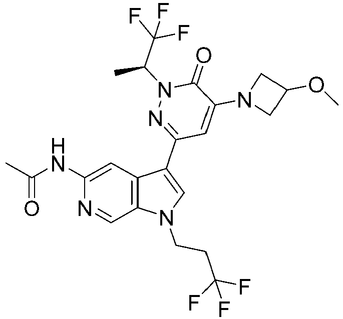

The compound was obtained from the method of Preparation Example 7.

¹H NMR (CHLOROFORM-d, 400 MHz) δ 8.94 (s, 1H), 8.42 (s, 1H), 7.9-8.0 (m, 1H), 7.53 (s, 1H), 6.07 (s, 1H), 5.8-5.9 (m, 1H), 4.4-4.6 (m, 4H), 4.3-4.4 (m, 1H), 4.1-4.3 (m, 2H), 3.37 (s, 3H), 2.6-2.8 (m, 2H), 2.2-2.3 (m, 1H), 2.26 (s, 3H), 1.8-1.9 (m, 4H)

### Example 85: Synthesis of (S)-N-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)acetamide

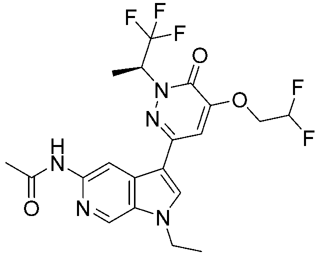

The compound was obtained from the method of Preparation Example 7.

¹H NMR (DMSO-d6, 400 MHz) δ 10.23 (s, 1H), 8.95 (s, 1H), 8.71 (d, 1H, J=1.0 Hz), 8.45 (s, 1H), 7.44 (s, 1H), 6.4-6.7 (m, 1H), 5.86 (quin, 1H, J=7.3 Hz), 4.5-4.6 (m, 2H), 4.35 (q, 2H, J=7.2 Hz), 2.10 (s, 3H), 1.82 (d, 3H, J=7.0 Hz), 1.48 (t, 3H, J=7.3 Hz)

### Example 86: Synthesis of (S)-N-(3-(5-(2,2-difluoropropoxy)-6-oxo-1-(1,1,1-trifluoropropan-2-yl)-1,6-dihydropyridazin-3-yl)-1-(2-methoxyethyl)-1H-pyrrolo[2,3-c]pyridin-5-yl)acetamide

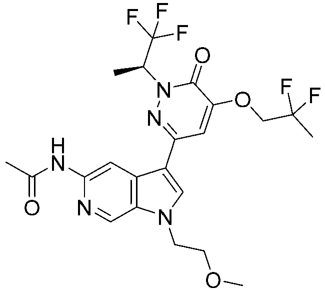

The compound was obtained from the method of Preparation Example 7.

¹H NMR (DMSO-d6, 400 MHz) δ 10.22 (s, 1H), 8.94 (s, 1H), 8.71 (d, 1H, J=0.9 Hz), 8.40 (s, 1H), 7.43 (s, 1H), 5.8-5.9 (m, 1H), 4.4-4.6 (m, 4H), 3.74 (t, 2H, J=5.1 Hz), 3.24 (s, 3H), 2.10 (s, 5H), 1.7-1.9 (m, 6H)

### Example 87: Synthesis of (S)-N-(1-(2-cyanoethyl)-3-(5-(2,2-difluoropropoxy)-6-oxo-1-(1,1,1-trifluoropropan-2-yl)-1,6-dihydropyridazin-3-yl)-1H-pyrrolo[2,3-c]pyridin-5-yl)acetamide

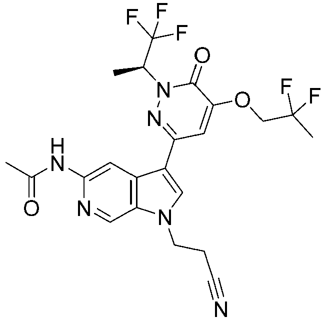

The compound was obtained from the method of Preparation Example 7.

¹H NMR (DMSO-d6, 400 MHz) δ 10.27 (s, 1H), 8.96 (s, 1H), 8.80 (d, 1H, J=1.0 Hz), 8.47 (s, 1H), 7.40 (s, 1H), 5.8-5.9 (m, 1H), 4.63 (dt, 2H, J=1.7, 6.5 Hz), 4.50 (br t, 2H, J=12.1 Hz), 3.19 (t, 2H, J=6.4 Hz), 2.10 (s, 3H), 1.7-1.9 (m, 6H)

### Example 88: Synthesis of (S)-N-(1-(3-cyanopropyl)-3-(5-(2,2-difluoropropoxy)-6-oxo-1-(1,1,1-trifluoropropan-2-yl)-1,6-dihydropyridazin-3-yl)-1H-pyrrolo[2,3-c]pyridin-5-yl)acetamide

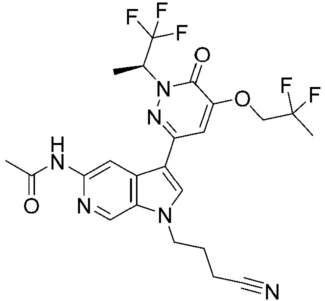

The compound was obtained from the method of Preparation Example 7.

¹H NMR (DMSO-d6, 400 MHz) δ 10.25 (s, 1H), 8.96 (s, 1H), 8.72 (d, 1H, J=1.0 Hz), 8.44 (s, 1H), 7.42 (s, 1H), 5.86 (td, 1H, J=7.5, 14.7 Hz), 4.49 (t, 2H, J=12.2 Hz), 4.38 (br t, 2H, J=7.3 Hz), 2.6-2.6 (m, 2H), 2.19 (quin, 2H, J=7.1 Hz), 2.10 (s, 3H), 1.8-1.9 (m, 6H)

### Example 89: Synthesis of (S)-N-(3-(5-(2,2-difluoropropoxy)-6-oxo-1-(1,1,1-trifluoropropan-2-yl)-1,6-dihydropyridazin-3-yl)-1-(3-oxocyclobutyl)-1H-pyrrolo[2,3-c]pyridin-5-yl)acetamide

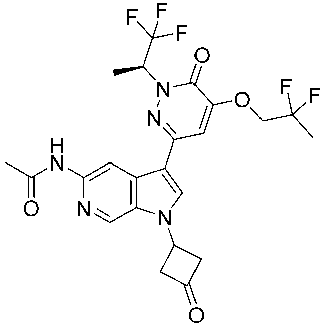

The compound was obtained from the method of Preparation Example 7.

¹H NMR (CHLOROFORM-d, 400 MHz) δ 9.00 (s, 1H), 8.48 (s, 1H), 7.94 (br s, 1H), 7.70 (s, 1H), 6.92 (s, 1H), 5.7-6.0 (m, 1H), 5.1-5.3 (m, 1H), 4.38 (t, 2H, J=11.1 Hz), 3.8-3.9 (m, 1H), 3.5-3.8 (m, 2H), 2.27 (s, 2H), 1.8-1.9 (m, 3H)

### Example 90: Synthesis of (S)-N-(3-(5-(2,2-difluoropropoxy)-6-oxo-1-(1,1,1-trifluoropropan-2-yl)-1,6-dihydropyridazin-3-yl)-1-((3-methyloxetan-3-yl)methyl)-1H-pyrrolo[2,3-c]pyridin-5-yl)acetamide

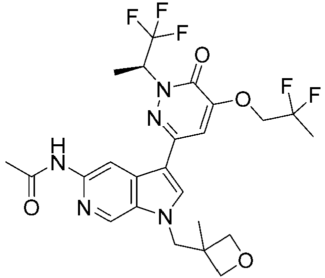

The compound was obtained from the method of Preparation Example 7.

¹H NMR (CHLOROFORM-d, 400 MHz) δ 8.97 (s, 1H), 8.50 (s, 1H), 8.04 (s, 1H), 7.54 (s, 1H), 6.89 (s, 1H), 5.8-6.0 (m, 1H), 4.67 (dd, 2H, J=2.6, 6.1 Hz), 4.4-4.5 (m, 4H), 4.37 (t, 2H, J=11.1 Hz), 2.25 (s, 3H), 2.03 (s, 1H), 1.8-1.9 (m, 6H), 1.37 (s, 3H)

### Example 91: Synthesis of (S)-N-(3-(5-(2,2-difluoropropoxy)-6-oxo-1-(1,1,1-trifluoropropan-2-yl)-1,6-dihydropyridazin-3-yl)-1-(2-morpholinoethyl)-1H-pyrrolo[2,3-c]pyridin-5-yl)acetamide

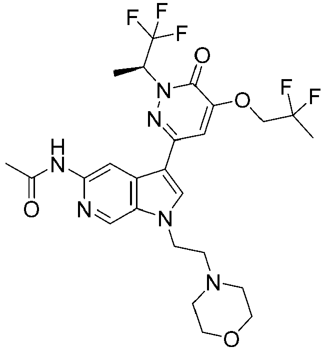

The compound was obtained from the method of Preparation Example 7.

¹H NMR (CHLOROFORM-d, 400 MHz) δ 8.95 (s, 1H), 8.50 (s, 1H), 7.95 (s, 1H), 7.69 (s, 1H), 6.92 (s, 1H), 5.8-6.0 (m, 1H), 4.3-4.4 (m, 4H), 3.7-3.8 (m, 4H), 2.83 (t, 2H, J=6.3 Hz), 2.5-2.6 (m, 4H), 2.26 (s, 3H), 1.8-1.9 (m, 6H)

### Example 92: Synthesis of (S)-N-(1-(cyclopropylmethyl)-3-(5-(2,2-difluoropropoxy)-6-oxo-1-(1,1,1-trifluoropropan-2-yl)-1,6-dihydropyridazin-3-yl)-1H-pyrrolo[2,3-c]pyridin-5-yl)acetamide

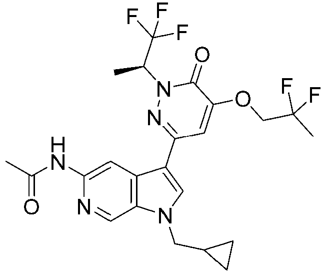

The compound was obtained from the method of Preparation Example 7.

¹H NMR (CHLOROFORM-d, 400 MHz) δ 8.95 (s, 1H), 8.50 (s, 1H), 7.95 (s, 1H), 7.69 (s, 1H), 6.92 (s, 1H), 5.8-6.0 (m, 1H), 4.3-4.4 (m, 4H), 3.7-3.8 (m, 4H), 2.83 (t, 2H, J=6.3 Hz), 2.5-2.6 (m, 4H), 2.26 (s, 3H), 1.8-1.9 (m, 6H)

### Example 93: Synthesis of (S)-N-(3-(5-(2,2-difluoropropoxy)-6-oxo-1-(1,1,1-trifluoropropan-2-yl)-1,6-dihydropyridazin-3-yl)-1-(oxetan-3-ylmethyl)-1H-pyrrolo[2,3-c]pyridin-5-yl)acetamide

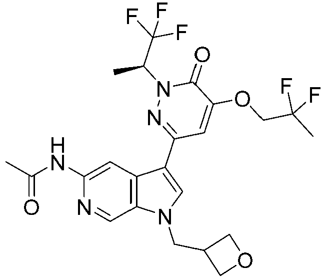

The compound was obtained from the method of Preparation Example 7.

¹H NMR (DMSO-d6, 400 MHz) δ 10.25 (s, 1H), 8.94 (s, 1H), 8.76 (d, 1H, J=1.0 Hz), 8.48 (s, 1H), 7.41 (s, 1H), 5.86 (quin, 1H, J=7.3 Hz), 4.6-4.7 (m, 4H), 4.4-4.5 (m, 4H), 3.5-3.6 (m, 1H), 2.09 (s, 3H), 1.7-1.9 (m, 6H)

### Example 94: Synthesis of N-(3-(5-(2,2-difluoropropoxy)-6-oxo-1-((S)-1,1,1-trifluoropropan-2-yl)-1,6-dihydropyridazin-3-yl)-1-((R)-tetrahydrofuran-3-yl)-1H-pyrrolo[2,3-c]pyridin-5-yl)acetamide

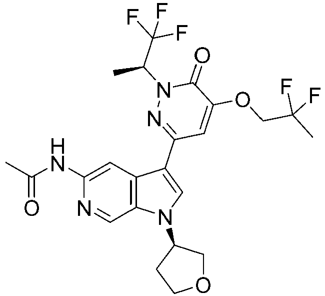

The compound was obtained from the method of Preparation Example 7.

¹H NMR (DMSO-d6, 400 MHz) δ 10.25 (s, 1H), 8.96 (s, 1H), 8.77 (d, 1H, J=0.9 Hz), 8.42 (s, 1H), 7.48 (s, 1H), 5.86 (quin, 1H, J=7.3 Hz), 5.4-5.5 (m, 1H), 4.51 (t, 2H, J=12.2 Hz), 4.15 (dt, 1H, J=6.2, 8.2 Hz), 4.0-4.1 (m, 2H), 3.88 (dt, 1H, J=6.5, 8.4 Hz), 2.6-2.6 (m, 1H), 2.2-2.3 (m, 1H), 2.10 (s, 3H), 1.7-1.9 (m, 6H)

### Example 95: Synthesis of (S)-N-(1-(6,6-difluorospiro[3.3]heptan-2-yl)-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide

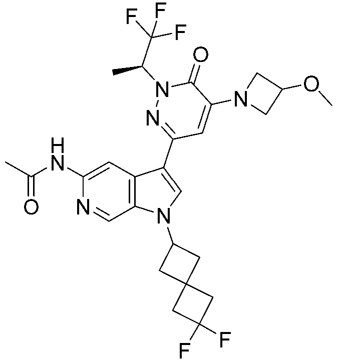

The compound was obtained from the method of Preparation Example 7.

¹H NMR (CHLOROFORM-d, 400 MHz) δ 8.90 (s, 1H), 8.39 (s, 1H), 7.93 (s, 1H), 7.57 (s, 1H), 6.09 (s, 1H), 5.86 (s, 1H), 5.69 (s, 1H), 4.87 (s, 1H), 4.5-4.6 (m, 2H), 4.36 (s, 4H), 3.3-3.4 (m, 4H), 2.8-2.9 (m, 4H), 2.6-2.8 (m, 4H), 2.24 (s, 3H), 1.83 (d, 3H, J=7.1 Hz)

### Example 96: Synthesis of (S)-N-(3-(5-(2,2-difluoropropoxy)-6-oxo-1-(1,1,1-trifluoropropan-2-yl)-1,6-dihydropyridazin-3-yl)-1-propyl-1H-pyrrolo[2,3-c]pyridin-5-yl)acetamide

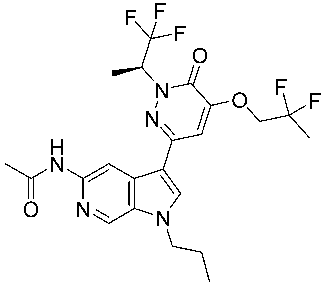

The compound was obtained from the method of Preparation Example 7.

¹H NMR (DMSO-d6, 400 MHz) δ 10.23 (s, 1H), 8.95 (s, 1H), 8.71 (d, 1H, J=1.0 Hz), 8.44 (s, 1H), 7.43 (s, 1H), 5.86 (quin, 1H, J=7.3 Hz), 4.49 (t, 2H, J=12.3Hz), 4.27 (t, 2H, J=7.1 Hz), 2.10 (s, 3H), 1.7-1.9 (m, 8H), 0.89 (t, 3H, J=7.4 Hz)

### Example 97: Synthesis of (S)-N-(3-(5-(2,2-difluoropropoxy)-6-oxo-1-(1,1,1-trifluoropropan-2-yl)-1,6-dihydropyridazin-3-yl)-1-(7-oxaspiro[3.5]nonan-2-yl)-1H-pyrrolo[2,3-c]pyridin-5-yl)acetamide

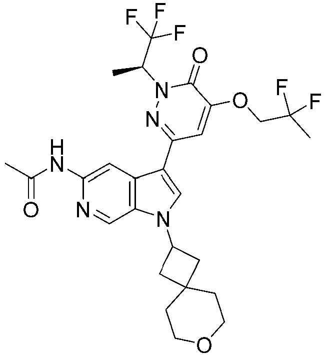

The compound was obtained from the method of Preparation Example 7.

¹H NMR (DMSO-d6, 400 MHz) δ 10.25 (s, 1H), 8.95 (s, 1H), 8.67 (d, 1H, J=1.0 Hz), 8.52 (s, 1H), 7.49 (s, 1H), 5.86 (td, 1H, J=7.2, 14.6 Hz), 5.19 (quin, 1H, J=8.4 Hz), 4.51 (t, 2H, J=12.1 Hz), 3.6-3.7 (m, 2H), 3.5-3.6 (m, 2H), 2.6-2.7 (m, 2H), 2.2-2.3 (m, 2H), 2.09 (s, 3H), 1.7-1.9 (m, 8H), 1.6-1.7 (m, 2H)

### Example 98: Synthesis of (S)-N-(1-(cyclopropanecarbonyl)-3-(5-(2,2-difluoropropoxy)-6-oxo-1-(1,1,1-trifluoropropan-2-yl)-1,6-dihydropyridazin-3-yl)-1H-pyrrolo[2,3-c]pyridin-5-yl)acetamide

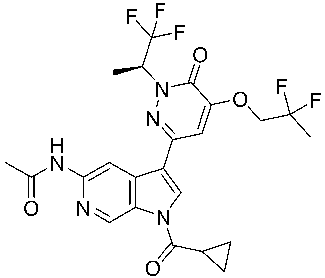

The compound was obtained from the method of Preparation Example 7.

¹H NMR (DMSO-d6, 400 MHz) δ 10.51 (s, 1H), 9.30 (d, 1H, J=1.0 Hz), 9.16 (s, 1H), 9.08 (s, 1H), 7.62 (s, 1H), 5.9-6.0 (m, 1H), 4.52 (t, 2H, J=12.1 Hz), 2.81 (quin, 1H, J=6.1 Hz), 2.12 (s, 3H), 1.8-1.9 (m, 6H), 1.25 (d, 4H, J=5.9 Hz)

### Example 99: Synthesis of (S)-N-(1-(2,2-difluoroethyl)-3-(5-(2,2-difluoropropoxy)-6-oxo-1-(1,1,1-trifluoropropan-2-yl)-1,6-dihydropyridazin-3-yl)-1H-pyrrolo[2,3-c]pyridin-5-yl)acetamide

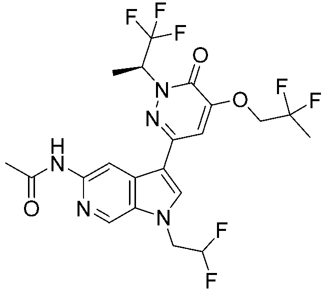

The compound was obtained from the method of Preparation Example 7.

¹H NMR (CHLOROFORM-d, 400 MHz) δ 9.00 (s, 1H), 8.49 (s, 1H), 8.00 (s, 1H), 7.60 (s, 1H), 6.89 (s, 1H), 6.12 (s, 1H), 5.9-6.0 (m, 1H), 4.57 (dt, 2H, J=3.4, 14.4 Hz), 4.36 (t, 2H, J=11.1 Hz), 2.26 (s, 3H), 1.8-1.9 (m, 6H)

### Example 100: Synthesis of N-(3-(5-(2,2-difluoropropoxy)-6-oxo-1-((S)-1,1,1-trifluoropropan-2-yl)-1,6-dihydropyridazin-3-yl)-1-(3-fluorocyclobut-2-en-1-yl)-1H-pyrrolo[2,3-c]pyridin-5-yl)acetamide

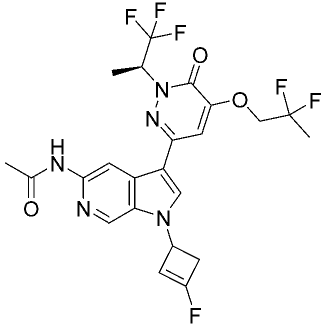

The compound was obtained from the method of Preparation Example 7.

¹H NMR (CHLOROFORM-d, 400 MHz) δ 9.00 (s, 1H), 8.63 (s, 1H), 7.98 (s, 1H), 7.80 (s, 1H), 7.4-7.5 (m, 1H), 6.92 (s, 1H), 6.2-6.4 (m, 1H), 5.8-6.1 (m, 1H), 4.87 (dd, 1H, J=3.3, 16.8 Hz), 4.71 (d, 1H, J=3.3 Hz), 4.59 (d, 1H, J=3.4 Hz), 4.38 (t, 2H, J=11.0 Hz), 2.27 (s, 3H), 1.8-2.0 (m, 6H)

### Example 101: Synthesis of (S)-N-(3-(5-(2,2-difluoropropoxy)-6-oxo-1-(1,1,1-trifluoropropan-2-yl)-1,6-dihydropyridazin-3-yl)-1-isopropyl-1H-pyrrolo[2,3-c]pyridin-5-yl)acetamide

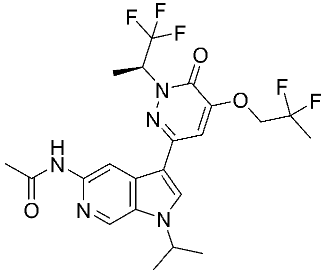

The compound was obtained from the method of Preparation Example 7.

¹H NMR (CHLOROFORM-d, 400 MHz) δ 8.97 (s, 1H), 8.52 (s, 1H), 7.92 (br s, 1H), 7.67 (s, 1H), 6.91 (s, 1H), 5.9-6.1 (m, 1H), 4.7-4.9 (m, 1H), 4.37 (t, 2H, J=11.1 Hz), 2.26 (s, 3H), 1.8-1.9 (m, 6H), 1.6-1.7 (m, 6H)

### Example 102: Synthesis of (S)-N-(1-ethyl-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide

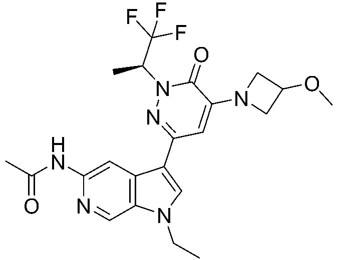

The compound was obtained from the method of Preparation Example 7.

¹H NMR (CHLOROFORM-d, 400 MHz) δ 8.92 (s, 1H), 8.45 (s, 1H), 7.9-8.0 (s, 1H), 7.59 (s, 1H), 6.12 (s, 1H), 5.86 (s, 1H), 4.4-4.6 (m, 2H), 4.1-4.4 (m, 4H), 3.37 (s, 2H), 2.25 (s, 2H), 1.84 (d, 3H, J=7.1 Hz)

### Example 103: Synthesis of (S)-N-(3-(5-(2-fluoro-2-methylpropoxy)-6-oxo-1-(1,1,1-trifluoropropan-2-yl)-1,6-dihydropyridazin-3-yl)-1-methyl-1H-pyrrolo[2,3-c]pyridin-5-yl)acetamide

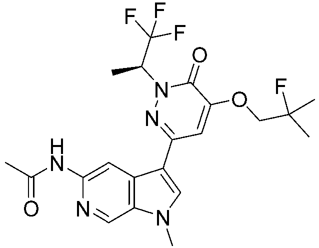

The compound was obtained from the method of Preparation Example 7.

¹H NMR (CHLOROFORM-d, 400 MHz) δ 8.96 (br s, 1H), 8.45 (br s, 1H), 7.94 (br s, 1H), 7.55 (s, 1H), 6.89 (s, 1H), 5.8-6.0 (m, 1H), 4.16 (br d, 2H, J=16.6 Hz), 3.94 (s, 3H), 2.25 (s, 3H), 1.8-2.0 (m, 3H), 1.5-1.6 (m, 6H)

### Example 104: Synthesis of (S)-N-(1-methyl-3-(6-oxo-5-(2,2,2-trifluoroethoxy)-1-(1,1,1-trifluoropropan-2-yl)-1,6-dihydropyridazin-3-yl)-1H-pyrrolo[2,3-c]pyridin-5-yl)acetamide

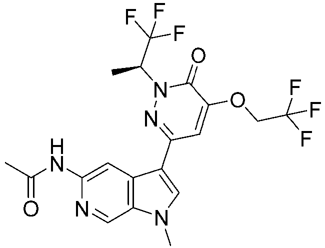

The compound was obtained from the method of Preparation Example 7.

¹H NMR (CHLOROFORM-d, 400 MHz) δ 8.95 (br s, 1H), 8.46 (br s, 1H), 8.00 (br s, 1H), 7.55 (s, 1H), 7.04 (s, 1H), 5.9-6.0 (m, 1H), 4.7-4.8 (m, 2H), 3.95 (s, 3H), 2.26 (s, 3H), 1.91 (br d, 3H, J=6.8 Hz)

### Example 105: Synthesis of (S)-N-(1-ethyl-3-(5-(2-fluoro-2-methylpropoxy)-6-oxo-1-(1,1,1-trifluoropropan-2-yl)-1,6-dihydropyridazin-3-yl)-1H-pyrrolo[2,3-c]pyridin-5-yl)acetamide

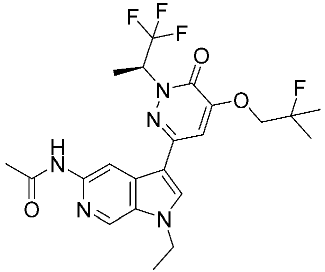

The compound was obtained from the method of Preparation Example 7.

¹H NMR (CHLOROFORM-d, 400 MHz) δ 8.97 (br s, 1H), 8.47 (br s, 1H), 8.00 (br s, 1H), 7.61 (s, 1H), 6.89 (s, 1H), 5.9-6.0 (m, 1H), 4.30 (br d, 2H, J=6.9 Hz), 4.16 (br d, 2H, J=16.6 Hz), 2.25 (br s, 3H), 1.89 (br d, 3H, J=6.3 Hz), 1.5-1.6 (m, 6H)

### Example 106: Synthesis of (S)-N-(1-ethyl-3-(6-oxo-5-(2,2,2-trifluoroethoxy)-1-(1,1,1-trifluoropropan-2-yl)-1,6-dihydropyridazin-3-yl)-1H-pyrrolo[2,3-c]pyridin-5-yl)acetamide

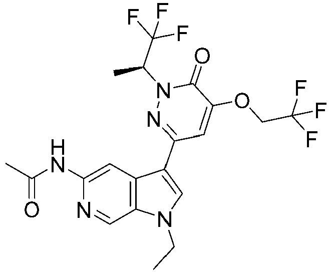

The compound was obtained from the method of Preparation Example 7.

¹H NMR (CHLOROFORM-d, 400 MHz) δ 8.96 (br s, 1H), 8.49 (br s, 1H), 7.97 (br s, 1H), 7.6-7.6 (s, 1H), 7.4 (s, 1H), 7.05 (s, 1H), 5.8-6.0 (m, 1H), 4.76 (q, 2H, J=7.7 Hz), 4.2-4.4 (m, 2H), 2.26 (s, 3H), 1.7-2.0 (m, 3H), 1.5-1.6 (m, 3H)

### Example 107: Synthesis of (S)-N-(1-cyclopropyl-3-(6-oxo-5-(2,2,2-trifluoroethoxy)-1-(1,1,1-trifluoropropan-2-yl)-1,6-dihydropyridazin-3-yl)-1H-pyrrolo[2,3-c]pyridin-5-yl)acetamide

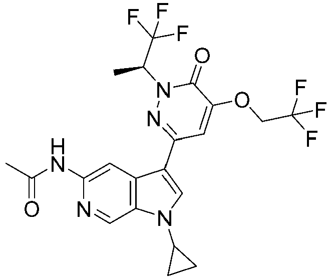

The compound was obtained from the method of Preparation Example 3.

¹H NMR (CHLOROFORM-d, 400 MHz) δ 8.94 (br s, 1H), 8.66 (br s, 1H), 7.95 (br s, 1H), 7.61 (s, 1H), 7.03 (s, 1H), 5.8-6.0 (m, 1H), 4.76 (br d, 2H, J=7.9 Hz), 3.51 (br s, 1H), 2.26 (s, 3H), 2.03 (s, 1H), 1.91 (br d, 3H, J=6.4 Hz), 1.2-1.3 (m, 2H), 1.13 (br s, 2H)

### Example 108: Synthesis of (S)-N-(1-ethyl-3-(5-(3-fluoropropoxy)-6-oxo-1-(1,1,1-trifluoropropan-2-yl)-1,6-dihydropyridazin-3-yl)-1H-pyrrolo[2,3-c]pyridin-5-yl)acetamide

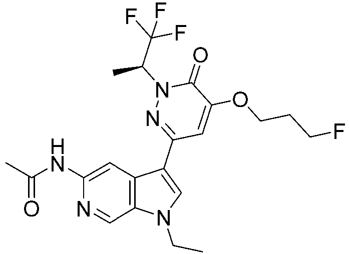

The compound was obtained from the method of Preparation Example 7.

¹H NMR (DMSO-d6, 400 MHz) δ 10.21 (s, 1H), 8.95 (s, 1H), 8.69 (s, 1H), 8.48 (s, 1H), 7.34 (s, 1H), 5.84 (br d, 1H, J=7.4 Hz), 4.71 (t, 1H, J=5.8 Hz), 4.59 (t, 1H, J=5.8 Hz), 4.2-4.4 (m, 4H), 2.1-2.3 (m, 2H), 2.09 (s, 3H), 1.81 (d, 3H, J=7.0 Hz), 1.48 (t, 3H, J=7.2 Hz)

### Example 109: Synthesis of (S)-N-(3-(5-(3-fluoropropoxy)-6-oxo-1-(1,1,1-trifluoropropan-2-yl)-1,6-dihydropyridazin-3-yl)-1-methyl-1H-pyrrolo[2,3-c]pyridin-5-yl)acetamide

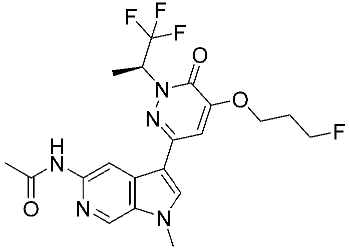

The compound was obtained from the method of Preparation Example 7.

¹H NMR (DMSO-d6, 400 MHz) δ 10.23 (s, 1H), 8.95 (s, 1H), 8.65 (d, 1H, J=1.0 Hz), 8.41 (s, 1H), 7.32 (s, 1H), 5.85 (td, 1H, J=7.3, 14.6 Hz), 4.71 (t, 1H,

J=5.8 Hz), 4.59 (t, 1H, J=5.8 Hz), 4.2-4.3 (m, 2H), 3.94 (s, 3H), 2.26 (t, 1H, J=6.0 Hz), 2.2-2.2 (m, 1H), 2.10 (s, 3H), 1.81 (d, 3H, J=7.1 Hz)

### Example 110: Synthesis of (S)-N-(1-cyclopropyl-3-(5-(2-fluoro-2-methylpropoxy)-6-oxo-1-(1,1,1-trifluoropropan-2-yl)-1,6-dihydropyridazin-3-yl)-1H-pyrrolo[2,3-c]pyridin-5-yl)acetamide

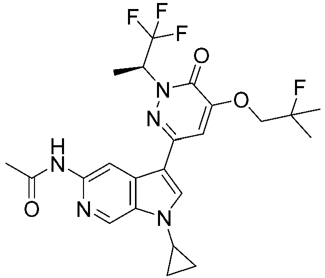

The compound was obtained from the method of Preparation Example 3.

¹H NMR (CHLOROFORM-d, 400 MHz) δ 8.95 (s, 1H), 8.64 (s, 1H), 8.07 (br s, 1H), 7.61 (s, 1H), 6.86 (s, 1H), 5.9-6.0 (m, 1H), 4.16 (d, 2H, J=16.6 Hz), 3.50 (br d, 1H, J=3.5 Hz), 2.25 (s, 3H), 1.89 (br d, 3H, J=6.9 Hz), 1.74 (br s, 1H), 1.6 (s, 3H), 1.5 (s, 3H), 1.2-1.3 (m, 4H), 1.13 (br s, 2H)

### Example 111: Synthesis of N-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)acetamide

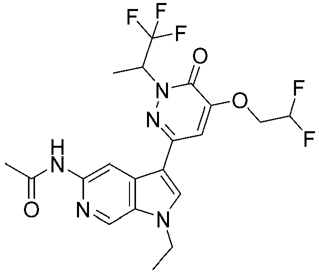

The compound was obtained from the method of Preparation Example 7.

¹H NMR (CHLOROFORM-d, 400 MHz) δ 8.95 (br s, 1H), 8.49 (br s, 1H), 8.00 (br s, 1H), 7.62 (s, 1H), 6.91 (s, 1H), 6.1-6.4 (m, 1H), 5.8-6.0 (m, 1H), 4.43 (dt, 2H, J=3.4, 12.5 Hz), 4.31 (q, 2H, J=7.0 Hz), 2.26 (s, 3H), 1.7-1.9 (m, 4H), 1.4-1.6 (m, 3H)

### Example 112: Synthesis of N-(1-cyclopropyl-3-(5-(2,2-difluoroethoxy)-6-oxo-1-(1,1,1-trifluoropropan-2-yl)-1,6-dihydropyridazin-3-yl)-1H-pyrrolo[2,3-c]pyridin-5-yl)acetamide

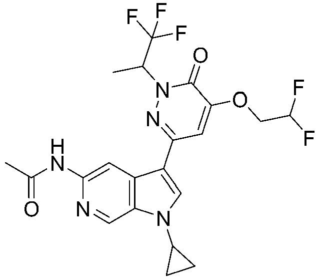

The compound was obtained from the method of Preparation Example 3.

¹H NMR (CHLOROFORM-d, 400 MHz) δ 8.94 (br s, 1H), 8.66 (br s, 1H), 7.95 (br s, 1H), 7.62 (s, 1H), 6.89 (s, 1H), 6.1-6.4 (m, 1H), 5.8-6.0 (m, 1H), 4.43 (dt, 2H, J=3.8, 12.7 Hz), 4.26 (br d, 1H, J=8.8 Hz), 3.51 (br s, 1H), 2.26 (s, 2H), 1.90 (br d, 3H, J=6.6 Hz), 1.23 (br d, 2H, J=5.0 Hz), 1.14 (br s, 2H)

### Example 113: Synthesis of (R)-N-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)acetamide

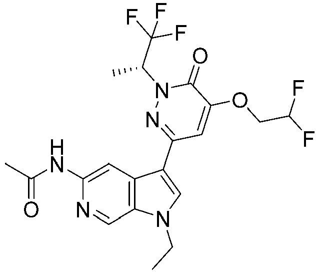

The compound was obtained from the method of Preparation Example 7.

¹H NMR (DMSO-d6, 400 MHz) δ 10.23 (s, 1H), 8.95 (s, 1H), 8.71 (d, 1H, J=0.9 Hz), 8.45 (s, 1H), 7.44 (s, 1H), 6.4-6.7 (m, 1H), 5.86 (quin, 1H, J=7.3 Hz), 4.5-4.6 (m, 2H), 4.35 (q, 2H, J=7.3 Hz), 2.10 (s, 3H), 1.82 (d, 3H, J=7.0 Hz), 1.48 (t, 3H, J=7.3 Hz)

### Example 114: Synthesis of (R)-N-(1-cyclopropyl-3-(5-(2,2-difluoroethoxy)-6-oxo-1-(1,1,1-trifluoropropan-2-yl)-1,6-dihydropyridazin-3-yl)-1H-pyrrolo[2,3-c]pyridin-5-yl)acetamide

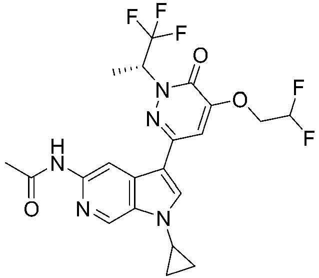

The compound was obtained from the method of Preparation Example 3.

¹H NMR (DMSO-d6, 400 MHz) δ 10.27 (s, 1H), 8.95 (s, 1H), 8.70 (d, 1H, J=1.0 Hz), 8.42 (s, 1H), 7.48 (s, 1H), 6.6-6.6 (m, 1H), 6.4-6.7 (m, 1H), 5.8-5.9 (m, 1H), 4.51 (tt, 2H, J=3.5, 14.1 Hz), 3.68 (tt, 1H, J=3.6, 7.0 Hz), 2.10 (s, 3H), 1.81 (d, 3H, J=7.0 Hz), 1.1-1.2 (m, 2H), 1.1-1.1 (m, 2H)

### Example 115: Synthesis of (S)-N-(3-(5-(2,2-difluoroethoxy)-6-oxo-1-(1,1,1-trifluoropropan-2-yl)-1,6-dihydropyridazin-3-yl)-1H-pyrrolo [2,3-c] pyridin-5-yl)acetamide

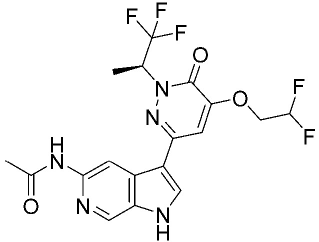

The compound was obtained from the method of Preparation Example 8.

¹H NMR (CHLOROFORM-d, 400 MHz) δ 9.0 (s, 1H), 8.5-8.7 (m, 2H), 7.9 (s, 1H), 7.7 (s, 1H), 6.94 (br s, 1H), 6.2-6.3 (m, 1H), 5.9-6.0 (m, 1H), 4.2-4.5 (m, 4H), 2.26 (br s, 3H), 1.8-2.0 (m, 3H)

### Example 116: Synthesis of (S)-N-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)-2-hydroxyacetamide

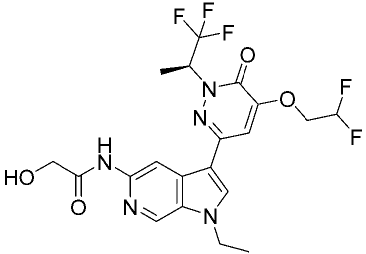

The compound was obtained from the method of Preparation Example 9.

¹H NMR (CHLOROFORM-d, 400 MHz) δ 9.31 (br s, 1H), 9.02 (br s, 1H), 8.46 (s, 1H), 7.61 (s, 1H), 6.88 (s, 1H), 6.1-6.4 (m, 1H), 5.94 (br s, 1H), 5.32 (s, 6H), 4.2-4.5 (m, 6H), 1.89 (br d, 3H, J=6.5 Hz), 1.5-1.6 (m, 3H)

### Example 117: Synthesis of (S)-1-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)-3-methylurea

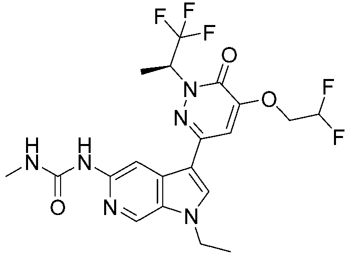

The compound was obtained from the method of Preparation Example 10.

¹H NMR (METHANOL-d4, 400 MHz) δ 8.54 (d, 1H, J=0.9 Hz), 8.2 (s, 1H), 8.1 (s, 1H), 7.84 (d, 1H, J=0.9 Hz), 7.37 (s, 1H), 6.2-6.5 (m, 1H), 5.9-6.0 (m, 1H), 4.50 (dt, 2H, J=3.9, 13.3 Hz), 4.3-4.4 (m, 2H), 3.13 (br s, 3H), 1.83 (d, 3H, J=7.0 Hz), 1.5-1.6 (m, 3H)

### TYK2 Pseudokinase Ligand

Like other JAK family proteins, TYK2 consists of four domains (FERM, SH2, JH2, and JH1 domains) (Prior arts document 7). The JH1 domain is a kinase domain having an active function that actually transmits intracellular signals, and the JH2 domain is a pseudokinase domain having a regulatory function that inhibits an activation of the JH1 domain until an external stimulus is received (Prior arts document 7). That is, the TYK2 pseudokinase domain stabilizes a structurally inactive state of the kinase domain. Therefore, a small molecule pseudokinase ligand may inhibit the activity of TYK2 by stabilizing this inactive structure.

### Experimental Example

The following experiment was conducted on the compounds of Examples prepared as described above.

### Experimental Example 1: Analysis of domain binding affinity to TYK2 pseudokinase

In order analyze the binding affinity of a test compound to the TYK2 pseudokinase domain, a polarized fluorescence-based TYK2 JH2 Pseudokinase Domain Inhibitor Screening Assay Kit (BPS bioscience) was used. The evaluation of the compound was performed according to the manufacturer's protocol, and the method thereof is as follows:

The test compound or control compound (Deucravacitnib) was serially diluted in DMSO, and then diluted 1/10 with distilled water to prepare 10x compound solution. After mixing the compound solution with Probe 1 prepared with a binding reaction buffer (JH2 binding buffer), a reaction was performed at 25°C for 10 minutes. Next, the reaction was initiated by adding a TYK2 pseudokinase domain enzyme prepared with the binding reaction buffer. The reaction was performed in a black 384-well plate at 25 °C for 1 hour. Polarized fluorescence was measured using Synergy neo (BioTek Instruments, Inc.). A reaction mixture that did not contain the enzyme was a negative control, and a mixture without an arbitrary compound was a positive control.

### Experimental Example 2: JAK Kinase Domain Activity Analysis

Kinase domain activity of the JAK family was analyzed using the ADP-Glo kinase assay kit (Promega). The evaluation of the compound was performed according to the manufacturer's protocol, and the method thereof is as follows:

A 10 mM test compound was diluted to 0.5 mM using DMSO in a 96-well plate, and then 5-fold serial dilutions were performed in DMSO. The diluted compound was diluted to 1/10 with 1x reaction buffer (composition) to prepare a 5x compound solution. 5 uL of the 5x compound solution was mixed with a substrate prepared in the reaction buffer (40 mM Tris, 20 mM MgCl₂ and 0.1 mg/ml BSA, 0.05 mM DTT, pH 7.5), and an ATP mixture (10 uL). Next, an enzyme reaction was initiated by adding the JAK family enzyme (10 uL) prepared in the reaction buffer. A short rotation was performed to settle all the solution to the bottom of the plate. The final concentrations of the test compounds in the reaction mixture were 10000, 2000, 400, 80, 16, and 3.2 nM. The concentrations of the control compounds (Brepocitinib or Tofacitinib) were 10 times lower. The enzyme reaction was performed at 25°C for 1 hour. 25 uL of ADP-Glo reagent was added and the reaction was performed at 25 °C for 1 hour. The reaction mixed 50 uL of kinase detection reagent and was stopped, and a luminescence signal was measured using Synergy neo (BioTek Instruments, Inc.). A reaction mixture that did not contain the enzyme was a negative control, and a mixture without an arbitrary compound was a positive control. Enzymes, substrates, and control drugs used are summarized in following Table 1. The experimental results are shown in Table 2.

**[Table 1]**

| Enzyme | Substrate | Control drug |
|---|---|---|
| JAK1 | IRS1 (Y608) peptide | Brepocitinib |
| JAK2 | IGF1R peptide | Tofacitinib |
| JAK3 | Poly (4:1 Glu-Tyr) peptide | Tofacitinib |
| TYK2 | IRS1 (Y608) peptide | Brepocitinib |

**[Table 2] Result of confirmation of TYK2 pseudokinase and JAK kinase domain activity**

| Example | TYK2 JH2 IC₅₀ (nM) | JAK1 JH1 IC₅₀ (nM) | JAK2 JH1 IC₅₀ (nM) | JAK3 JH1 IC₅₀ (nM) | TYK2 JH1 IC₅₀ (nM) |
|---|---|---|---|---|---|
| 1 | 32 | >10000 | >10000 | >10000 | >10000 |
| 2 | 34 | >10000 | >10000 | >10000 | >10000 |
| 3 | 182 | ND | ND | ND | ND |
| 4 | 60 | ND | ND | ND | ND |
| 5 | 38 | ND | ND | ND | ND |
| 6 | 20 | ND | ND | ND | ND |
| 7 | 37 | ND | ND | ND | ND |
| 8 | 18 | ND | ND | ND | ND |
| 9 | 4 | >10000 | 1825 | >10000 | >10000 |
| 10 | 20 | >10000 | >10000 | >10000 | >10000 |
| 11 | 21 | 7466 | 2194 | 3989 | >10000 |
| 12 | 61 | ND | ND | ND | ND |
| 13 | 111 | ND | ND | ND | ND |
| 14 | 91 | ND | ND | ND | ND |
| 15 | 60 | ND | ND | ND | ND |
| 16 | 47 | ND | ND | ND | ND |
| 17 | 149 | ND | ND | ND | ND |
| 18 | 121 | ND | ND | ND | ND |
| 19 | 19 | 1649 | 597 | >10000 | 4056 |
| 20 | 92 | ND | ND | ND | ND |
| 21 | 27 | ND | ND | ND | ND |
| 22 | 30 | ND | ND | ND | ND |
| 23 | 14 | ND | ND | ND | ND |
| 24 | 33 | ND | ND | ND | ND |
| 25 | 19 | ND | ND | ND | ND |
| 26 | 21 | ND | ND | ND | ND |
| 27 | 31 | ND | ND | ND | ND |
| 28 | 35 | ND | ND | ND | ND |
| 29 | 30 | ND | ND | ND | ND |
| 30 | 33 | ND | ND | ND | ND |
| 31 | 32 | ND | ND | ND | ND |
| 32 | 15 | 2358 | 1043 | >10000 | 5063 |
| 33 | 34 | >10000 | >10000 | >10000 | 10000 |
| 34 | 20 | ND | ND | ND | ND |
| 35 | 32 | >10000 | 6619 | >10000 | >10000 |
| 36 | 52 | >10000 | >10000 | >10000 | >10000 |
| 37 | 22 | 814 | 553 | 3507 | 1463 |
| 38 | 26 | >10000 | 9147 | >10000 | >10000 |
| 39 | 89 | ND | ND | ND | ND |
| 40 | 83 | ND | ND | ND | ND |
| 41 | 66 | ND | ND | ND | ND |
| 42 | 122 | ND | ND | ND | ND |
| 43 | 130 | ND | ND | ND | ND |
| 44 | 53 | ND | ND | ND | ND |
| 45 | 38 | >10000 | 2385 | >10000 | >10000 |
| 46 | 30 | ND | ND | ND | ND |
| 47 | 14 | ND | ND | ND | ND |
| 48 | 26 | ND | ND | ND | ND |
| 49 | 45 | >10000 | >10000 | >10000 | >10000 |
| 50 | 154 | ND | ND | ND | ND |
| 51 | 75 | 6541 | 2815 | >10000 | >10000 |
| 52 | 78 | ND | ND | ND | ND |
| 53 | 41 | ND | ND | ND | ND |
| 54 | 43 | 4034 | 1891 | >10000 | 4652 |
| 55 | 68 | ND | ND | ND | ND |
| 56 | 42 | ND | ND | ND | ND |
| 57 | 21 | ND | ND | ND | ND |
| 58 | 73 | ND | ND | ND | ND |
| 59 | 39 | ND | ND | ND | ND |
| 60 | 27 | ND | ND | ND | ND |
| 61 | 26 | 9359 | 2017 | >10000 | >10000 |
| 62 | 29 | 4102 | 1713 | >10000 | >10000 |
| 63 | 59 | ND | ND | ND | ND |
| 64 | 35 | >10000 | >10000 | >10000 | >10000 |
| 65 | 18 | ND | ND | ND | ND |
| 66 | 16 | ND | ND | ND | ND |
| 67 | 33 | 1535 | 384 | 7824 | 1577 |
| 68 | 25 | >10000 | >10000 | >10000 | >10000 |
| 69 | 36 | ND | ND | ND | ND |
| 70 | 26 | ND | ND | ND | ND |
| 71 | 14 | 9066 | >10000 | >10000 | >10000 |
| 72 | 29 | 3489 | 1194 | >10000 | >10000 |
| 73 | 32 | 2997 | 1093 | >10000 | 7556 |
| 74 | 11 | 7744 | 7450 | >10000 | >10000 |
| 75 | 46 | ND | ND | ND | ND |
| 76 | 42 | ND | ND | ND | ND |
| 77 | 35 | ND | ND | ND | ND |
| 78 | 22 | >10000 | >10000 | >10000 | >10000 |
| 79 | 20 | >10000 | >10000 | >10000 | >10000 |
| 80 | 68 | >10000 | >10000 | >10000 | >10000 |
| 81 | 21 | ND | >10000 | ND | ND |
| 82 | 57 | ND | ND | ND | ND |
| 83 | 200 | ND | ND | ND | ND |
| 84 | 29 | ND | >10000 | ND | ND |
| 85 | 23 | >10000 | >10000 | >10000 | >10000 |
| 86 | 31 | ND | ND | ND | ND |
| 87 | 46 | ND | ND | ND | ND |
| 88 | 24 | ND | ND | ND | ND |
| 89 | 60 | ND | ND | ND | ND |
| 90 | 105 | ND | ND | ND | ND |
| 91 | 68 | ND | ND | ND | ND |
| 92 | 54 | >10000 | >10000 | >10000 | >10000 |
| 93 | 40 | ND | ND | ND | ND |
| 94 | 67 | ND | ND | ND | ND |
| 95 | 184 | ND | ND | ND | ND |
| 96 | 20 | >10000 | >10000 | >10000 | >10000 |
| 97 | 29 | ND | ND | ND | ND |
| 98 | 46 | ND | ND | ND | ND |
| 99 | 57 | ND | ND | ND | ND |
| 100 | 53 | ND | ND | ND | ND |
| 101 | 81 | >10000 | >10000 | >10000 | >10000 |
| 102 | 79 | >10000 | >10000 | >10000 | >10000 |
| 103 | 45 | 3108 | 1114 | >10000 | >10000 |
| 104 | 44 | 4350 | 1645 | >10000 | >10000 |
| 105 | 57 | >10000 | 6140 | >10000 | >10000 |
| 106 | 50 | >10000 | >10000 | >10000 | >10000 |
| 107 | 50 | ND | ND | ND | ND |
| 108 | 41 | ND | ND | ND | ND |
| 109 | 32 | ND | ND | ND | ND |
| 110 | 45 | ND | ND | ND | ND |
| 111 | 53 | ND | ND | ND | ND |
| 112 | 61 | ND | ND | ND | ND |
| 113 | 355 | >10000 | >10000 | >10000 | >10000 |
| 114 | 358 | >10000 | >10000 | >10000 | >10000 |
| 115 | 40 | ND | ND | ND | ND |
| 116 | 27 | ND | ND | ND | ND |
| 117 | 144 | ND | ND | ND | ND |

| | | | | | |
|---|---|---|---|---|---|
| ND: Not determined | | | | | |

### Experimental Example 3: Analysis of TYK2/JAKI cell activity by IFNα2B stimulation

An inhibition effect of TYK2/JAK1 activity of the test compound was analyzed using HEK-blue IFNα/β cell line (InvivoGen) that secretes SEAP (Secreted embryonic alkaline phosphatase) depending on the degree of cell stimulation. Cell culture and evaluation were performed according to the manufacturer's recommendations, and the method thereof is as follows: Cells cultured in a growth medium (Dulbecco's Modified Eagle Medium, 10% Fetal bovine serum, 1% Penicillin-Streptomycin, 100 ug/mL Normocin, 10 mg/mL Blasticidin, 100 mg/mL Zeocin) were collected and resuspended in a test medium (DMEM, 10% FBS, 1% Penicillin-Streptomycin), and 3.5x10⁴ cells (160 uL) were dispensed into each well of a 96-well plate. After culturing for 24 hours in a 37°C CO₂ incubator, 20 uL of a test compound or control compound (Deucravacitinib) diluted in the test medium was added, and then incubated at 37°C for 1 hour and 30 minutes. Next, 20 uL of human IFNα2B recombinant protein (PBL assay science) prepared with the test medium was added to each well (final concentration 1 U/mL) and then incubated at 37°C for 24 hours. 20 uL and 180 uL of cell supernatant were mixed with a QUANTI-blue reagent (InvivoGen) and then reacted at 37°C for 20-30 minutes. An absorbance at 630 nm was measured using Synergy neo (BioTek Instruments, Inc.). A reaction mixture that did not contain IFNα2B was a negative control, and a mixture without an arbitrary compound was a positive control. The experimental results are shown in Table 3.

**[Table 3] Result of TYK2/JAK1 cell activity by IFNα2B stimulation test**

| A: IC₅₀ <50 nM; B: IC50 >50 nM and < 500 nM; C: IC₅₀ > 500nM | |
|---|---|
| Example | IC₅₀ |
| 1 | A |
| 2 | B |
| 3 | ND |
| 4 | ND |
| 5 | A |
| 6 | A |
| 7 | A |
| 8 | A |
| 9 | ND |
| 10 | A |
| 11 | A |
| 12 | ND |
| 13 | ND |
| 14 | ND |
| 15 | ND |
| 16 | A |
| 17 | ND |
| 18 | C |
| 19 | B |
| 20 | B |
| 21 | B |
| 22 | A |
| 23 | A |
| 24 | A |
| 25 | A |
| 26 | B |
| 27 | B |
| 28 | B |
| 29 | B |
| 30 | B |
| 31 | A |
| 32 | A |
| 33 | A |
| 34 | A |
| 35 | A |
| 36 | A |
| 37 | A |
| 38 | A |
| 39 | A |
| 40 | A |
| 41 | A |
| 42 | B |
| 43 | A |
| 44 | A |
| 45 | A |
| 46 | B |
| 47 | B |
| 48 | B |
| 49 | A |
| 50 | C |
| 51 | A |
| 52 | A |
| 53 | A |
| 54 | A |
| 55 | A |
| 56 | A |
| 57 | A |
| 58 | A |
| 59 | ND |
| 60 | A |
| 61 | A |
| 62 | A |
| 63 | A |
| 64 | A |
| 65 | A |
| 66 | A |
| 67 | A |
| 68 | A |
| 69 | A |
| 70 | A |
| 71 | A |
| 72 | A |
| 73 | A |
| 74 | A |
| 75 | A |
| 76 | A |
| 77 | A |
| 78 | A |
| 79 | A |
| 80 | A |
| 81 | A |
| 82 | B |
| 83 | B |
| 84 | A |
| 85 | A |
| 86 | B |
| 87 | B |
| 88 | A |
| 89 | B |
| 90 | B |
| 91 | B |
| 92 | ND |
| 93 | ND |
| 94 | ND |
| 95 | A |
| 96 | A |
| 97 | A |
| 98 | B |
| 99 | A |
| 100 | A |
| 101 | A |
| 102 | A |
| 103 | A |
| 104 | A |
| 105 | A |
| 106 | A |
| 107 | A |
| 108 | A |
| 109 | A |
| 110 | A |
| 111 | A |
| 112 | A |
| 113 | C |
| 114 | C |
| 115 | C |
| 116 | A |
| 117 | ND |

| | |
|---|---|
| ND: Not determined | |

## Claims

1. A compound represented by following Chemical Formula 1, a stereoisomer thereof, or a pharmaceutically acceptable salt thereof;
wherein; 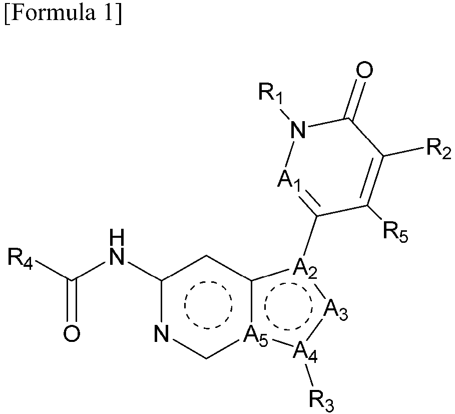 In the above Chemical formula,
A₁, A2, A3, A₄ and A₅ are each independently C, CH, CH₂ or N,
R₁ is C₁-C₁₀alkyl, C₁-C₁₀haloalkyl, C₃-C₈ cycloalkyl, or a 4- to 12-membered heterocycloalkyl, wherein the C₁-C₁₀alkyl, the C₁-C₁₀haloalkyl, the C₃-C₈cycloalkyl, or the 4-to 12-membered heterocycloalkyl is unsubstituted or substituted with one or more substituents selected from a group consisting of oxo, halo, C₁-C₁₀alkyl, C₁-C₁₀alkoxy, and C₁-C₁₀haloalkyl;
R₂ is H, C₁-C₁₀alkyl, C₁-C₁₀alkoxy, C₃-C₈cycloalkyl, 4-12 membered heterocycloalkyl, OR' or NR'R", wherein the C₁-C₁₀alkyl, the C₁-C₁₀alkoxy or the 4-12-membered heterocycloalkyl is unsubstituted or substituted one or more, preferably 1 to 4 substituents selected from a group consisting of halo, cyano, C₁-C₁₀alkyl, C₁-C₁₀alkoxy, C₁-C₁₀alkoxyC₁-C₁₀alkyl, 4-12-membered heterocycloalkyl, C₆-C₁₀aryl, and C₃-C₈cycloalkyl,
wherein, R' and R" are each independently H, or C₁-C₁₀alkyl, wherein, the C₁-C₁₀alkyl is unsubstituted or substituted with one or more substituents selected from a group consisting of halo, cyano, 4-12-membered heterocycloalkyl, C₆-C₁₀aryl, C₃-C₈cycloalkyl and C₁-C₁₀alkoxy, or
R' and R" can form **4-** to 12-membered heterocycloalkyl together with N **in** the NR'R", wherein, the 4- to 12-membered heterocycloalkyl is unsubstituted or substituted with halo, C₁-C₁₀alkoxy, or C₁-C₁₀alkoxyC₁-C₁₀alkyl,
R₃ is H, C₁-C₁₀alkyl, C₁-C₁₀deuteroalkyl, C₂-C₁₀alkenyl, C₃-C₈cycloalkyl, -C₁-C₁₀ alkyl-C₃-C₈cycloalkyl, -C(=O)-C₃-C₈cycloalkyl, C₃-C₈cycloalkenyl, 4- to 12-membered heterocycloalkyl, or -C₁-C₁₀alkyl-4-12-membered heterocycloalkyl, wherein, the C₁-C₁₀alkyl, C₁-C₁₀deuteroalkyl, C₂-C₁₀alkenyl, C₃-C₈cycloalkyl, -C₁-C₁₀alkyl-C₃-C₈cycloalkyl, -C(=O)-C₃-C₈cycloalkyl, C₃-C₈cycloalkenyl, 4-12-membered heterocycloalkyl, or -C₁-C₁₀alkyl-4-12-membered heterocycloalkyl is unsubstituted or substituted with one or more substituents selected from a group consisting of halo, cyano, oxo, C₁-C₁₀alkyl, C₂-C₁₀alkenyl, C₁-C₆alkoxy, C₃-C₈cycloalkyl unsubstituted or substituted with 1 to 3 halo, and 4-12-membered heterocycloalkyl,
R₄ is C₁-C₁₀alkyl, C₁-C₁₀deuteroalkyl, or amino, wherein C₁-C₁₀alkyl, C₁-C₁₀deuteroalkyl, or amino is unsubstituted or substituted with one or more, preferably 1 to 4 substituents selected from a group consisting of hydroxy and C₁-C₁₀alkyl,
R₅ is H, halo, nitro, cyano, amino, hydroxy, -C(=O)R"', -CO(=O)R"', -N(R‴)₂, - C(=O)NR"', -SO₂R‴, C₁-C₁₀alkyl, C₁-C₁₀deuteroalkyl, C₁-C₁₀haloalkyl, C₁-C₁₀hydroxyalkyl, C₁-C₁₀alkoxy, C₂-C₁₀alkenyl, C₂-C₁₀alkynyl, C₃-C₈cycloalkyl, C₆-C₁₀aryl, C₆-C₁₀arylC₁-C₁₀alkyl, 4-12-membered heterocycloalkyl, 4-12-membered heterocycloalkylC₁-C₁₀alkyl, 4-12-membered heteroaryl or 4-12-membered heteroarylC₁-C₁₀alkyl, wherein, the C₁-C₁₀alkyl, C₁-C₁₀deuteroalkyl, C₁-C₁₀haloalkyl, C₁-C₁₀hydroxyalkyl, C₁-C₁₀alkoxy, C₂-C₁₀alkenyl, C₂-C₁₀alkynyl, C₃-C₈cycloalkyl, C₆-C₁₀aryl, C₆-C₁₀arylC₁-C₁₀alkyl, 4-12-membered heterocycloalkyl, 4-12-membered heterocycloalkylC₁-C₁₀alkyl, 4-12-membered heteroaryl or 4-12-membered heteroarylC₁-C₁₀alkyl is unsubstituted, substituted with one or more selected from a group consisting of halo, cyano, nitro, amino, hydroxy, C₁-C₁₀alkyl, C₂-C₁₀alkenyl, C₁-C₁₀alkoxy, C₆-C₁₀aryl, 4-12-membered heterocycloalkyl, 4-12-membered heteroaryl, and C₃-C₈cycloalkyl, and
the R‴ is H, halo, C₁-C₁₀alkyl, C₁-C₁₀deuteroalkyl, C₁-C₁₀haloalkyl, C₁-C₁₀hydroxyalkyl, C₁-C₁₀alkoxy, C₂-C₁₀alkenyl, C₂-C₁₀alkynyl, C₃-C₈cycloalkyl, C₆-C₁₀aryl, C₆-C₁₀arylC₁-C₁₀alkyl, 4-12-membered heterocycloalkyl, 4-12-membered heterocycloalkylC₁-C₁₀alkyl, 4-12-membered heteroaryl or 4-12-membered heteroarylC₁-C₁₀alkyl.

2. The compound, the stereoisomer thereof, or the pharmaceutically acceptable salt thereof of claim 1, wherein the R₁ is C₁-C₆alkyl, C₁-C₆haloalkyl, C₃-C₆cycloalkyl, or 4- to 10-membered heterocycloalkyl, wherein the C₁-C₆alkyl, the C₁-C₆haloalkyl, the C₃-C₆cycloalkyl, or the 4- to 10-membered heterocycloalkyl is unsubstituted or substituted with 1 to 4 substituents selected from a group consisting of oxo, fluoro, chloro, bromo, C₁-C₆alkyl, C₁-C₆alkoxy, and C₁-C₆haloalkyl.

3. The compound, the stereoisomer thereof, or the pharmaceutically acceptable salt thereof of claim 1, wherein C₃-C₆cycloalkyl of the R₁ is cyclobutyl, cyclopentyl or cyclohexyl.

4. The compound, the stereoisomer thereof, or the pharmaceutically acceptable salt thereof of claim 1, wherein 4- to 10-membered heterocycloalkyl of the R₁ is oxetanyl, tetrahydrofuranyl or tetrahydropyranyl.

5. The compound, the stereoisomer thereof, or the pharmaceutically acceptable salt thereof of claim 1, wherein the C₁-C₁₀alkyl, C₁-C₁₀haloalkyl,C₃-C₈cycloalkyl, or 4- to 12-membered heterocycloalkyl of the R₁ is unsubstituted or substituted with 1 to 3 substituents selected from a group consisting of oxo, fluoro, methyl, ethyl, propyl, isopropyl, trifluoromethyl, and methoxy.

6. The compound, the stereoisomer thereof, or the pharmaceutically acceptable salt thereof of claim 1, wherein the R₁ is selected from the following bases:

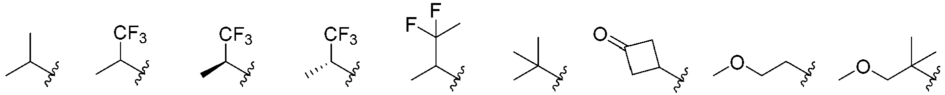

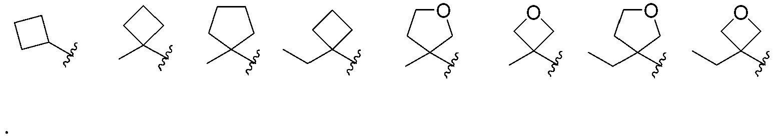

7. The compound, the stereoisomer thereof, or the pharmaceutically acceptable salt thereof of claim 1,
wherein the R₂ is H, C₁-C₆alkyl, C₁-C₆alkoxy, C₃-C₆cycloalkyl, 4-10-membered heterocycloalkyl, OR' or NR'R", and the C₁-C₆alkyl, the C₁-C₆alkoxy or the 4-10-membered heterocycloalkyl is unsubstituted or substituted with 1 to 4 substituents selected from a group consisting of fluoro, chloro, bromo, cyano, C₁-C₆alkyl, C₁-C₆alkoxy, C₁-C₆alkoxyC₁-C₆alkyl, 4-10-membered heterocycloalkyl, C₆-C₁₀aryl, and C₃-C₆cycloalkyl,
wherein, R' and R" are each independently H, or C₁-C₆alkyl, and wherein, the C₁-C₆alkyl is unsubstituted or substituted with 1 to 4 substituents selected from a group consisting of fluoro, chloro, bromo, cyano, 4-10-membered heterocycloalkyl, C₆-C₁₀aryl, C₃-C₆cycloalkyl, and C₁-C₆alkoxy, or
R' and R" can form a 4- to 10-membered heterocycloalkyl together with N of the NR'R", wherein the 4- to 10-membered heterocycloalkyl is unsubstituted or substituted with fluoro, chloro, bromo, C₁-C₆alkoxy, or C₁-C₆alkoxyC₁-C₆alkyl.

8. The compound, the stereoisomer thereof, or the pharmaceutically acceptable salt thereof of claim 1, wherein the R₂ is selected from the following bases:

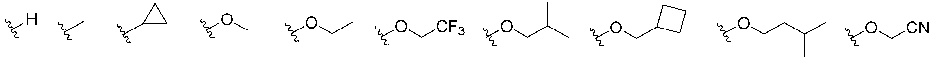

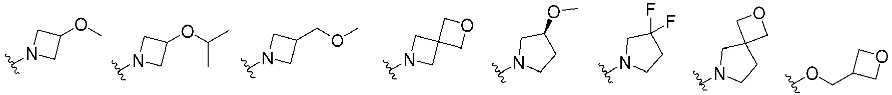

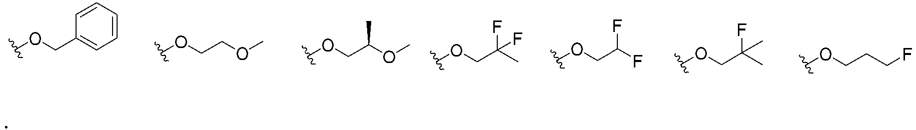

9. The compound, the stereoisomer thereof, or the pharmaceutically acceptable salt thereof of claim 1, wherein the R₃ is H, C₁-C₆alkyl, C₁-C₆deuteroalkyl, C₂-C₆alkenyl, C₃-C₈cycloalkyl, -C₁-C₆alkyl-C₃-C₈cycloalkyl, -C(=O)-C₃-C₈cycloalkyl, C₃-C₈cycloalkenyl, 4-10-membered heterocycloalkyl, or -C₁-C₆ alkyl-4-10-membered heterocycloalkyl, and wherein, the C₁-C₆alkyl, the C₁-C₆deuteroalkyl, the C₂-C₆alkenyl, the C₃-C₈cycloalkyl, the - C₁-C₆alkyl-C₃-C₈cycloalkyl, the -C(=O)-C₃-C₈cycloalkyl, the C₃-C₈cycloalkenyl, the 4-10-membered heterocycloalkyl, or the -C₁-C₆alkyl-4-10-membered heterocycloalkyl is unsubstituted or substituted with 1 to 4 substituents selected from a group consisting of fluoro, chloro, bromo, cyano, oxo, C₁-C₆alkyl, C₂-C₆alkenyl, C₁-C₆alkoxy, C₃-C₈cycloalkyl unsubstituted or substituted with 1 to 3 halo, and 4-10-membered heterocycloalkyl.

10. The compound, the stereoisomer thereof, or the pharmaceutically acceptable salt thereof of claim 1, wherein the R₃ is selected from the following bases:

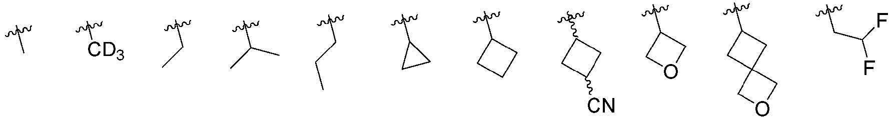

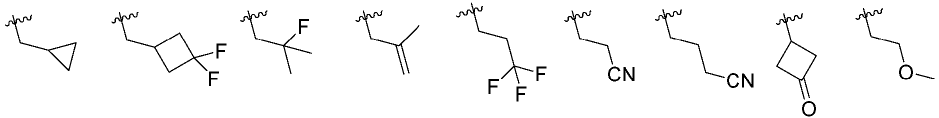

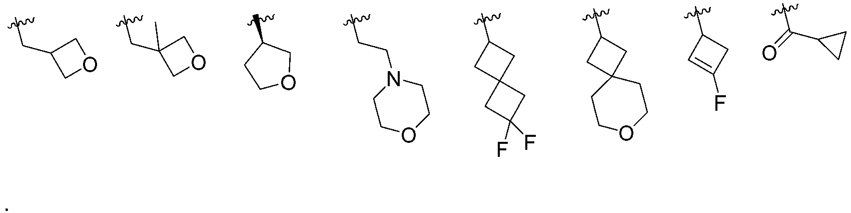

11. The compound, the stereoisomer thereof, or the pharmaceutically acceptable salt thereof of claim 1, wherein the R₄ is C₁-C₆alkyl, C₁-C₆deuteroalkyl, or amino, and wherein the C₁-C₆alkyl, the C₁-C₆deuteroalkyl, or the amino is unsubstituted or substituted with 1 or 2 substituents selected from a group consisting of hydroxy and C₁-C₆alkyl.

12. The compound, the stereoisomer thereof, or the pharmaceutically acceptable salt thereof of claim 1, wherein the R₄ is CH₃, CD₃, CH₂OH, NH₂ or NHCH₃.

13. The compound, the stereoisomer thereof, or the pharmaceutically acceptable salt thereof of claim 1,
wherein the R₅ is H, halo, nitro, cyano, amino, hydroxy, -C(=O)R"', -CO(=O)R"', - N(R‴)₂, -C(=O)NR‴, -SO₂R‴, C₁-C₆alkyl, C₁-C₆deuteroalkyl, C₁-C₆haloalkyl, C₁-C₆hydroxyalkyl, C₁-C₆alkoxy, C₂-C₆alkenyl, C₂-C₆alkynyl, C₃-C₆cycloalkyl, C₆-C₁₀aryl, C₆-C₁₀arylC₁-C₆alkyl, 4-10-membered heterocycloalkyl, 4-10-membered heterocycloalkylC₁-C₆alkyl, 4-10-membered heteroaryl or 4-10-membered heteroarylC₁-C₆alkyl, and wherein, the C₁-C₆alkyl, the C₁-C₆deuteroalkyl, the C₁-C₆haloalkyl, the C₁-C₆hydroxyalkyl, the C₁-C₆alkoxy, the C₂-C₆alkenyl, the C₂-C₆alkynyl, the C₃-C₆cycloalkyl, the C₆-C₁₀aryl, the C₆-C₁₀arylC₁-C₆alkyl, the 4-10-membered heterocycloalkyl, the 4-10-membered heterocycloalkylC₁-C₆alkyl, the 4-10-membered heteroaryl or the 4-10-membered heteroarylC₁-C₆alkyl is unsubstituted or substituted with 1 to 4 substituents selected from a group consisting of halo, cyano, nitro, amino, hydroxy, C₁-C₆alkyl, C₂-C₆alkenyl, C₁-C₆alkoxy, C₆-C₁₀aryl, 4-10-membered heterocycloalkyl, 4-10-membered heteroaryl and C₃-C₆cycloalkyl, and
wherein the R‴ is H, halo, C₁-C₆alkyl, C₁-C₆deuteroalkyl, C₁-C₆haloalkyl, C₁-C₆hydroxyalkyl, C₁-C₆alkoxy, C₂-C₆alkenyl, C₂-C₆alkynyl, C₃-C₆cycloalkyl, C₆-C₁₀aryl, C₆-C₁₀arylC₁-C₆alkyl, 4-10-membered heterocycloalkyl, 4-10-membered heterocycloalkylC₁-C₆alkyl, 4-10-membered heteroaryl or 4-10-membered heteroarylC₁-C₆alkyl.

14. The compound, the stereoisomer thereof, or the pharmaceutically acceptable salt thereof of claim 1,
wherein the compound is selected from a group consisting of the following compounds 1 to 117:
Compound 1: N-(1-methyl-3-(1-(3-methyltetrahydrofuran-3-yl)-6-oxo-1,6-dihydropyridazin-3-yl)-1H-pyrrolo[2,3-c]pyridin-5-yl)acetamide,
Compound 2: N-(1-(3-cyanocyclobutyl)-3-(1-(3-methyltetrahydrofuran-3-yl)-6-oxo-1,6-dihydropyridazin-3-yl)-1H-pyrrolo[2,3-c]pyridin-5-yl)acetamide,
Compound 3: N-(1-methyl-3-(1-(3-methyloxetan-3-yl)-6-oxo-1,6-dihydropyridazin-3-yl)-1H-pyrrolo[2,3-c]pyridin-5-yl)acetamide,
Compound 4: 1-(1-methyl-3-(1-(3-methyltetrahydrofuran-3-yl)-6-oxo-1,6-dihydropyridazin-3-yl)-1H-pyrrolo[2,3-c]pyridin-5-yl)urea,
Compound 5: N-(1-(3-cyanocyclobutyl)-3-(5-methyl-1-(3-methyltetrahydrofuran-3-yl)-6-oxo-1,6-dihydropyridazin-3-yl)-1H-pyrrolo[2,3-c]pyridin-5-yl)acetamide,
Compound 6: N-(1-(3-cyanocyclobutyl)-3-(1-(3-ethyltetrahydrofuran-3-yl)-5-methyl-6-oxo-1,6-dihydropyridazin-3-yl)-1H-pyrrolo[2,3-c]pyridin-5-yl)acetamide,
Compound 7: 1-(3-(1-(3-ethyltetrahydrofuran-3-yl)-5-methyl-6-oxo-1,6-dihydropyridazin-3-yl)-1-methyl-1H-pyrrolo[2,3-c]pyridin-5-yl)urea,
Compound 8: N-(1-(3-cyanocyclobutyl)-3-(5-cyclopropyl-1-(3-methyltetrahydrofuran-3-yl)-6-oxo-1,6-dihydropyridazin-3-yl)-1H-pyrrolo[2,3-c]pyridin-5-yl)acetamide,
Compound 9: N-(1-(3-cyanocyclobutyl)-3-(5-ethoxy-1-(3-methyltetrahydrofuran-3-yl)-6-oxo-1,6-dihydropyridazin-3-yl)-1H-pyrrolo[2,3-c]pyridin-5-yl)acetamide,
Compound 10: N-(1-(3-cyanocyclobutyl)-3-(5-(3-methoxyazetidin-1-yl)-1-(3-methyltetrahydrofuran-3-yl)-6-oxo-1,6-dihydropyridazin-3-yl)-1H-pyrrolo[2,3-c]pyridin-5-yl)acetamide,
Compound 11: 1-(3-(5-(3-methoxyazetidin-1-yl)-1-(3-methyltetrahydrofuran-3-yl)-6-oxo-1,6-dihydropyridazin-3-yl)-1-methyl-1H-pyrrolo[2,3-c]pyridin-5-yl)urea,
Compound 12: N-(1-(3-cyanocyclobutyl)-3-(5-cyclopropyl-6-oxo-1-(3-oxocyclobutyl)-1,6-dihydropyridazin-3-yl)-1H-pyrrolo[2,3-c]pyridin-5-yl)acetamide,
Compound 13: N-(1-(3-cyanocyclobutyl)-3-(1-(3-methyltetrahydrofuran-3-yl)-6-oxo-1,6-dihydropyridin-3-yl)-1H-pyrrolo[2,3-c]pyridin-5-yl)acetamide,
Compound 14: 1-(1-methyl-3-(1-(3-methyltetrahydrofuran-3-yl)-6-oxo-1,6-dihydropyridin-3-yl)-1H-pyrrolo[2,3-c]pyridin-5-yl)urea,
Compound 15: N-(3-methyl-1-(1-(3-methyltetrahydrofuran-3-yl)-6-oxo-1,6-dihydropyridazin-3-yl)-1H-pyrrolo[3,2-c]pyridin-6-yl)acetamide,
Compound 16: N-(5-(5-(3-methoxyazetidine-1-yl)-1-(3-methyltetrahydrofuran-3-yl)-6-oxo-1,6-dihydropyridazin-3-yl)-7-methylpyrrolo[1,2-c]pyrimidin-3-yl)acetamide,
Compound 17: N-(3-(5-(3-methoxyazetidin-1-yl)-1-(2-methoxyethyl)-6-oxo-1,6-dihydropyridazin-3-yl)-1-methyl-1H-pyrrolo[2,3-c]pyridin-5-yl)acetamide,
Compound 18: N-(3-(1-(1-methoxy-2-methylpropan-2-yl)-6-oxo-1,6-dihydropyridazin-3-yl)-1-methyl-1H-pyrrolo[2,3-c]pyridin-5-yl)acetamide,
Compound 19: N-(3-(5-isobutoxy-1-isopropyl-6-oxo-1,6-dihydropyridazin-3-yl)-1-methyl-1H-pyrrolo[2,3-c]pyridin-5-yl)acetamide,
Compound 20: N-(3-(1-isopropyl-5-(3-methoxyazetidin-1-yl)-6-oxo-1,6-dihydropyridazin-3-yl)-1-methyl-1H-pyrrolo[2,3-c]pyridin-5-yl)acetamide,
Compound 21: N-(1-cyclopropyl-3-(1-isopropyl-5-(3-methoxyazetidin-1-yl)-6-oxo-1,6-dihydropyridazin-3-yl)-1H-pyrrolo[2,3-c]pyridin-5-yl)acetamide,
Compound 22: N-(1-(3-cyanocyclobutyl)-3-(5-isobutoxy-1-isopropyl-6-oxo-1,6-dihydropyridazin-3-yl)-1H-pyrrolo[2,3-c]pyridin-5-yl)acetamide,
Compound 23: N-(3-(1-cyclobutyl-5-isobutoxy-6-oxo-1,6-dihydropyridazin-3-yl)-1-methyl-1H-pyrrolo[2,3-c]pyridin-5-yl)acetamide,
Compound 24: N-(3-(1-cyclobutyl-5-(3-(methoxymethyl)azetidin-1-yl)-6-oxo-1,6-dihydropyridazin-3-yl)-1-methyl-1H-pyrrolo[2,3-c]pyridin-5-yl)acetamide,
Compound 25: N-(3-(5-(isopentyloxy)-1-isopropyl-6-oxo-1,6-dihydropyridazin-3-yl)-1-methyl-1H-pyrrolo[2,3-c]pyridin-5-yl)acetamide,
Compound 26: N-(3-(1-isopropyl-6-oxo-5-(2,2,2-trifluoroethoxy)-1,6-dihydropyridazin-3-yl)-1-methyl-1H-pyrrolo[2,3-c]pyridin-5-yl)acetamide,
Compound 27: N-(3-(5-(benzyloxy)-1-isopropyl-6-oxo-1,6-dihydropyridazin-3-yl)-1-methyl-1H-pyrrolo[2,3-c]pyridin-5-yl)acetamide,
Compound 28: N-(3-(1-(tert-butyl)-5-isobutoxy-6-oxo-1,6-dihydropyridazin-3-yl)-1-methyl-1H-pyrrolo[2,3-c]pyridin-5-yl)acetamide,
Compound 29: N-(3-(1-(tert-butyl)-5-(3-methoxyazetidin-1-yl)-6-oxo-1,6-dihydropyridazin-3-yl)-1-methyl-1H-pyrrolo[2,3-c]pyridin-5-yl)acetamide,
Compound 30: N-(3-(5-isobutoxy-1-(1-methylcyclopentyl)-6-oxo-1,6-dihydropyridazin-3-yl)-1-methyl-1H-pyrrolo[2,3-c]pyridin-5 -yl)acetamide,
Compound 31: N-(3-(5-(3-methoxyazetidin-1-yl)-1-(1-methylcyclopentyl)-6-oxo-1,6-dihydropyridazin-3-yl)-1-methyl-1H-pyrrolo[2,3-c]pyridin-5-yl)acetamide,
Compound 32: N-(3-(5-isobutoxy-1-(1-methylcyclobutyl)-6-oxo-1,6-dihydropyridazin-3-yl)-1-methyl-1H-pyrrolo[2,3-c]pyridin-5-yl)acetamide,
Compound 33: N-(1-cyclobutyl-3-(5-isobutoxy-1-isopropyl-6-oxo-1,6-dihydropyridazin-3-yl)-1H-pyrrolo[2,3-c]pyridin-5-yl)acetamide,
Compound 34: 1-(3-(5-isobutoxy-1-isopropyl-6-oxo-1,6-dihydropyridazin-3-yl)-1-methyl-1H-pyrrolo[2,3-c]pyridin-5-yl)urea,
Compound 35: 1-(1-cyclobutyl-3-(5-isobutoxy-1-(1-methylcyclobutyl)-6-oxo-1,6-dihydropyridazin-3-yl)-1H-pyrrolo[2,3-c]pyridin-5-yl)urea,
Compound 36: N-(1-cyclobutyl-3-(5-isobutoxy-6-oxo-1-(1,1,1-trifluoropropan-2-yl)-1,6-dihydropyridazin-3-yl)-1H-pyrrolo[2,3-c]pyridin-5-yl)acetamide,
Compound 37: 1-(3-(5-isobutoxy-6-oxo-1-(1,1,1-trifluoropropan-2-yl)-1,6-dihydropyridazin-3-yl)-1-methyl-1H-pyrrolo[2,3-c]pyridin-5-yl)urea,
Compound 38: 1-(1-cyclobutyl-3-(5-isobutoxy-1-isopropyl-6-oxo-1,6-dihydropyridazin-3-yl)-1H-pyrrolo[2,3-c]pyridin-5-yl)urea,
Compound 39: N-(3-(5-(3-methoxyazetidin-1-yl)-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 40: N-(3-(5-(3-methoxyazetidin-1-yl)-1-(1-methylcyclobutyl)-6-oxo-1,6-dihydropyridazin-3-yl)-1-methyl-1H-pyrrolo[2,3-c]pyridin-5-yl)acetamide,
Compound 41: N-(1-cyclobutyl-3-(5-(3-methoxyazetidin-1-yl)-1-(1-methylcyclobutyl)-6-oxo-1,6-dihydropyridazin-3-yl)-1H-pyrrolo[2,3-c]pyridin-5-yl)acetamide,
Compound 42: (S)-N-(3-(5-(3-methoxypyrrolidin-1-yl)-1-(1-methylcyclobutyl)-6-oxo-1,6-dihydropyridazin-3-yl)-1-methyl-1H-pyrrolo[2,3-c]pyridin-5-yl)acetamide,
Compound 43: (S)-N-(1-cyclobutyl-3-(5-(3-methoxypyrrolidin-1-yl)-1-(1-methylcyclobutyl)-6-oxo-1,6-dihydropyridazin-3-yl)-1H-pyrrolo[2,3-c]pyridin-5-yl)acetamide,
Compound 44: N-(1-cyclobutyl-3-(1-(1-methylcyclobutyl)-6-oxo-5-(2-oxa-6-azaspiro[3.4]octan-6-yl)-1,6-dihydropyridazin-3- yl)-1H-pyrrolo[2,3-c]pyridin-5-yl)acetamide,
Compound 45: N-(1-methyl-3-(1-(1-methylcyclobutyl)-6-oxo-5-(2-oxa-6-azaspiro[3.4]octan-6-yl)-1,6-dihydropyridazin-3- yl)-1H-pyrrolo[2,3-c]pyridin-5-yl)acetamide,
Compound 46: N-(1-methyl-3-(1-(1-methylcyclobutyl)-6-oxo-5-(2-oxa-6-azaspiro[3.3]heptan-6-yl)-1,6-dihydropyridazin-3- yl)-1H-pyrrolo[2,3-c]pyridin-5-yl)acetamide,
Compound 47: N-(3-(5-(cyanomethoxy)-1-(1-methylcyclobutyl)-6-oxo-1,6-dihydropyridazin-3-yl)-1-methyl-1H-pyrrolo[2,3-c]pyridin-5-yl)acetamide,
Compound 48: N-(1-methyl-3-(1-(1-methylcyclobutyl)-5-(oxetan-3-ylmethoxy)-6-oxo-1,6-dihydropyridazin-3-yl)-1H-pyrrolo[2,3-c]pyridin-5-yl)acetamide,
Compound 49: N-(1-cyclopropyl-3-(5-isobutoxy-6-oxo-1-(1,1,1-trifluoropropan-2-yl)-1,6-dihydropyridazin-3-yl)-1H-pyrrolo[2,3-c]pyridin-5-yl)acetamide,
Compound 50: (R)-N-(3-(5-isobutoxy-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 51: N-(3-(5-isobutoxy-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 52: N-(1-cyclopropyl-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide,
Compound 53: N-(1-cyclopropyl-3-(1-(1-methylcyclobutyl)-6-oxo-5-(2-oxa-6-azaspiro[3.4]octan-6-yl)-1,6-dihydropyridazin-3-yl)-1H-pyrrolo[2,3-c]pyridin-5-yl)acetamide,
Compound 54: (S)-N-(3-(5-isobutoxy-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 55: N-(3-(5-(cyclobutylmethoxy)-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 56: N-(3-(5-(2,2-difluoropropoxy)-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 57: (R)-N-(3-(1-(1-ethylcyclobutyl)-5-(2-methoxypropoxy)-6-oxo-1,6-dihydropyridazin-3-yl)-1-methyl-1H-pyrrolo[2,3-c]pyridin-5-yl)acetamide,
Compound 58: (S)-N-(1-cyclopropyl-3-(6-oxo-5-(2-oxa-6-azaspiro[3.4]octan-6-yl)-1-(1,1,1-trifluoropropan-2 -yl)-1,6-dihydropyridazin-3-yl)-1H-pyrrolo[2,3-c]pyridin-5-yl)acetamide,
Compound 59: N-(3-(1-(3,3-difluorobutan-2-yl)-5-isobutoxy-6-oxo-1,6-dihydropyridazin-3-yl)-1-methyl-1H-pyrrolo[2,3-c]pyridin-5-yl)acetamide,
Compound 60: N-(3-(1-(1-ethylcyclobutyl)-5-isobutoxy-6-oxo-1,6-dihydropyridazin-3-yl)-1-methyl-1H-pyrrolo[2,3-c]pyridin-5-yl)acetamide,
Compound 61: (S)-N-(3-(5-(3-methoxyazetidin-1-yl)-6-oxo-1-(1,1,1-trifluoropropan-2-yl)-1,6-dihydropyridazin-3- yl)-1-methyl-1H-pyrrolo[2,3-c]pyridin-5-yl)acetamide,
Compound 62: (S)-N-(3-(5-(2,2-difluoropropoxy)-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 63: N-(1-((1S,3R)-3-cyanocyclobutyl)-3-(5-isobutoxy-6-oxo-1-((S)-1,1,1-trifluoropropan-2-yl)-1,6-dihydropyridazin-3-yl)-1H-pyrrolo[2,3-c]pyridin-5-yl)acetamide,
Compound 64: (S)-N-(1-cyclopropyl-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide,
Compound 65: N-(1-cyclopropyl-3-(5-((R)-2-methoxypropoxy)-6-oxo-1-((S)-1,1,1-trifluoropropan-2-yl)-1,6-dihydropyridazin-3-yl)-1H-pyrrolo[2,3-c]pyridin-5-yl)acetamide,
Compound 66: (S)-N-(1-cyclopropyl-3-(5-(2-methoxyethoxy)-6-oxo-1-(1,1,1-trifluoropropan-2-yl)-1,6-dihydropyridazin-3-yl)-1H-pyrrolo[2,3-c]pyridin-5-yl)acetamide,
Compound 67: (S)-N-(3-(5-(3,3-difluoropyrrolidin-1-yl)-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 68: N-(1-((1S,3R)-3-cyanocyclobutyl)-3-(5-(2,2-difluoropropoxy)-6-oxo-1-((S)-1,1,1-trifluoropropan-2-yl)-1,6-dihydropyridazin-3-yl)-1H-pyrrolo[2,3-c]pyridin-5-yl)acetamide,
Compound 69: (S)-N-(3-(5-(3-methoxyazetidin-1-yl)-6-oxo-1-(1,1,1-trifluoropropan-2-yl)-1,6-dihydropyridazin-3-yl)-1-methyl-1H-pyrrolo[2,3-c]pyridin-5-yl)acetamide-2,2,2-d3,
Compound 70: (S)-N-(3-(5-(2,2-difluoroethoxy)-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 71: (S)-N-(1-cyclopropyl-3-(5-(2,2-difluoroethoxy)-6-oxo-1-(1,1,1-trifluoropropan-2-yl)-1,6-dihydropyridazin-3-yl)-1H-pyrrolo[2,3-c]pyridin-5-yl)acetamide,
Compound 72: (S)-N-(3-(5-(2,2-difluoropropoxy)-6-oxo-1-(1,1,1-trifluoropropan-2-yl)-1,6-dihydropyridazin-3-yl)-1-methyl-1H-pyrrolo[2,3-c]pyridin-5-yl)acetamide-2,2,2-d3,
Compound 73: (S)-N-(3-(5-(2,2-difluoropropoxy)-6-oxo-1-(1,1,1-trifluoropropan-2-yl)-1,6-dihydropyridazin-3-yl)-1-(methyl-d3)-1H-pyrrolo[2,3-c]pyridin-5-yl)acetamide,
Compound 74: (S)-N-(1-cyclopropyl-3-(5-(2,2-difluoropropoxy)-6-oxo-1-(1,1,1-trifluoropropan-2-yl)-1,6-dihydropyridazin-3-yl)-1H-pyrrolo[2,3-c]pyridin-5-yl)acetamide,
Compound 75: (S)-N-(3-(5-(3-isopropoxyazetidin-1-yl)-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 76: (S)-N-(1-ethyl-3-(5-(3-isopropoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide,
Compound 77: (S)-N-(1-cyclopropyl-3-(5-(3-isopropoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide,
Compound 78: (S)-N-(3-(5-(2,2-difluoropropoxy)-6-oxo-1-(1,1,1-trifluoropropan-2-yl)-1,6-dihydropyridazin-3-yl)-1-ethyl-1H-pyrrolo[2,3-c]pyridin-5-yl)acetamide,
Compound 79: (S)-N-(3-(5-(2,2-difluoropropoxy)-6-oxo-1-(1,1,1-trifluoropropan-2-yl)-1,6-dihydropyridazin-3-yl)-1-(2-oxaspiro[3.3]heptan-6-yl)-1H-pyrrolo[2,3-c]pyridin-5-yl)acetamide,
Compound 80: (S)-N-(1-(cyclopropyl methyl)-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide,
Compound 81: (S)-N-(1-((3,3-difluorocyclobutyl)methyl)-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide,
Compound 82: (S)-N-(1-(2-fluoro-2-methylpropyl)-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide,
Compound 83: (S)-N-(3-(5-(3-methoxyazetidin-1-yl)-6-oxo-1-(1,1,1-trifluoropropan-2-yl)-1,6-dihydropyridazin-3-yl)-1-(2-methylallyl)-1H-pyrrolo[2,3-c]pyridin-5-yl)acetamide,
Compound 84: (S)-N-(3-(5-(3-methoxyazetidin-1-yl)-6-oxo-1-(1,1,1-trifluoropropan-2-yl)-1,6-dihydropyridazin-3-yl)-1-(3,3,3-trifluoropropyl)-1H-pyrrolo[2,3-c]pyridin-5-yl)acetamide, and
Compound 85: (S)-N-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)acetamide,
Compound 86: (S)-N-(3-(5-(2,2-difluoropropoxy)-6-oxo-1-(1,1,1-trifluoropropan-2-yl)-1,6-dihydropyridazin-3-yl)-1-(2-methoxyethyl)-1H-pyrrolo[2,3-c]pyridin-5-yl)acetamide,
Compound 87: (S)-N-(1-(2-cyanoethyl)-3-(5-(2,2-difluoropropoxy)-6-oxo-1-(1,1,1-trifluoropropan-2-yl)-1,6-dihydropyridazin-3-yl)-1H-pyrrolo[2,3-c]pyridin-5-yl)acetamide,
Compound 88: (S)-N-(1-(3-cyanopropyl)-3-(5-(2,2-difluoropropoxy)-6-oxo-1-(1,1,1-trifluoropropan-2-yl)-1,6-dihydropyridazin-3-yl)-1H-pyrrolo[2,3-c]pyridin-5-yl)acetamide,
Compound 89: (S)-N-(3-(5-(2,2-difluoropropoxy)-6-oxo-1-(1,1,1-trifluoropropan-2-yl)-1,6-dihydropyridazin-3-yl)-1-(3-oxocyclobutyl)-1H-pyrrolo[2,3-c]pyridin-5-yl)acetamide,
Compound 90: (S)-N-(3-(5-(2,2-difluoropropoxy)-6-oxo-1-(1,1,1-trifluoropropan-2-yl)-1,6-dihydropyridazin-3-yl)-1-((3-methyloxetan-3-yl)methyl)-1H-pyrrolo[2,3-c]pyridin-5-yl)acetamide,
Compound 91: (S)-N-(3-(5-(2,2-difluoropropoxy)-6-oxo-1-(1,1,1-trifluoropropan-2-yl)-1,6-dihydropyridazin-3-yl)-1-(2-morpholinoethyl)-1H-pyrrolo[2,3-c]pyridin-5-yl)acetamide,
Compound 92: (S)-N-(1-(cyclopropylmethyl)-3-(5-(2,2-difluoropropoxy)-6-oxo-1-(1,1,1-trifluoropropan-2-yl)-1,6-dihydropyridazin-3-yl)-1H-pyrrolo[2,3-c]pyridin-5-yl)acetamide,
Compound 93: (S)-N-(3-(5-(2,2-difluoropropoxy)-6-oxo-1-(1,1,1-trifluoropropan-2-yl)-1,6-dihydropyridazin-3-yl)-1-(oxetan-3-ylmethyl)-1H-pyrrolo[2,3-c]pyridin-5-yl)acetamide,
Compound 94: N-(3-(5-(2,2-difluoropropoxy)-6-oxo-1-((S)-1,1,1-trifluoropropan-2-yl)-1,6-dihydropyridazin-3-yl)-1-((R)-tetrahydrofuran-3-yl)-1H-pyrrolo[2,3-c]pyridin-5-yl)acetamide,
Compound 95: (S)-N-(1-(6,6-difluorospiro[3.3]heptan-2-yl)-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide,
Compound 96: (S)-N-(3-(5-(2,2-difluoropropoxy)-6-oxo-1-(1,1,1-trifluoropropan-2-yl)-1,6-dihydropyridazin-3-yl)-1-propyl-1H-pyrrolo[2,3-c]pyridin-5-yl)acetamide,
Compound 97: (S)-N-(3-(5-(2,2-difluoropropoxy)-6-oxo-1-(1,1,1-trifluoropropan-2-yl)-1,6-dihydropyridazin-3-yl)-1-(7-oxaspiro[3.5]nonan-2-yl)-1H-pyrrolo[2,3-c]pyridin-5-yl)acetamide,
Compound 98: (S)-N-(1-(cyclopropanecarbonyl)-3-(5-(2,2-difluoropropoxy)-6-oxo-1-(1,1,1-trifluoropropan-2-yl)-1,6-dihydropyridazin-3-yl)-1H-pyrrolo[2,3-c]pyridin-5-yl)acetamide,
Compound 99: (S)-N-(1-(2,2-difluoroethyl)-3-(5-(2,2-difluoropropoxy)-6-oxo-1-(1,1,1-trifluoropropan-2-yl)-1,6-dihydropyridazin-3-yl)-1H-pyrrolo[2,3-c]pyridin-5-yl)acetamide,
Compound 100: N-(3-(5-(2,2-difluoropropoxy)-6-oxo-1-((S)-1,1,1-trifluoropropan-2-yl)-1,6-dihydropyridazin-3-yl)-1-(3-fluorocyclobut-2-en-1-yl)-1H-pyrrolo[2,3-c]pyridin-5-yl)acetamide,
Compound 101: (S)-N-(3-(5-(2,2-difluoropropoxy)-6-oxo-1-(1,1,1-trifluoropropan-2-yl)-1,6-dihydropyridazin-3-yl)-1-isopropyl-1H-pyrrolo[2,3-c]pyridin-5-yl)acetamide,
Compound 102: (S)-N-(1-ethyl-3-(5-(3-methoxyazetidin-1-yl)-6-oxo-1-(1,1,1-trifluoropropan-2-yl)-1,6-dihydropyridazin-3-yl)-1H-pyrrolo[2,3-c]pyridin-5-yl)acetamide,
Compound 103: (S)-N-(3-(5-(2-fluoro-2-methylpropoxy)-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 104: (S)-N-(1-methyl-3-(6-oxo-5-(2,2,2-trifluoroethoxy)-1-(1,1,1-trifluoropropan-2-yl)-1,6-dihydropyridazin-3-yl)-1H-pyrrolo[2,3-c]pyridin-5-yl)acetamide,
Compound 105: (S)-N-(1-ethyl-3-(5-(2-fluoro-2-methylpropoxy)-6-oxo-1-(1,1,1-trifluoropropan-2-yl)-1,6-dihydropyridazin-3-yl)-1H-pyrrolo[2,3-c]pyridin-5-yl)acetamide,
Compound 106: (S)-N-(1-ethyl-3-(6-oxo-5-(2,2,2-trifluoroethoxy)-1-(1,1,1-trifluoropropan-2-yl)-1,6-dihydropyridazin-3-yl)-1H-pyrrolo[2,3-c]pyridin-5-yl)acetamide,
Compound 107: (S)-N-(1-cyclopropyl-3-(6-oxo-5-(2,2,2-trifluoroethoxy)-1-(1,1,1-trifluoropropan-2-yl)-1,6-dihydropyridazin-3-yl)-1H-pyrrolo[2,3-c]pyridin-5-yl)acetamide,
Compound 108: (S)-N-(1-ethyl-3-(5-(3-fluoropropoxy)-6-oxo-1-(1,1,1-trifluoropropan-2-yl)-1,6-dihydropyridazin-3-yl)-1H-pyrrolo[2,3-c]pyridin-5-yl)acetamide,
Compound 109: (S)-N-(3-(5-(3-fluoropropoxy)-6-oxo-1-(1,1,1-trifluoropropan-2-yl)-1,6-dihydropyridazin-3-yl)-1-methyl-1H-pyrrolo[2,3-c]pyridin-5-yl)acetamide,
Compound 110: (S)-N-(1-cyclopropyl-3-(5-(2-fluoro-2-methylpropoxy)-6-oxo-1-(1,1,1-trifluoropropan-2-yl)-1,6-dihydropyridazin-3-yl)-1H-pyrrolo[2,3-c]pyridin-5-yl)acetamide,
Compound 111: N-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)acetamide,
Compound 112: N-(1-cyclopropyl-3-(5-(2,2-difluoroethoxy)-6-oxo-1-(1,1,1-trifluoropropan-2-yl)-1,6-dihydropyridazin-3-yl)-1H-pyrrolo[2,3-c]pyridin-5-yl)acetamide,
Compound 113: (R)-N-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)acetamide,
Compound 114: (R)-N-(1-cyclopropyl-3-(5-(2,2-difluoroethoxy)-6-oxo-1-(1,1,1-trifluoropropan-2-yl)-1,6-dihydropyridazin-3-yl)-1H-pyrrolo[2,3-c]pyridin-5-yl)acetamide,
Compound 115: (S)-N-(3-(5-(2,2-difluoroethoxy)-6-oxo-1-(1,1,1-trifluoropropan-2-yl)-1,6-dihydropyridazin-3-yl)-1H-pyrrolo[2,3-c]pyridin-5-yl)acetamide,
Compound 116: (S)-N-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)-2-hydroxyacetamide, and
Compound 117: (S)-1-(3-(5-(2,2-difluoroethoxy)-6-oxo-1-(1,1,1-trifluoropropan-2-yl)-1,6-dihydropyridazin-3-yl)-1-ethyl-1H-pyrrolo[2,3-c]pyridin-5-yl)-3-methylurea.

15. A pharmaceutical composition for preventing, improving or treating a TYK2-related disease, which includes a TYK2 inhibitor compound of any one of claims 1 to 14, a stereoisomer thereof, or a pharmaceutically acceptable salt thereof.

16. The pharmaceutical composition of claim 15, wherein the TYK2-related disease is an autoimmune disease, an inflammatory disease, cancer, or a neurodegenerative disease.

17. The pharmaceutical composition of claim 16, wherein the autoimmune and the inflammatory diseases are one or more selected from a group consisting of asthma, autoimmune inflammatory bowel disease, alopecia areata, ankylosing spondylitis, lupus, lupus nephritis, crohn's disease, ulcerative colitis, rheumatoid arthritis, psoriasis (including plaque, scalp, and nail psoriasis), sjogren's syndrome, multiple sclerosis, palmar pustulosis, celiac disease, atopic dermatitis, contact dermatitis, delayed hypersensitivity, treatment of eye disease and conditions such as ocular allergies, conjunctivitis, dry keratoconjunctivitis, and vernal conjunctivitis, diseases affecting a nose including allergic rhinitis, interstitial pulmonary fibrosis, psoriatic arthritis, systemic juvenile idiopathic arthritis, cryopyrinassociated periodic syndrome, nephritis, vasculitis, diverticulitis, interstitial cystitis, glomerulonephritis, endometritis, leptospirosis, kidney disease, cardiac hypertrophy, muscle wasting, catabolic disorders, fetal growth retardation, hypercholesterolemia, heart disease, chronic heart failure, mesothelioma, anhidrotic ecodermal dysplasia, Behcet's disease, incontinentia pigmenti, pancreatitis, hereditary periodic fever syndrome, asthma, acute lung injury, acute respiratory distress syndrome, eosinophilia, hypersensitivity, anaphylaxis, nasal sinusitis, ocular allergy, silica-induced disease, chronic obstructive pulmonary disease (COPD), cystic fibrosis, acid-induced lung injury, pulmonary hypertension, COVID119, cataracts, muscle inflammation with systemic sclerosis, inclusion body myositis, myasthenia gravis, thyroiditis, addison's disease, lichen planus, type 1 diabetes, or type 2 diabetes, temporal arteritis, tendonitis, dermatomyositis, appendicitis, bronchiolitis, bronchitis, bursitis, cervicitis, cholangitis, cholecystitis, chronic graft rejection, colitis, conjunctivitis, crohn's disease, cystitis, dacryoadenitis, encephalitis, endocarditis, endometritis, enteritis, enterocolitis, epicondylitis, epididymitis, fasciitis, fibrositis, gastritis, Henoch-Schonlein purpura, hepatitis, hidradenitis suppurativa, leukocyte adhesion deficiency, immunoglobulin A nephropathy, interstitial lung disease, laryngitis, mastitis, meningitis, myelitis, myocarditis, myositis, nephritis, oophoritis, orchitis, osteitis, otitis, pancreatitis, parotitis, pericarditis, peritonitis, pharyngitis, pleuritis, phlebitis, pneumonia, polymyositis, proctitis, prostatitis, pyelonephritis, rhinitis, salpingitis, sinusitis, stomatitis, synovitis, tendonitis, tonsillitis, ulcerative colitis, uveitis, vaginitis, vasculitis, and vulvitis.

18. The pharmaceutical composition of claim 16, wherein the cancer is one or more selected from a group consisting of breast cancer, ovarian cancer, cervical cancer, prostate cancer, testicular cancer, penile cancer, genitourinary cancer, seminoma, esophageal cancer, laryngeal cancer, gastric cancer, stomach cancer, gastrointestinal cancer, skin cancer, keratoacanthoma, follicular carcinoma, melanoma, lung cancer, small cell lung carcinoma, non-small cell lung carcinoma (NSCLC), lung adenocarcinoma, lung squamous cell carcinoma, adenomatous polyp, colon cancer, pancreatic cancer, thyroid cancer, papillary cancer, bladder cancer, liver cancer, biliary tract cancer, renal cancer, bone cancer, bone marrow disease, lymphatic disease, trichoblastoma, oral cancer and pharyngeal cancer (oral), lip cancer, tongue cancer, oral cancer, salivary gland cancer, pharyngeal cancer, small intestine cancer, colon cancer, rectal cancer, anal cancer, renal cancer, prostate cancer, vulvar cancer, thyroid cancer, colon cancer, endometrial cancer, cervical cancer, brain cancer, central nervous system cancer, peritoneum cancer, hepatocellular carcinoma, head cancer, neck cancer, hopkins disease and leukemia (acute myeloid leukemia).

19. The pharmaceutical composition of claim 16, wherein the neurodegenerative disease is one or more selected from a group consisting of alzheimer's disease, parkinson's disease, and amyotrophic lateral sclerosis.

20. A preparing method of Chemical Formula 1 of claim 1, comprising borylating a compound represented by a following Chemical Formula 3 and then performing a Suzuki reaction or Ullmann reaction with a compound corresponding to Chemical Formula 2 to obtain a compound represented by Chemical Formula 1: 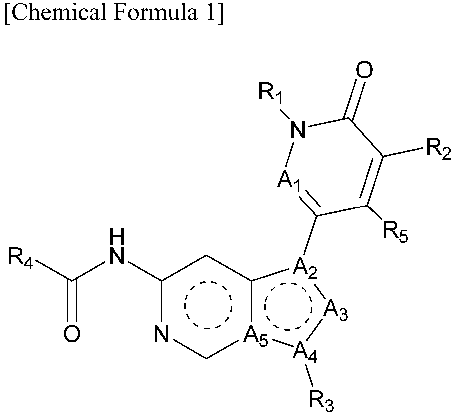 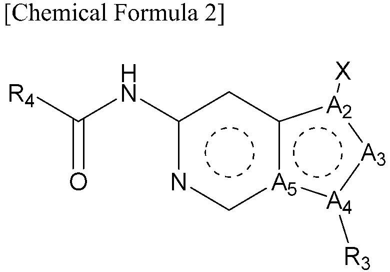 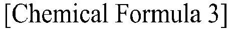 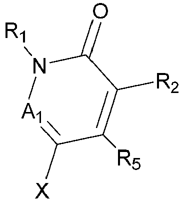 In the above formula,
A₁, A₂, A₃, A₄, A₅, R₁, R₂, R₃, R₄, and R₅ are as defined in claim 1, and
X is halo.

21. The preparing method of claim 20, wherein the compound of Chemical Formula 2 is prepared by a preparing method including the following steps:
preparing a compound represented by the following Chemical Formula 5 by reacting a compound represented by the following Chemical Formula 4 with NBS or NIS;
preparing a compound represented by the following Chemical Formula 6 by reacting the compound represented by the following Chemical Formula 5 to S_{N}2 reaction or Chan-Lam reaction; and
preparing the compound represented by Chemical Formula 2 by deacetylating the compound represented by the following Chemical Formula 6 with an acid, and then reacting the same with isocyanate or acetyl chloride.
wherein; 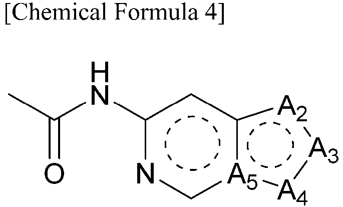 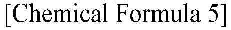 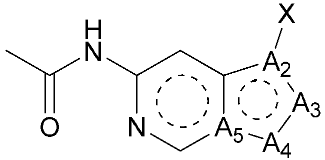 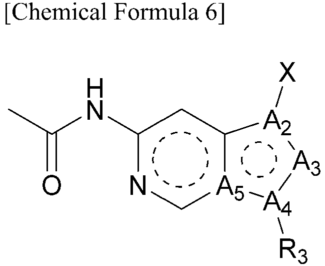
In the above formula,
A₂, A₃, A₄, A₅, R₃ and R₄ are as defined in Formula 1, and
X is halo.

22. The preparing method of claim 20, wherein the compound of Chemical Formula 3 is prepared by a preparing method including the following steps:
preparing a compound represented by Chemical Formula 8 by Mitsunobu reaction or S_{N}2 reaction from a compound represented by the following Chemical Formula 7; and
preparing a compound represented by Chemical Formula 3 by reacting a compound represented by the following Chemical Formula 8 with HOR' or HNR'R" compound as a base:
wherein; 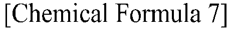 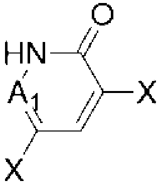 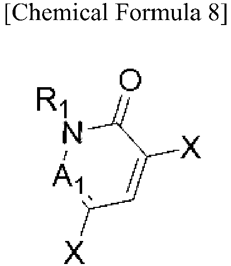 In the above formula,
A₁ and R₁ are as defined in Formula 1, and
X is halo.

23. The preparing method of claim 20, wherein the compound of Chemical Formula 3 is prepared by a preparing method including the following steps:
preparing a compound represented by Chemical Formula 8 by Mitsunobu reaction or S_{N}2 reaction from a compound represented by the following Chemical Formula 9; 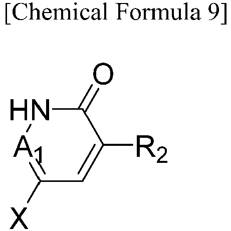
In the above formula,
A₁ and R₁ are as defined in Formula 1, and
X is halo.

24. A preparing method of Chemical Formula 1 of claim 1, comprising:
a step of borylating the compound of Chemical Formula 3, then performing a Suzuki reaction or Ullmann reaction with the compound of Chemical Formula 6 to obtain a compound of Chemical Formula 10; and
a step of deacetylating the compound of Chemical Formula 10 with an acid, and then reacting the deacetylated compound with acetoxy acetyl chloride or methylamine to obtain a compound of Chemical Formula 1: 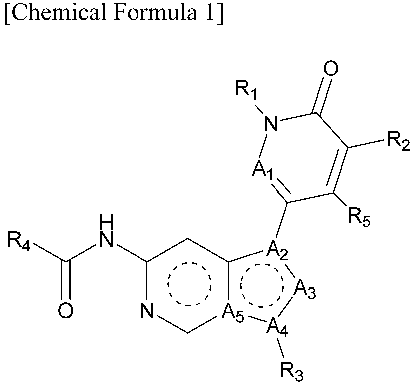 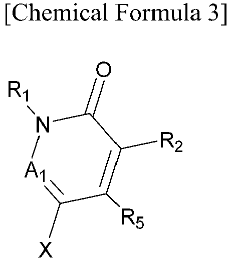 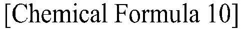 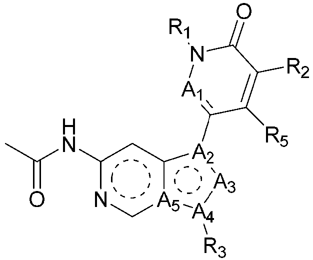 in the above Formulas,
A₁, A₂, A₃, A₄, A₅, R₁, R₂, R₃, R₄, and R₅ are as defined in claim 1, and
X is halo.
